# EUROPEAN PATENT SPECIFICATION

(11) **EP 3 024 481 B1**
(45) Date of publication and mention of the grant of the patent: **11.07.2018**
(21) Application number: 14744338.6
(22) Date of filing: 28.07.2014
(51) Int. Cl.: A61K 38/57, C07K 14/81

(54) **INSECT METALLOPROTEINASE INHIBITORS**
INSEKTEN-METALLOPROTEINASEHEMMER
INHIBITEURS DE LA MÉTALLOPROTÉINASE D'INSECTES

(30) Priority: 26.07.2013 EP 13178180
(43) Date of publication of application: 01.06.2016
(73) Proprietor: Fraunhofer Gesellschaft zur Förderung der angewandten Forschung E.V., 80686 München (DE)
(72) Inventor: VILCINSKAS, Andreas, 35463 Fernwald (DE); FISCHER, Rainer, 52076 Aachen (DE)
(74) Representative: Roth, Andy Stefan
(86) International application number: PCT/EP2014/066165
(87) International publication number: WO 2015/011293

(56) References cited:
- WEDDE MARIANNE ET AL: "Purification and characterization of an inducible metalloprotease inhibitor from the hemolymph of greater wax moth larvae, Galleria mellonella", EUR. J. BIOCHEM., vol. 255, no. 3, 1 August 1998 (1998-08-01), pages 535-543, XP002214760, cited in the application
- ANJA CLERMONT ET AL: "Cloning and expression of an inhibitor of microbial metalloproteinases from insects contributing to innate immunity", THE BIOCHEMICAL JOURNAL, vol. 382, 15 August 2004 (2004-08-15), pages 315-322, XP055101475, England DOI: 10.1042/BJ20031923 cited in the application

## Description

### FIELD OF THE INVENTION

This invention relates to peptides and proteins inhibiting protease activity. More specifically, the invention relates to peptides exhibiting activity against a spectrum of proteases of microbial or fungal origin, in particular against the metzincin family including thermolysin, anthrax neutral protease 599 (npr599), pseudolysin, and aureolysin. The invention relates further to methods for producing the peptides, to pharmaceutical compositions comprising the peptides or proteins, and to methods of using the peptides or proteins to prevent and/or treat bacterial or fungal infections and their symptoms, in particular to reduce the toxic effects of secreted or membrane bound bacterial proteases such as aureolysin, bacillolysin, pseudolysin, vibriolysin, and anthrax npr599 by inhibiting their respective proteolytic activity.

### BACKGROUND OF THE INVENTION

Antibiotics are the actual standard treatment for bacterial infections. The widespread and sometimes inadequate use of antibiotics, however, has led to pathogens developing resistance against one or even multiple antibiotics. Multidrug resistant bacteria pose an increasing and serious threat to infected patients. Therefore the search for new companion compounds dampening the bacterial virulence has become an urgent need during the last years. A a rapid effect of compounds against dangerous or life threatening symptoms of bacterial infection is desirable to protect the patient immediately against these symptoms even before antibiotics or alternative compounds can kill the microbes.

For decades it has been known that bacteria release proteases (Duthie et al. 1949). Later the pathogenic role of microbial proteases as virulence factors was recognized experimentally (Snell et al. 1978). Proteolytic enzymes produced by pathogens and parasites are essential for their successful infection of the host. Regardless of their substrate specificity the key function of individual pathogen-derived proteinases is the degradation of host proteins and peptides (Armstrong et al. 2006). Extracellular digestion of host proteins facilitates, for example, penetration across physical barriers of the host, for which the degradation of the extracellular matrix may serve as a prominent example. Furthermore, the degradation of immunity-related defense molecules of the host, including antimicrobial peptides (AMP) (Otto 2008), and the acquisition of nutrients are important functions for the pathogen to survive. However, only those pathogen-associated proteolytic enzymes can serve as virulence factors that cannot or can only partially be inactivated by corresponding, endogenous host proteinase inhibitors. Virulence factors usually complement each other in degrading host peptides and proteins during infection. Consequently, pathogen-derived proteinases without corresponding endogenous inhibitors in the host will be evolutionarily selected because pathogens would otherwise waste their limited resources (Vilcinskas 2010).

Mammals and many invertebrates lack specific inhibitors for proteases of the M4 or Metzincin family of metalloproteinases, of which thermolysin from *Bacillus thermoproteolyticus* is a salient example. Thermolysin-like metalloproteinases encompass many prominent toxins produced by human pathogens, including aureolysin, bacillolysin, pseudolysin, vibriolysin, and anthrax npr599, which are presumably at the origin of many pathological symptoms associated with severe infections such as septicemia, hemorrhagic tissue bleeding, necrosis and enhancement of vascular permeability (Chung et al. 2006). Severe diseases like gastric and peptic ulcers and gastric carcinoma originate at least partly from the effect of metalloproteinases of the M4 family from pathogens (Schmidtchen et al. 2003, Smith et al. 1994).

For example, the major symptoms of an infection with bacillus anthracis are caused by the release of virulence factors from the bacteria. The well known virulence factor anthrax toxin leads to the destruction of the host macrophages, thereby enabling the bacteria to spread in the host tissue without encountering host defense. The anthrax toxin is a holoenzyme containing three different proteins, each of which is present in multiple copies. They are called protective antigen (PA), edema factor (EF), and lethal factor (LF). To be become active, it is required that at least PA and EF or PA and LF are combined. Just a single protein or just EF and LF without PA are not active in laboratory animals.

More recently it became evident that the combined activity of the PA, EF and LF alone does not cover all observed clinical anthrax symptoms. It was shown that anthrax harbors further genes coding for secreted proteins exhibiting proteolytic activity. Amongst those, gene BA0599 from bacillus anthracis (BA) codes for a protease called neutral protease 599 (npr599), which belongs to the M4 family of proteases and is highly homologous to bacillolysins from other *bacillus* species (Popov et al. 2005, Chung et al. 2006, Chung et al. 2008).

Currently several treatment options for anthrax infections exist. A vaccine is available which protects with a safety of more than 90%, but usually the degree of vaccination against anthrax in a human population is low and so far it is not expected that this degree will increase. Anthrax can further be treated with antibiotics in case of an acute infection. However, the toxins shed by the bacteria continue to cause irreversible damage to tissue before the number of bacteria is sufficiently reduced by antibiotics. It is even likely that exposure to antibiotics stimulates bacteria to temporarily shed increased amounts of virulence factors, causing accelerated and irreversible damage of patient tissue. Furthermore, manmade synthetic or recombinant versions of the natural bacterial toxins may be inhaled by humans without bacteria being present, in which cases an antibiotic would have no curing effect at all.

Therefore the task of inhibiting or antagonizing bacterial toxins directly was recognized as a challenge, and inhibition strategies were published. In the case of Pseudomonas aeruginosa virulence factor LasB (Cathgart G.R. et al, 2011), N-mercaptoacetyl-Phe-Tyr-amide (K(i) = 41 nM) was suggested as inhibitor. Similarly, Anthrax Lethal Factor protease inhibitors were suggested in the past, for example (2R)-2-[(4-fluoro-3-methylphenyl)sulfonylamino]-*N*-hydroxy-2-(tetra-hydro-2H-pyran-4-yl)acetamide (Xiong Y. et al, 2006, US 7,504,425 B2), as well as a compound named NSC12155 (Panchal R.G. et al. 2004), and substrate derived hydroxamates with Ki of up to 1 nM (In-2-LF) (Tonello F. et all 2002). A peptide derived from the substrate (MLARRKKVYPYPMEPTIAEG-amide) was found to inhibit LF with a Ki of 1 nM as well (Turk B.E. et al. 2002). No inhibitor of LF reached a clinical development stage so far. Side effects of some inhibitors are known, comprising, for example, the inhibition of endogenous furin in parallel to LF. In the laboratory, Nrp599 can be inhibited with EDTA and 1,10-phenanthroline (Chung et al. 2006), but further, therapeutically acceptable Nrp599 inhibitors are not published up to now.

Several inhibitors of other members of the thermolysin family were described earlier. Phosphoramidon is produced by the Bacterium *Streptomyces tanashiensis,* and Talopeptin is derived from it. Phosphonomadites termed ZG^{P}LL and ZF^{P}LA display inhibitory constants in the low nanomolar range (Holden et al. 1987) with respect to thermolysin.

Khan et al described 1β-d-arabinofuranosyl-*N*⁴-lauroylcytosine as potent inhibitor of thermolysin out of set of 12 inhibitors found in virtual screening run (Khan et al. 2009); and 3-Phenyl-2-(trifluoromethyl) quinazolin-4(3*H*)-one as most potent inhibitor out of a set of novel 38 quinazolin-4(3*H*)-ones (Khan et al. 2010).

Therapeutic inhibition of thermolysin activity by administering non-specific metalloproteinase inhibitors, however, is biased by negative side effects induced by collateral inhibition of essential host enzymes such as its matrix metalloproteinases. Phosphoramidon, for example, is inhibiting the endogenous protein *endothelin converting enzyme* (ECE). Furthermore, some of these compounds are toxic, while for others the toxicity is obviously unknown. Consequently, a strong demand for molecules capable of specifically inhibiting one microbial enzyme (Adekoya et al. 2009) or a corresponding enzyme family persists.

Streptomyces metalloproteinase inhibitor (SMPI) (Oda et al. 1979) is a known proteinaceous inhibitior of thermolysin. SMPI consists of 102 amino acids and exhibits two cystein bridges exhibiting an IC50 of 0.6 nM for Thermolysin (Hiraga et al. 1999). It was discovered, however, that SMPI is degraded by other bacterial proteases (Tsuru et al.1992), disqualifying SMPI for use as a compound for treating bacterial infections. Furthermore, its low molecular weight of ca. 12 kD means that it is rapidly cleared from the bloodstream by renal filtration.

The only other peptide inhibitor of thermolysin reported to date which specifically inhibits thermolysin-like enzymes is the insect metalloproteinase inhibitor IMPIa. It was originally discovered in and purified from the hemolymph of immunized G. *mellonella* larvae (Wedde et al. 1998). Its active moiety comprises 69 amino acids including intramolecular cystein bonds, and a molecular weight of 7667.7 Da. The molecule has a reported IC50 of 0.62, 0.86 and only 81.66 nM for thermolysin, bacillolysin and pseudolysin, respectively, all of which are enzymes belonging to the M4 protease family (Clermont et al.2004). IMPIα was tested against human metallo-matrix proteases MMP1,2,3,7,8, and -9, of which only MMP1 and MMP2 showed a negligible inhibition. IMPIa was recombinantly produced in Schneider cells (Clermont et al. 2004) and later in E.coli. Although the structure of IMPI was not known until recently (Gomes-Rueth. 2011), it was suggested by homology searches that IMPIa should belong to the 18 or Ascaris family of serin protease inhibitors, despite the fact that IMPIa inhibits a metalloprotease and not a serin protease (Wedde et al., 2007). From this comparison it was further deduced that an active site loop would be present in IMPIa between aa 33 - aa40, including a cleavage site between aa37 (Asparagine) and aa38 (Isoleucin). Other known protein inhibitors of metalloproteinases do not inhibit proteinases of the M4 protease family.

In summary, most known inhibitors o of members of the M4 protease family are too toxic, unspecific, unstable or difficult to manufacture or express, or require a different specificity profile against metalloproteases.

### SUMMARY OF THE INVENTION

It is therefore an object of the present invention to provide inhibitors of M4 protease family members with an increased potency for specifc M4 proteases, a selected if not engineered specificity profile including the potential requirement for a broad band metzincin inhibitior. Furthermore, higher stability, lower toxicity or more efficient manufacturing or expression profile is requested.

The object underlying the invention is accomplished by a polypeptide according to claim 1 and other independent claims. The claims depending on one or more independent claims are concerning specific embodiment of the invention.

Tfhe polypeptide is a polypeptide having at least 70% homology, in particular 80%, 90% or 95% homology to the polypeptide of SEQ ID No 2 representing the wild-type of the protein insect metalloproteinase inhibitor IMPIa and having at least one mutation at position 35, 36 and/or 39 of the amino acid sequence of IMPIa (SEQ ID No 2), wherein
- the nonpolar amino acid isoleucine at position 35 of IMPIa is replaced either by a nonpolar amino acid selected from the group consisting of leucine, methionine and phenylalanine or by polar amino acid selected from the group consisting of cysteine, asparagine, glutamine, histidine, lysine and arginine; and/or
- the nonpolar amino acid isoleucine at position 36 of IMPIa is replaced either by a nonpolar amino acid selected from the group consisting of valine, phenylalanine and tryptophan or by polar amino acid selected from the group consisting of tyrosine, serine, threonine, asparagine, glutamine, histidine, lysine and arginine; and/or
- the polar amino acid position 39 of IMPIa is replaced either by the nonpolar amino acid valine or by the polar amino acids histidine or lysine.

The polypeptide of of the invention shows in particular an IC₅₀ value to thermolysine of less than the IC₅₀ value of IMPIa.

In an embodiment of the invention the polypeptide of the invention has the amino acid sequences of SEQ ID numbers 10, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84.

In an another embodiment of the invention the polypeptide of the invention may comprise chemical modifications in the side chain or at the N and/or C terminal for improving biological or chemical properties such as bio availability, stability, effectivity. The modification may also provide for a detectable label, for example a chemiluminescent structural element, one or more radioactive isotopes in one or more side chains of an amino acid in the polypeptide, an enzyme which is able to generate a colour reaction and the like.

Subject matter of the present invention is also a fusion polypeptide comprising the polypeptide of the invention and at least one polypeptide having a physiological function in particular an antibody or antibody fragment, scaffolds such as lipocalin, ankyrin, fibronectin, transferrin, tetranectin, adnectin, albumin, uteroglobin, or protein A, functional peptides such as transferrin, peptides useful for diagnostic applications, such as green fluorescent protein (GFP), or peptide tags enabling immobilization on technical surfaces, such as hexahistidine, or glutathione-S-transferase (GST).

In an embodiment of the invention the polypeptide of the invention has the amino acid sequences of SEQ ID numbers ,6,8, 12, 86, 88, 90, 92.

There are three superfamilies (cytosolic, mitochondrial, and MAPEG) of GSTs: while classes from the cytosolic superfamily of GSTs possess more than 40% sequence homology, those from other classes may have less than 25%. Cytosolic GSTs are divided into 13 classes based upon their structure: alpha, beta, delta, epsilon, zeta, theta, mu, nu, pi, sigma, tau, phi, and omega. Mitochondrial GSTs are in class kappa. The MAPEG superfamily of microsomal GSTs consists of subgroups designated I-IV, between which amino acid sequences share less than 20% identity. Human cytosolic GSTs belong to the alpha, zeta, theta, mu, pi, sigma, and omega classes, while six isozymes belonging to classes I, II, and IV of the MAPEG superfamily are known to exist:

| GST Class | *Homo sapiens* GST Class Members |
|---|---|
| Alpha | GSTA1, GSTA2, GSTA3, GSTA4, GSTA5 |
| Kappa | GSTK1 |
| Mu | GSTM1, GSTM1L (RNAi), GSTM2, GSTM3, GSTM4, GSTM5 |
| Omega | GSTO1, GSTO2 |
| Pi | GSTP1 |
| Theta | GSTT1, GSTT2, GSTT4 |
| Zeta | GSTZ1 (aka GSTZ1 MAAI-Maleylacetoacetate isomerase) |
| Microsomal | MGST1, MGST2, MGST3 |

In a further embodiment of the invention the fusion polypeptide of the invention is linked by a peptide of 1 to 100 amino acids in length to the the polypeptide of the invention. Alternatively polypeptide of the invention can be conjugated by a chemical linking group with a polypeptide having a physiological function to yield the fusion polypeptide of the invention.

Polypeptides or proteins can be manufactured by chemical methods, such as solid phase syntheses or by means of genetic engineering by means of utilizing coding polynucletides in a suitable expression system. Subject matter of the present invention is also a polynucleotide coding for the polypeptide of the invention or the fusion polypeptide of the invention. The polynucleotide of the invention may be operably linked to a heterologous promoter.

Subject matter of the present invention is also a pharmaceutical composition comprising the polypeptide of the invention and/or the fusion polypeptide of the invention or their derivatives and/or a nucleic acid comprising a section coding for the polypeptide of invention.

The polypeptide of the invention or the fusion polypeptide of the invention can be used in the treatment of an animal or human infected by microorganisms secreting bacterial toxins of the M4 or Metzincin family of metalloproteinases, in particular thermolysine, aureolysin, bacillolysin, pseudolysin, vibriolysin or anthrax npr599.

The polypeptide and/or the fusion polypeptide of the invention can be used for detection of the presence or activity of proteases belonging to the M4 family in a sample.

The invention is based on the result that mutations between amino acid 35 and 39 of IMPIa (SEQ ID NO: 10, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84) could be used to alter systematically the specificity profile and potency related to specifically inhibiting members of the thermolysin M4 protease family. Accordingly, the polypeptide IMPIa of the invention inhibit further proteases of the thermolysinM4 protease family, particular thermolysine, aureolysin, bacillolysin, pseudolysin, vibriolysin or anthrax npr599, and that the mutein IMPIa exhibit different inhibitory constants with respect to the various proteases.

As used herein, the following terms have the following meanings unless expressly stated to the contrary.

The term "wtIMPIα" refers to a protein with an amino acid sequence as in SEQ ID NO:2 and consists of the N-terminal fragment of the full length IMPI molecule which is endogenously cleaved from the larger precursor molecule IMPI.

The term "wtIMPIβ" refers to a protein with an amino acid sequence as in SEQ ID NO:4 and consists of the C-terminal fragment of the full length IMPI molecule which is endogenously cleaved from the larger precursor molecule IMPI.

The term "loop mutein IMPIα" comprises all proteins comprising an amino acid sequence identical to SEQ ID NO:2 (wtIMPIa), with the exception of the amino acid stretch 35 to 39 , and in particular with exception of the amino acid stretch 35, 36, or 39, in which at least one amino acid is exchanged, added, or deleted. By this definition "Loop mutein IMPIα" includes, for example, amino acid sequence SEQ ID NO: 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, in which one amino acid at position 35,36, or 39 is exchanged. In SEQ ID NO 10 two additional amino acids at positions 37 and 38 are exchanged.

The term "mtIMPIα" or "Mutein IMPIα" is the polypeptide of the invention and includes all recombinant or synthetic proteins with an amino acid sequence which is at least 70%, especially 80% 90%, 95% homologous, but not identical to SEQ ID NO: 2 and which inhibits thermolysin or another protease of the M4 protease family with an IC₅₀ < 1000 nM, especially IC₅₀ < 100 nM, in particular those which can be expressed in E.coli and especially those including proteins of the group "loop mutein IMPIα" and "structure variants of IMPIα". mtIMPIα include inserted, substituted or deleted residues, including N-terminal and C-terminal and intrasequence deletions, provided the selected mutations preserve the biological activity of the Mutein IMPIα.

The term "M4 family of proteases" refers to the definition given in the MEROPS online database of peptidases (http://merops.sanger.ac.uk/cgi-bin/famsum?family=M4), and described also by Barret et al. (2004).

The term "biologically active" as used herein refers to wtIMPIα or the polypeptide of the invention demonstrating inhibition of thermolysin or another protease of the M4 protease family with an IC₅₀ <1000 nM especially IC₅₀ < 100 nM or better.

The term "IMPIα family" alone shall include biologically active mtIMPIα and wtIMPIα

### BRIEF DESCRIPTION OF THE FIGURES

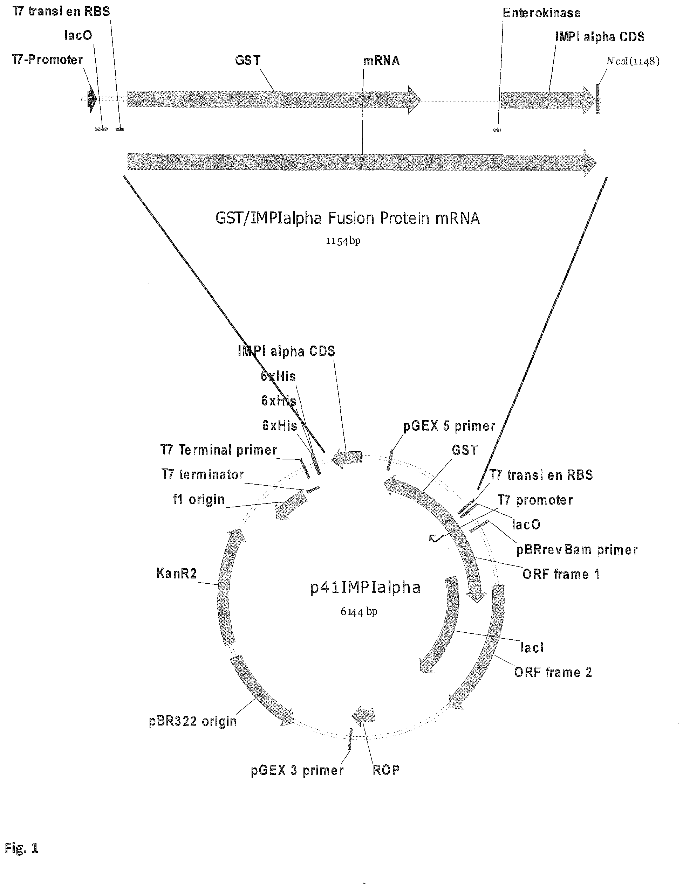

- Fig.1:: Vectormap of the hybridplasmid pET41IMPIa. Total mRNA was isolated from immunchallenged Galleria melonella und subsequently used as template for the amplification of DNA coding for IMPIa. The amplicon was then cloned into the PshA1 linearized pET-41A vector (Novagen) resulting in a GST (Glutathion-S-transferase) fusionprotein. Accurate in frame cloning of IMPIa into the vector was verified by sequencing.

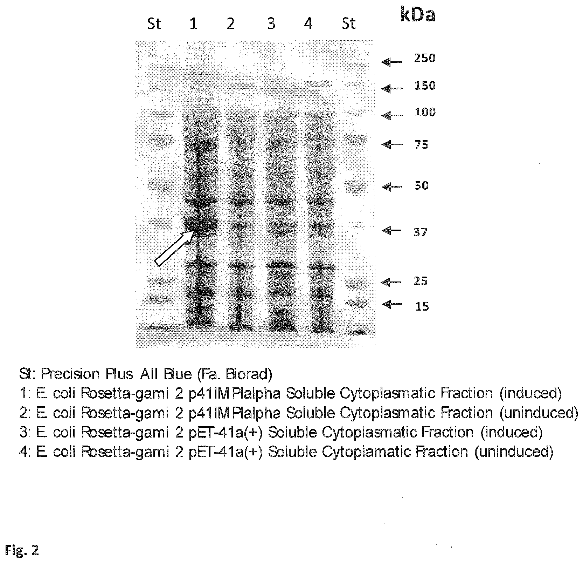

- Fig. 2:: SDS-PAGE of the soluble cytoplasmatic fraction of Rosetta gami 2 three hours after induction of the T7-Expression system. The overexpressed fusionprotein is marked by an arrow. The corresponding controls (empty pET-41 and the uninduced T7-Expression system) show no corresponding bands.

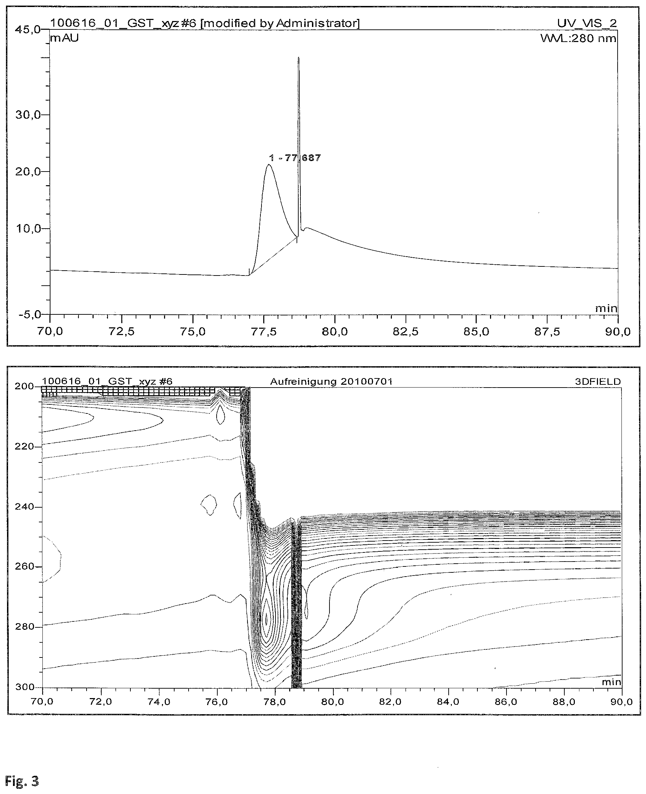

- Fig. 3: HPLC UV detector data from purification run of GST-tagged IMPIa. The purification has been carried out by affinity chromatography on a HPLC, using a resin with immobilized glutathion at room temperature. The purification has been carried out by affinity chromatography on a HPLC, using a resin with immobilized glutathion at room temperature.

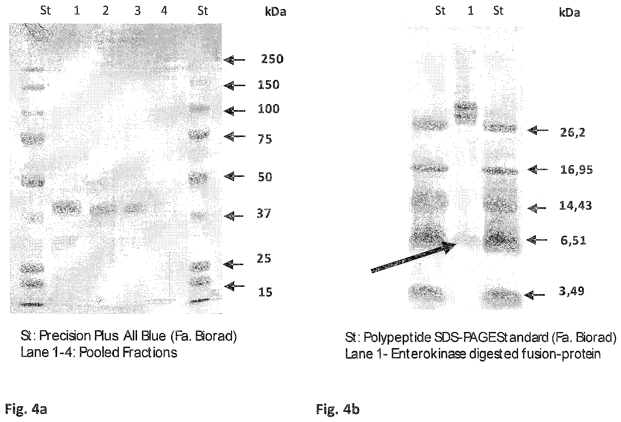

- Fig. 4a: SDS-PAGE of the purified GST-tagged IMPIa For larger amounts of mutein GST Bind Fractogel Cartriges (Merck, Darmstadt) were used, while for amounts less than 40 mg glutathione spin columns (Pierce; Thermo Scientific, Rockford) have been deployed.
- Fig. 4b:: SDS-PAGE after the specific cleavage of the n-terminal GST-tagged IMPI alpha by recombinant enterokinase (Merck, Darmstadt). The cleavage was carried out for 16 hours at room temperature. Untagged IMPIa is marked by an arrow.

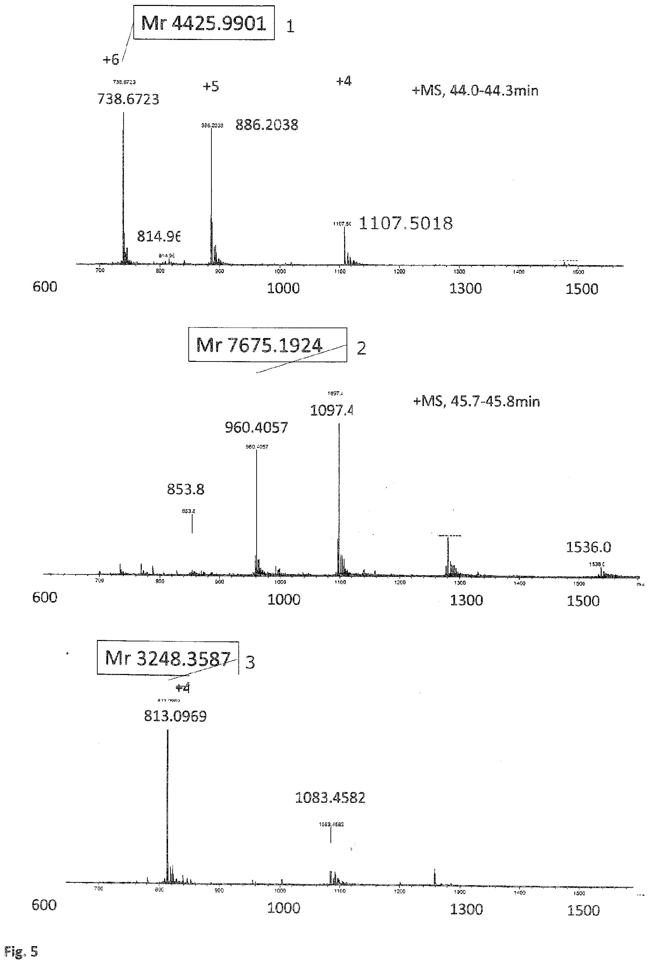

- Fig. 5:: Sucessfull downstream processing was verfied by mass spectroscopy on a micrOTOF-Q mass spectrometer (Bruker Daltonics). Analysis of samples was achieved by chromatography on a reverse-phase column (Acclaim 120, C8, 3 µm, 120 Å, 2.1 x 150 mm; Dionex) by applying a gradient of 1-80 % (1,63 %/min) acetonitril in water containing 0.1 percent formic acid. By-product (peak termed 1 and 3) and main product (peak 1) show the correct mass of 7,677 kDa.

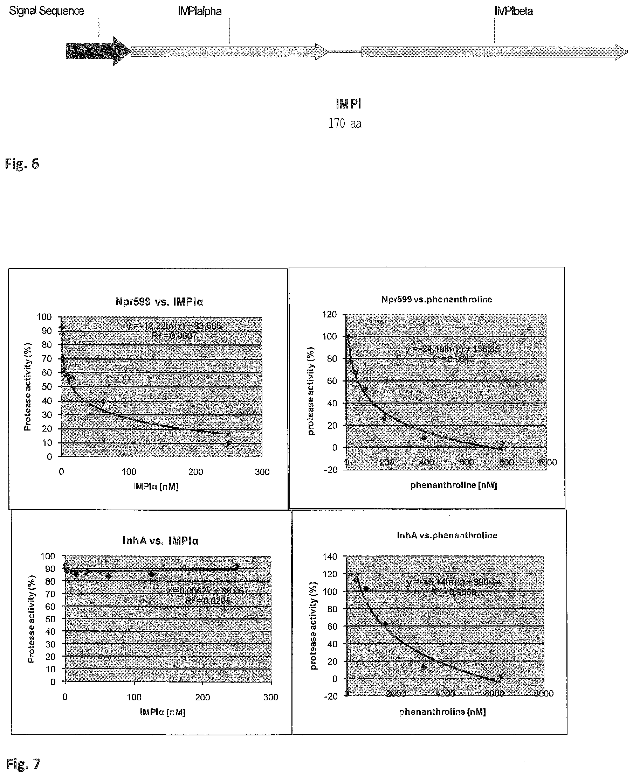

- Fig. 6:: wild type protein assembly of full length IMPI, including IMPIa and IMPIβ in G. melonella.
- Fig. 7:: inhibitory plot of wtIMPIα and phenanthronin vs. the anthrax protein nrp599 and InhA

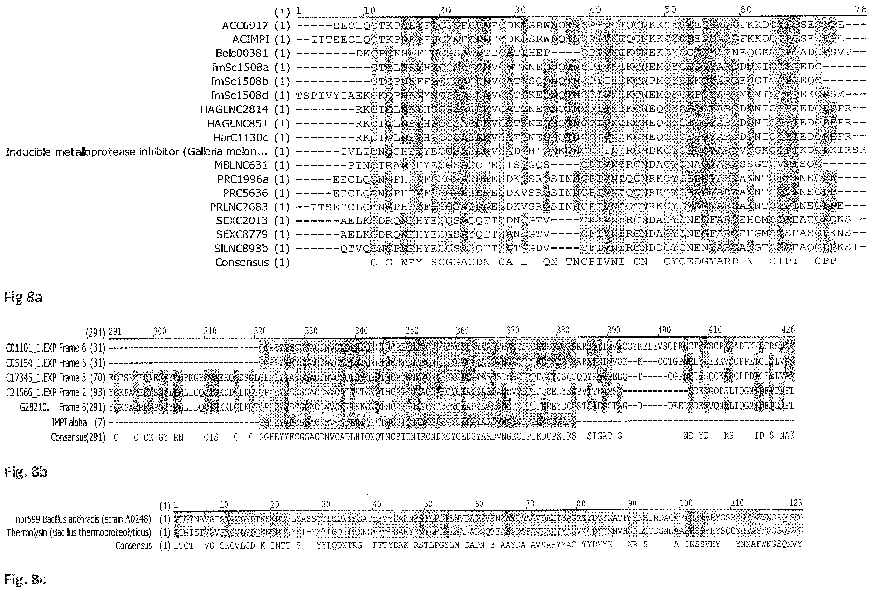

- Fig. 8a:: Multiple alignement of IMPIa homologues of *Galleria melonella.*
- Fig. 8b:: Multiple alignment of IMPI's found in the transcriptome of other lepidoptera species (Sequences unbublished).
- Fig. 8c:: Pairwise alignment of the M4-metalloprotease thermolysin from *Bacillus thermoproteolyticus* with the metalloprotease npr599 from Bacillus anthracis (strain A0248)
- Fig. 9:: The binding of the peptidic inhibitor IMPIa to the M4-metalloprotease thermolysin (*Bacillus thermoproteolyticus*) is depicted. Red areas represent Van der Waals contact areas between inhibitor and protease. The isoleucin (P1')(purple) fits perfectly into the S1' pocket which is responsible for specificity of the metalloptotease hence the inhibitor is binding in a lock and key kind of fashion

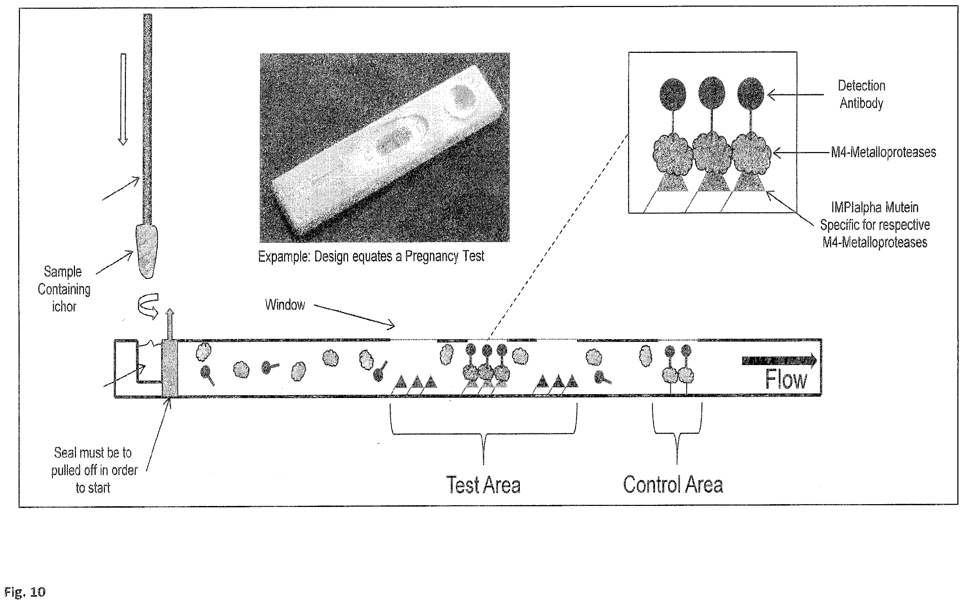

- Fig. 10:: Schematic drawing of a lateral flow device for detecting protease specific activity. The principle is based on thin layer chromatography. The eluent carries ichor collected by a cotton swap via tangential flow through the device. M4-metalloproteases produced by pathogens therefore also migrate through the device, until they get bound to IMPI muteins exhibiting certain specificity for different metalloproteases. The different muteins are bound at concentrated spots on the solid phase of the device, so that different metalloproteases can be clearly identified. Spots are made visible afterwards by detection antibodies present in the mobile phase.
- Table 1:: Overview of amino acid exchanges and respective functionality for Thermolysine and Pseudolysine, and respective ratio of IC₅₀ values. The ratio changes mean that an inhibitor with designed ratio of IC₅₀S with respect to different proteases can be created.
- Table 2:: Detailled experimental parameters and results for inhibition of Thermolysine.

### DETAILED DESCRIPTION OF THE INVENTION

In one aspect of the invention, biologically active loop polypeptide of the invention polypeptides are provided which exhibit a specificity profile different from the wtIMPIα profile for inhibiting members of the M4 protease family. The fact that the loop polypeptide of the invention are biologically active at all is surprising because the loop amino acids were particularly well conserved during evolution. It is even more surprising that altering a specific loop amino acid could increase the binding constant for thermolysin or pseudolysin, or even both.

New ways of improving the inhibitory constant by substituting 136 (Isoleucine) of wtIMPIα have been developed, which is surprising since it was assumed that at this position 36 isoleucine would already exhibit a perfect profile for a C-C interaction with amino acids H146 (histidine) and T157 (tyrosine) of the peptidase, and therefore no attempts were reported to design IMPI mutants or small molecules with the goal to strengthen this interaction further. The inventors found, however, that a long aliphatic side chain of an amino acid or modified amino acid at this position, preferably with an elevated pKa of 12 or higher, improves the inhibitory constant substantially. Examples of well suited amino acids comprise K, M, H, and in particular R.

Surprisingly it was found that further substitutions, for example those listed in the ensuing paragraph, could increase the potency of the inhibitor (Tables 1, 2):
Loop the polypeptide of the invention molecules exhibiting improved inhibition of Thermolysin comprise I35L, I36F, K, R, V, Y, Q, D, H, M, T, or W, R39K or V.

Loop the polypeptide of the invention molecules exhibiting improved inhibition of Pseudolysin comprise I35L or F, M, W or Y, I36R, Q, or M, R39K and A.

Loop polypeptide of the invention molecules exhibiting improved inhibition of both, Thermolysin and Pseudolysin in parallel comprise I35L, I36R, Q, or M, R39K.

Loop polypeptide of the invention exhibiting equal potency against both, Thermolysin and Pseudolysin, comprise I35L, or W.

This result is surprising because there no reasonable expectation of success if one amino acid is altered close to an area associated with the function of the respective protein. In particular this result is surprising considering the general assumption that a peptidic inhibitor of an enzyme should contain hydrophobic amino acids binding to the enzyme close to its hydrophobic active center (Handbook of proteolytic Enzymes 2013, Biela et al. 2013) The results show, however, that hydrophobicity of the amino acids in the polypeptide of the invention is not the dominant factor for the inhibition.

It is understood that combinations of single site mutations could alter the properties of the polypeptide of the invention even further, and further increase the potency of the inhibitor.

The polypeptide of the invention may be obtained by known techniques for directed evolution as described by Arnold et al., 2003a and Arnold et al., 2003b, or may further be obtained by knowledge based engineering of the respective amino acid sequence, including application of rules for conservative or non-conservative amino acid replacement considering size, charge, polarity and other biochemical parameters of the amino acids as know by one of ordinary skill in the art by the teaching and principles disclosed in US Pat. Nos. 7,732,587 and 4,554,101, or by using suitable computer programs calculating or estimating the binding energy of the Mutant IMPIa and the selected target proteases, by assessing homologies to other proteins or the antigenicity of the protein sequence, and introducing the calculated, modified gene sequence into the bacterial genome by standard molecular biotechnology methods. Amino acid deletions within any IMPIa and its muteins may be made in regions outside the loop region (aa 35 to 39 of SEQ ID NO:2), where they are supposed to not directly interact with the target protease. Amino acid deletions may also be made within the loop region, where they are supposed to more likely modify the biological activity.

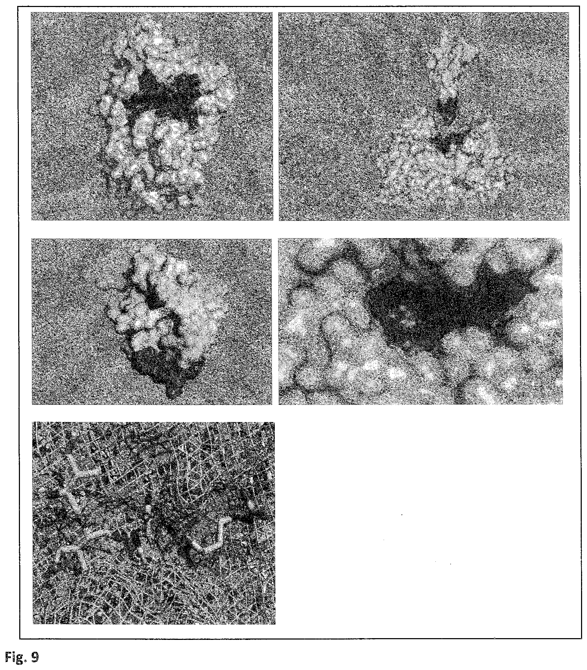

Comparison of wtIMPIα with similar proteins from other species, such as SEQ ID: NO 95 from Solenopsis, and 23 other insect related aa sequences depicted in Fig. 9 reveals that the loop region (aa 33 to 41 in SEQ ID: NO 2, and in particular in region 35 to 39) is highly conserved with the exception of aa39 (R). It is thus surprising that Loop Mutein IMPIα, for example, SEQ ID: NO 10, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84 also exhibit a strong, undexpected thermolysin inhibiting activity.

In another aspect of the present invention it has been found that wtIMPIα and Mutein IMPIα, and in particular Loop polypeptide of the invention can be recombinantly produced in bacterial cells under conditions identified by the inventors, especially at room temperature and up to 37°C, under conditions which do not require cooling of the fermenter or refolding of the protein produced. The method according to the invention, equipment and reagents used are described in detail in example 2 below. It should be understood, however, that modifications in concentrations of reagents used, and modifications in the genetic configuration of the producer organism (E.coli) could be made without affecting the output of correctly folded protein. For example, E.coli could be replaced by other bacteria as production host, additional plasmids or gene stretches with additional function could be added to the producer bacteria. Furthermore, medium components could be provided in different order or concentrations, and incubation times could be varied. It should be understood that those modifications which influence the production efficacy of biologically active Mutein or wtIMPIα negatively by 50% or less or even positively are considered within the scope of the invention.

The invention contemplates further modifications of elements of the expression system inasmuch as the expression of a functional Mutein or wtIMPIα without the need for refolding is maintained to at least 50% compared to the system disclosed in Example 3 and 4, and no cooling of the fermenter is required. For example, other expression vectors than pET-41a(+) may be used, which may not be linked to a fusion tag, or may be linked to a fusion tag other than GST, His-tag; and other promoters than T7, and may also include further regulators or other fusion elements. Depending on the operons employed, IPTG or other inducers may be employed. Other restriction sites than PshAI may be used in pET-41a(+) or in other vectors, for example XhoI, EcoR I, BamH 1. Other E.coli strains than Rosetta-gami 2 pLysS may be used, with or without additional t-RNA genes or ahp C mutation, and other bacteria than E.coli may also be employed.

Further modifications may relate to the downstream process steps which are known to the one skilled in the art, including but not limited, to incubation times, reagents and their concentrations and disposables used, for example capture resins and chromatography equipment and separation media for purification.

Recombinant production of Mutein or wtIMPIα in bacteria is very cost effective compared to all other production methods for proteins. Cloning and the subsequent development of a cell line producing the peptide are faster and less costly with bacteria as, for example, with mammalian or insect cells. During production, the amount of protein produced per volume fermentation broth is much higher compared to eukaryotic cell cultures. Consequently there is a strong demand for methods of recombinant protein producing in bacteria, especially in E.coli since they are a proven host frequently used for protein production. It is known that wtIMPIα retains its biological activity after deglycosylation (Wedde et al. 1998); therefore bacterially produced Mutein or wtIMPIα lacking glycosylation are biologically active once folded correctly.

The skilled artisan would expect, however, that a protein like Mutein or wtIMPIα exhibiting 5 cystein bridges would not be correctly folded and thus be accumulated almost entirely in inclusion bodies of the E.coli host. While several processes were developed earlier to isolate and dissolve inclusion bodies, and to denature its protein content using chaotropic salts like urea or guanidylchloride, and to withdraw the salts again in an ensuing step, for example by dialysis, to enable refolding of the protein in a kinetically controlled manner. However, the success rate of refolding proteins with multiple cystein bridges, such as as IMPIα, is usually low, and even today time consuming and resource consuming trial and error methods are required to find adequate refolding conditions. It is generally not yet possible to predict or experimentally determine suitable refolding conditions.

It is thus surprising that, without any known exception, a family of cystein rich proteins such as Mutein or wtIMPIα with a complex tertiary structure can be recombinantly produced in bacteria by the method of invention and without the need for refolding steps, in particular that the production can take place without cooling of the fermenter, the latter being a common measure to reduce the protein production rate of the bacteria and thereby to reduce the ratio of incorrectly folded protein vs. correctly folded protein.

In another aspect of the invention, the use of Mutein or wtIMPIα, and in particular of wtIMPIα is provided for inhibition of the anthrax protease nrp599. Current approaches to treat Anthrax include vaccination such as BioThrax (Emergent Biosolutions, Rockville MD, U.S.A) for disease prevention, or antibiotics such as Ciprofloxacin or Doxycyclin (http://www.nationalter-roralert.com/anthraxtreatment/). While the vaccine is only approved for preventive use, it takes several days until antibiotics exert effects on bacteria to an extent that the symptoms caused by their virulence factors are reduced. Compounds neutralizing virulence factors were developed recently and are generally well known in the state of the art and for anthrax most compounds targeting virulence factors developed so far target LF. However, no specific inhibitor of nrp599 was reported so far, although recent evidence shows that nrp599 is one of the major anthrax virulence factors inducing hemorrhagic bleeding, for example (Chung et al., 2006) by targeting the fibrinolytic system (Chung et al, 2011), and that it also degrades the van Willebrand-Factor and thereby interferes with the coagulation cascade (Chung et al., 2008).

US 2004/018193 teaches the concurrent or offset administration of both, antibiotics and antigenic compounds, in particular to treat anthrax infected humans. The authors provide several examples of protease inhibitors including hypothetical antibodies, but they do not disclose nrp599 as a target.

Surprisingly it was found that polypeptides of the invention and in particular wt IMPIα is a potent and specific inhibitor of nrp599, despite the fact that bacillus anthracis is not known to infect insects. It was found that wtIMPIα inhibits npr599 at nanomolar concentrations.

To treat anthrax infection or symptoms caused by anthrax toxins, the polypeptide of the invention and in particular wt IMPIα may be combined with antibiotics, in particular Ciprofloxacin or Doxycyclin, with antimicrobial peptides, with other inhibitors of bacterial proteases, or with compounds improving the physiological state of the infected patient.

The polypeptide of the invention may also be produced effectively in recombinant cell lines and tissue, including mammalian and insect expression systems, plant and bacterial systems; or synthetically.

Mutein and wtIMPIα each may typically be isolated and purified to be free of other proteins and protein fragments. Preferably, Mutein and wtIMPIα is about 80% free of other proteins which may be present due to the production technique used in the manufacturing process. More preferably, the polypeptide of the invention or wtIMPIα is about 90% free of other proteins, particularly preferably about 95% free of other proteins, and most preferably about >98% free of other proteins. It will be appreciated, however, that the desired protein may be combined with other active ingredients, chemical compositions and/or suitable pharmaceutical formulation materials prior to administration as medication, as described in further detail below.

The biological activity of Mutein or wtIMPIα can be assessed in vitro by means of assays, such as measuring binding of the polypeptide of the invention or wtIMPIα to a protease immobilized on coated glass carrier used for Plasmon resonance spectroscopy. Alternatively, the polypeptide of the invention or wtIMPIα can be immobilized on the glass carrier and the protease could be added to start the assay. This assay is able to measure on- and off-rates for the respective interaction.

The biological activity can also be tested by means of an Parallel Line assay, for example in a microtiter plate format, and by detecting a coloration effect or by measuring changes in the fluorescence emitted upon excitation with a suitable light source.

Amino acid sequence additions may further include N- or C-terminal fusions ranging in length from one residue to 150 or more residues, as well as internal intrasequence insertions of single or multiple amino acid residues into Mutein or wtIMPIα ranging typically from about 1 to 10 amino acids, more typically from 1 to 5 amino acids, and most typical from 1 to 3 amino acids. N-terminal additions include the addition of a methionine or an additional amino acid residue or sequence. Another example of N-terminal addition includes the fusion of a signal sequence - provided the signal sequence SEQ ID NO: 94 is not bluntly added to wtIMPIα of SEQ ID NO: 2.

Further examples for additions to Mutein or wtIMPIα comprise fusions with antibodies (Abs) or antibody fragments. Ab fragments may comprise for example scFAb, scFv fragments, or single domain antibodies, or antibodies of camelide (hcIG) or shark (IgNAR) origin, or their respective VHH domains. The fusion may serve to provide a targeting moiety (the CDR domains) fused to to Mutein or wtIMPIα so that the fusion protein could be accumulated and enriched at surface displaying antigens specific for the antibody fragments. Another motivation for fusing Mutein or wtIMPIα to antibodies may be to benefit from the dimerization properties of the constant antibody regions to obtain a bivalent form of Mutein or wtIMPIα, in a way similar to Etanercept (Enbrel) in which 2 TNF receptor fragments are fused to the constant domains of an antibody. In addition, such bivalent antibody chimera is sufficiently large to escape from rapid renal clearance, for which a size exclusion barrier of roughly 65 kDa exists, so that the lifetime of the fusion protein in the patient body is strongly prolonged. Additions may also comprise other scaffolds derived, for example, from lipocalin, ankyrin, fibronectin, transferrin, tetranectin, adnectin, albumin, uteroglobin, or protein A. Other additions contemplated comprise functional peptides, for example transferrin enabling the fusion protein to cross the blood brain barrier, a property potentially useful for inhibiting bacterial toxins accumulated in the brain or spinal cord, or additions useful for diagnostic applications, such as green fluorescent protein (GFP) enabling fluorescence detection, or peptide tags enabling immobilization on technical surfaces Specific the polypeptide of the invention described herein may involve addition or deletion of or substitution with a non-native amino acid at the N- or C-terminus or at any site of the protein that is modified by the addition of an N-linked or O-linked carbohydrate. A cystein, for example, may be added for linking a water soluble polymer such as polyethylene glycol, or other amino acids like lysine, cysteine, histidine, arginine, asparaginic acid, glutamic acid, serine, threonine, or tyrosin could also be used for coupling polymers to the peptide.. Another example is the insertion of tripeptide sequences NXT or NXS or fragments thereof with X designating any amino acid except P, which may be recognized by a cellular enzyme adding glycosylation elements. Suitable, clinically acceptable, water soluble polymers include polyethylenglycol (PEG) and polysialic acid (PSA).

Another mutation or addition may include tags, in particular peptide tags, such as hexa-histidine which can be used to facilitate binding, for example, to a moiety of a purification column. These tags may comprise a peptide sequence serving as a substrate for a protease, so that the tag can be cleaved in purpose once it is not required anymore.

Further contemplated are Mutein or wtIMPIα to which chemical compounds or nanoparticles are added. This can be achieved for example by employing the enzyme O-6-Alkylguanin-alkyltransferase (AGT) or derivatives thereof to Mutein or wtIMPIα, or to a peptide tag. Other examples comprise conjugation to a cysteine being introduced by a mutation (Jagath et al. 2008), or to carbohydrate moieties (Fischer-Durand et al. 2010). Chemical compounds may include for example labeling moieties, in particular for in vivo imaging applications, targeting moieties such as receptor ligands to enrich the conjugated IMPIα family members in specific organs. Another compound suited to immobilize conjugated IMPIα family members on a surface or particle is biotin, for example for diagnostic purposes. Nanoparticles such as quantum dots may be added for in vivo detection or imaging applications. Dendrimers or others particles increasing the size of the conjugate may be added to prevent the conjugate from cleared rapidly from the blood stream by the kidneys.

Modifications to the peptide backbone are also contemplated under the invention, such as beta peptides, where the amino group is attached to the beta carbon of the respective amino acid instead of the alpha carbon, rendering them invulnerable to proteases. Other modifications of the peptide backbone are possible, such as alkylation of the amid nitrogen and bioisosteric replacement of amide groups.

The present invention further provides polynucleotides which contain nucleotide sequences encoding Mutein or wtIMPIa, provided the polynucleotide is not identical to the complete wild type sequence SEQ ID NO: 1, or SEQ ID NO: 1 directly followed by SEQ ID NO:3. Further are considered nucleotide sequences in which oligonucleotide stretches are inserted which do not code for wtIMPIα. Based upon the present description and using the universal codon table, one of ordinary skill in the art can determine all of the nucleic acid sequences which encode Mutein or wtIMPIα. Presently preferred nucleic acid sequences include those encoding SEQ ID NO: 9, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83.

Chemical synthesis of Mutein or wtIMPIα is also contemplated, and is the obligatory route for synthesizing modified Mutein or wtIMPIα containing non-natural amino acids or backbone modifications. While chemical synthesis was traditionally regarded as being too expensive for larger peptides, recent advances in synthesizing technology like microwave assisted synthesis have recently shifted this limit towards larger proteins. Recent approaches also solve the issue of correct configuration of currently up to 3 cystein bridges by using several distinct protective groups in parallel.

While the invention contemplates the use of E.coli for producing Mutein or wtIMPIα with high quality and efficiency, other hosts may also be used for recombinantly producing Mutein or wtIMPIα, including other strains of E.coli, other bacteria like bacillus megaterium, fungal systems like yeast, pichia, or hansenula, eukaryotic cells like protozoa, insect cell lines like Schneider T7 or Sf9 mammalian animal cell lines like CHO, human cell lines like HEK293, or transgenic animals like goats, or plants like tobacco.

Mutein or wtIMPIα or modified versions thereof may be combined with ingredients to form a pharmaceutical composition. The pharmaceutical composition may include water and salts at physiological concentrations, solubilizing or dispersing agents, or anti-oxidant, or particles forming micelles, such as liposomes. Liposomes may also contain baIMPIα or modified versions thereof internally. This pharmaceutical composition may be filled in a glass or plastic vial, or in a syringe. The pharmaceutical composition may also contain additives supporting drying or freeze-drying of the pharmaceutical composition, for example cyclodextrins or saccharides, in particular disaccharides.

Mutein or wtIMPIα or modified versions thereof may be used as a medicine, in particular as a medicine for parenteral injection, to treat diseases in an animal, in particular in a mammal or bird, or in a human, and which are related to the activity of proteases from the M4 family. Such disease could be, for example, a bacterial infection, in particular infection by staphylococcus aureus, in particular multi-resistant staphylococcus aureus, bacillus anthracis, pseudomonas aeruginosa, helicobacter pylori, vibrio cholerae, or legionella pneumophilia. Such a disease could further be systemic inflammatory response syndrome (SIRS), or sepsis

Mutein or wtIMPIα or modified versions thereof may be administered parenterally, orally, or topically using suitable pharmaceutical compositions, or attached to a patch or wound debridement from where the medication elutes into a wound of the patient.

Mutein or wtIMPIα or modified versions thereof may be administered in combination with other treatments, in parallel or staggered, in particular with other inhibitors of virulence factors, antibiotics, or antimicrobial peptides.

Further contemplated are RNA molecules whose sequences comprise RNA sequences of Mutein or wtIMPIα, in particular sequences like SEQ ID 1, 5, 9, 11, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, and which can be transcribed into Mutein or wtIMPIα protein in the target organism. Such RNA molecules may be administered in combination with transfection agent like liposomes or PEI.

The invention also contemplates the application of Mutein or wtIMPIα in devices for diagnostic purposes. In several cases it is beneficial to determine the protease activity in biological fluids, and in particular the protease activity profile if several of them are present.

For fluids contaminated with bacteria determining the protease activity would provide information not only about the presence of bacteria, but also of their activity and relative concentrations.

In wounds, and especially in slowly healing or chronic wounds, determining the protease activity profile also is of interest since the physiological stages of wound healing are characterized also by a specific evolution of protease activity during healing. It is even more important to distinguish between the activity of endogenous host proteases and the activity of bacterial proteases.

PCR or real time PCR assays do not provide sufficient information because these tests quantify the concentration of particular bacteria present, but they are not suited to test for the activity of the bacteria.

Currently laboratory tests for protease activity exist where the cleaved protease substrate becomes fluorigenic. Many of these tests determine overall protease presence; some of them are able to profile the protease concentrations (Chen et al. 2013). Currently the only Point-of-Care test for protease activity, Woundchek® manufactured by Systagenix, (Gatwick, GroBbritannien) measures overall protease activity, but cannot quantify the concentration of each protease species separately (Strohal et al. 2012).

A diagnostic method according to the invention solves this issue. Mutein or wtIMPIα is used therein to bind M4 proteases specifically. Different concentrations of proteases can be determined when more than one type of Mutein or wtIMPIα is used. The difference in binding strength between the polypeptide of the invention or wtIMPIα is then used to calculate differences in concentrations of Mutein or wtIMPIα.

A preferred embodiment of the diagnostic method uses a device similar to Lateral Flow tests known in the state of the art; in which a membrane strip (suagfähig) is used to take up the fluid sample and to transport the sample across the membrane by capillary forces. In a preferred embodiment, the membrane consists of nitrocellulose or nitrocellulose endorsed by nylon fibres or tissue. At a particular location on that strip, Mutein or wtIMPIα are immobilized so that at least part of the sample fluid passes across this location during flow. Proteases present in the sample are captured by the immobilized Mutein or wtIMPIα. In a second step one further solution containing detection capture molecules targeted against different binding epitopes of the proteases are added. These detection capture molecules are prepared to deliver a detection signal, which can be, for example, chromophoric, fluorescent, or electrochemical by means of a direct or enzymatic label. It is possible to increase signal strength by secondary capture molecules targeting the detection capture molecule. In a preferred embodiment, at least one kind of capture molecule is an antibody. A particular advantage of the diagnostic method according to the invention is that several proteases can be distinguished by means of specific binding to speiific locations where different Mutein or wtIMPIα are immobilized, while only a single sort of antibody is required. Furthermore, a control area is present at a different location on the membrane strip where detection capture molecules or secondary capture molecules bind to even if no proteases are present in the sample. In a preferred embodiment, the capture functionality in the control area is provided by immobilized proteases or immobilized detection capture molecules.

In a different embodiment, capture molecules other than Mutein or wtIMPIα, in particular antibodies are immobilized on the membrane strip and the proteases are added as detection capture molecule, in particular in combination with secondary capture molecules. This embodiment also requires a contral area containing immobilized proteases or Mutein or wtIMPIα.

The assessment of the protease content is made by visual inpection or a sensor device, including CMOS based imaging sensor or scanners. In case that sensors are used, a software can be employed to calculate the true concentration ratios of the proteases from potentially overlapping binding profiles of the polypeptide of the invention or wtIMPIα if the individual binding constants or other binding parameters are known. In particular such software may use a linear equation system to calculate the individual concentrations.

In another preferred embodiment, the binding kinetics are sampled, in particular by using an imaging sensor, to determine concentrations more accurately.

The sample may be added, for example, by a pipetting device, a paper strip, a cotton swab or a needle. The device may dispose of a seal preventing uncontrolled flow across the membrane strip.

**Table 1**

| | | **Pos39** | | | | | | | | | | | | | |
|---|---|---|---|---|---|---|---|---|---|---|---|---|---|---|---|
| | wt (avg) | RnG | RnK | RnL | RnV | RnW | RnF | RnI | RnM | RnA | RnQ | RnT | RnY | | |
| IC50 mutein TLN | | 347,031 | 7,78 | 58,729 | 9,244 | 64,356 | 245,863 | 87,136 | 24,551 | 18,073 | 56,329 | | | | |
| IC50 wt TLN | 12,53 | 12,728 | 12,524 | 12,41 | 12,538 | 12,362 | 12,489 | 12,548 | 12,571 | 12,542 | 12,605 | | | | |
| IC50 mutein PLN | | 1053,848 | 11,373 | 587,631 | 139,828 | 525,002 | 95,622 | 384,625 | 85,036 | 13,284 | 105,717 | 26,032 | 10000 | | |
| IC50 wt PLN | 21,39 | 20,318 | 21,987 | 20,102 | 21,757 | 19,948 | 20,523 | 21,168 | 22,142 | 22,076 | 22,145 | 21,943 | 22,526 | | |
| ratio TLN:PLN | **0,59** | **0,33** | **0,68** | **0,10** | **0,07** | **0,12** | **2,57** | **0,23** | **0,29** | **1,36** | **0,53** | | | | |
| | | | | | | | | | | | | | | | |
| | | | | | | | | | | | | | | | |

| | | **Pos38** | | | | | | | | | | | | | |
|---|---|---|---|---|---|---|---|---|---|---|---|---|---|---|---|
| | wt(avg) | InF | InG | InQ | InL | InV | InH | InM | InW | InY | | | | | |
| IC50 mutein TLN | | 38,331 | 144,113 | 267,172 | 92,044 | 26,282 | 336,219 | 210,619 | 571,873 | 5,625 | | | | | |
| IC50 wt TLN | 12,52 | 12,571 | 12,481 | 12,614 | 12,563 | 12,563 | 12,491 | 12,478 | 12,375 | 12,553 | | | | | |
| IC50 mutein PLN | | 86,095 | 484,676 | 291,099 | 273,497 | 35,181 | 777,328 | 280,957 | 128,451 | 10000 | | | | | |
| IC50 wt PLN | 21,80 | 21,085 | 22,796 | 22,201 | 21,734 | 21,523 | 23,139 | 21,397 | 21,148 | 21,148 | | | | | |
| ratio TLN:PLN | **0,57** | **0,45** | **0,30** | **0,92** | **0,34** | **0,75** | **0,43** | **0,75** | **4.45** | **0,00** | | | | | |
| | | | | | | | | | | | | | | | |
| | | | | | | | | | | | | | | | |

| | | **Pos37** | | | | | | | | | | | | | |
|---|---|---|---|---|---|---|---|---|---|---|---|---|---|---|---|
| | wt(avg) | NnC | NnD | NnE | NnG | NnH | NnQ | NnS | NnY | NnA | NnM | NnP | NnV | NnW | NnL |
| IC50 mutein TLN | | 307,492 | 1379,887 | 183,989 | 590,669 | 198,251 | 10000 | 488,91 | 179,264 | 5000 | 428,764 | 749,35 | 227,708 | 250,085 | 97,854 |
| IC50 wt TLN | 12,37 | 12,674 | 12,136 | 12,644 | 12,649 | 12,78 | 12,553 | 12,499 | 12,546 | 12,553 | 11,678 | 11,982 | 12,679 | 12,674 | 11,097 |
| IC50 mutein PLN | | 332,781 | 45,714 | 5000 | 68,213 | 174,993 | 310,997 | 176,936 | 296,224 | 5000 | 136,086 | 137,03 | 234,271 | 125,939 | 137,03 |
| IC50 wt PLN | 22,17 | 20,731 | 22,607 | 21,945 | 21,827 | 21,18 | 22,224 | 21,472 | 21,501 | 21,945 | 30,792 | 20,761 | 21,194 | 21,464 | 20,761 |
| ratio TLN:PLN | **0,56** | **0,92** | **30,19** | **0,04** | **8,66** | **1,13** | **32,15** | **2,76** | **0,61** | **1,00** | **3,15** | **5,47** | **0,97** | **1,99** | **0,71** |
| | | | | | | | | | | | | | | | |
| | | | | | | | | | | | | | | | |

| | | **Pos36** | | | | | | | | | | | | | |
|---|---|---|---|---|---|---|---|---|---|---|---|---|---|---|---|
| | wt(avg) | InA | InF | InG | InK | InR | InV | InY | InQ | InD | InE | InH | InM | InT | InW |
| IC50 mutein TLN | | 13,887 | 11,627 | 10000 | 5,656 | 3,816 | 11,119 | 10,893 | 10,371 | 10,599 | 43,488 | 5,344 | 4,795 | 10,841 | 10,599 |
| IC50 wt TLN | 12,51 | 12,408 | 12,555 | 12,553 | 12,545 | 12,581 | 12,551 | 12,553 | 12,552 | 12,53 | 12,526 | 12,379 | 12,415 | 12,432 | 12,53 |
| IC50 mutein PLN | | 74,4 | 105,107 | 28,916 | 45,25 | 16,069 | 22,275 | 34,934 | 15,404 | 47,007 | 5000 | 71,648 | 11,097 | 103,341 | 62,954 |
| IC50 wt PLN | 21,91 | 22,009 | 22,338 | 21,56 | 22,028 | 22,261 | 22,087 | 22,167 | 21,925 | 20,857 | 21,56 | 22,032 | 21,961 | 21,923 | 22,076 |
| ratio TLN:PLN | **0,57** | **0,19** | **0,11** | **345,83** | **0,12** | **0,24** | **0,50** | **0,31** | **0,67** | **0,23** | **0,01** | **0,07** | **0,43** | **0,10** | **0,17** |
| | | | | | | | | | | | | | | | |
| | | | | | | | | | | | | | | | |

| | | **Pos35** | | | | | | | | | | | | | |
|---|---|---|---|---|---|---|---|---|---|---|---|---|---|---|---|
| | wt (avg) | InA | InF | InL | InV | InM | InW | InY | | | | | | | |
| IC50 mutein TLN | | 34,34 | 13,26 | 9,06 | 14,93 | 12,55 | 14,10 | 16,25 | | | | | | | |
| IC50 wt TLN | 12,57 | 12,58 | 12,58 | 12,57 | 12,60 | 12,55 | 12,58 | 12,55 | | | | | | | |
| IC50 mutein PLN | | 259,77 | 16,27 | 8,26 | 63,55 | 8,22 | 13,92 | 12,43 | | | | | | | |
| IC50 wt PLN | 21,98 | 21,81 | 21,95 | 21,91 | 22,19 | 21,94 | 22,12 | 21,95 | | | | | | | |
| ratio TLN:PLN | **0,57** | **0,13** | **0,81** | **1,10** | **0,23** | **1,53** | **1,01** | **1,31** | | | | | | | |

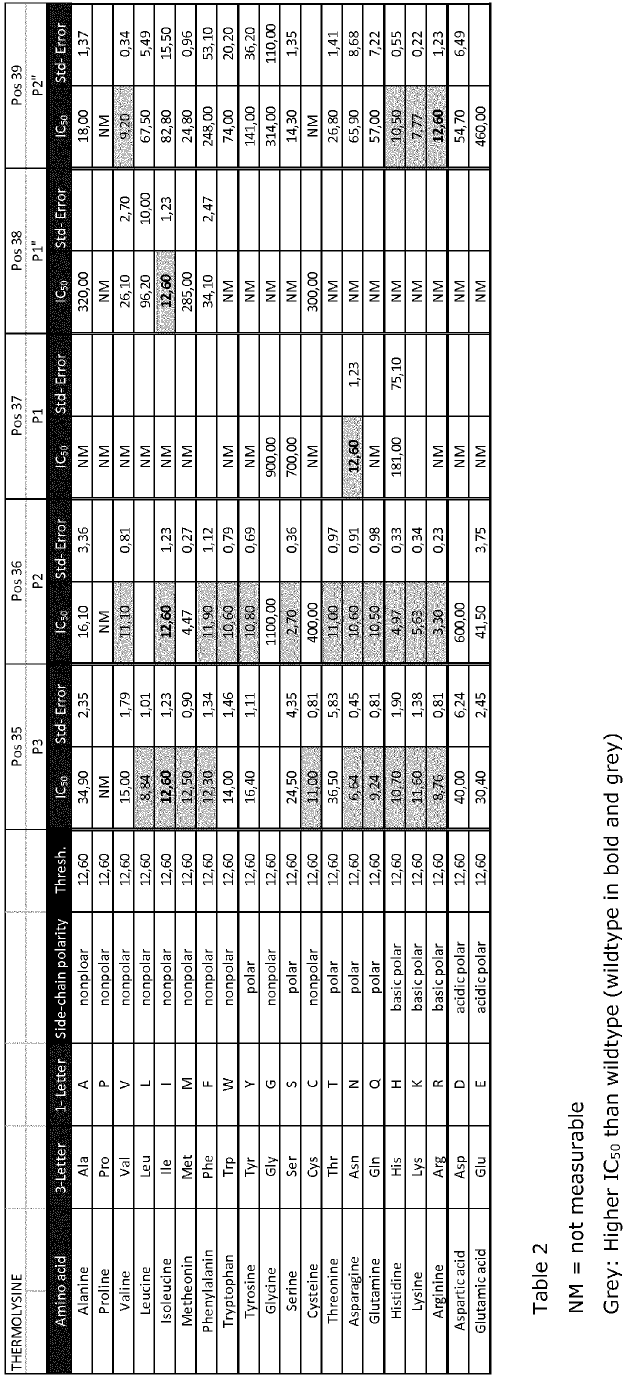

### EXAMPLE 1

### Total RNA isolation and cDNA synthesis

Last instar larvae were used for immunization using the following solutions: (4.05*10⁴ cfu/ml) *Escherichia coli* strain BL21 (DE3) (Invitrogen, Carlsbad,CA) and (0.7*10⁴ cfu/ml) *Micrococcus luteus* (DMSZ Reference number 495). Ten microliters of sample volume from each solution was injected dorsolaterally into the hemocoel using 1-ml disposable syringes and 0.4-mm needles mounted on a microapplicator. Larvae were homogenized at 8 h postinjection for total RNA isolation using the TRIzol reagent (Invitrogen, Germany) according to manufacturer's instructions. cDNA was synthesized using the OneStep RT-PCR System (Qiagen, Germany) according to the manufacturer's instructions.

### EXAMPLE 2

### preparation of IMPIs (Expression and purification)

1. Construction of a heterologous expression system
   The T7-based expression system, pET-41a(+), which allows protein expression upon induction of the T7-polymerase in *Escherichia coli* cells by IPTG, was chosen to obtain strong and reproducible expression of the recombinant protein. The sequence of the mature IMPIa was amplified using the forward primer (5'-GATAGTCCTAATTTGTAACGGTGGACAC-3') and the reverse primer (5'- CTAC-GAACGTATTTTAGGACAGTCTTTTATCG-3'). The fragment was cloned into the *PshAI* site of vector pET-41a(+), which was then designated p41IMPIa and transformed into *E.coli* Rosetta-gami 2 pLysS (Novagen, Germany). The resulting clones were verified via sequencing (Eurofins MWG Operon, Germany)
2. Expression and production of recombinant IMPIa
   i. Preparatory Culture: LB-medium containing 1% (w/v) Glucose, Cm₃₄, Kan₅₀ und Tc_{12,5} was inoculated with cell material from a colony and cultivation started on an orbital shaker at 32°C up to a OD₆₀₀ of 1. The preparatory culture was subsequently stored at 4°C in a refrigerator.
   ii. Main culture: LB-Medium containg 1% (w/v) Glucose, Cm₃₄, Kan₅₀, was inoculated with 3 %(v/v) of the preparatory culture and cultivation started on an orbital shaker at 32°C up to a OD₆₀₀ of 1. Upon reaching an OD₆₀₀ of 1 the expression of the recombinant protein was induced with 1 mM IPTG. The main culture was subsequently incubated for further 3 hours at 32°C on an orbital shaker.
   iii. Cells were harvested from liquid culture by centrifugation at 10,000 g for 10 min using a centrifuge tube of known weight. The pellet was decanted and allowed to drain, removing a maximum amount of liquid before the wet weight of the pellet was determined.
   iv. The cell pellet was resuspended in BugBuster Reagent (Novagen, Germany) at room temperature by pipeting and gentle vortexing. 5 ml reagent per g wet cell paste was used. 1 µl (25 units) Benzonase® Nuclease (Novagen, Germany) per 1 ml of BugBuster Reagent was added.
   v. The suspension obtained in step(iv) of this protocol was incubated at room temperature on a shaking platform at a slow setting for 20 min. Care was taken that the extract was not viscous at the end of the incubation.
   vi. Insoluble cell debris was removed by centrifugation at 16,000 × g for 20 min at 4°C.
   vii. The supernatant was transferred to a fresh tube. The soluble extract was applied directly to GST●Bind™ Resin (Novagen, Germany).
   viii. Affinity chromatography was performed according to manufacturer's instructions (GE Healthcare Life Sciences.
3. Main culture: LB-Medium containg 1% (w/v) Glucose, Cm₃₄, Kan₅₀ was inoculated with 3 %(v/v) of the preparatory culture and cultivation started on an orbital shaker at 32°C until an OD₆₀₀ of 1 was reached. At 1 OD the expression of the recombinant protein was induced with 1 mM IPTG. The main culture was subsequently incubated for further 3 hours at 32°C on an orbital shaker.

### EXAMPLE 3

### Bioinformatics Analysis

Protein motifs were identified using SMART (http://smart.embl-heidelberg.de/) and the Conserved Domain Database from NCBI (http://ncbi:nlm.nih.gov/Structure/cdd/wrpsb.cgi). The signal peptide was predicted using SignalP (http://www.cbs.dtu.dk/services /SignaIP/), and the theoretical isolelectric point and molecular weight were predicted using Compute pI/MW (http://expasy.org/tools/protparam.html). Cysteine disulfide bonding state and connectivity prediction was done by using DISULFIND (http://disulfind.dsi.unifi.it/). Sequence similarity was analysed by BLAST from NCBI (http://www.ncbi.nlm.nih.gov/BLAST/). Multi-sequence alignment was generated using Vector NTI 9.0 (Invitrogen, Germany). Homology modeling was performed by the CPHmodels 3.0 server (http://www.cbs.dtu.dk/services/CPHmodels/) using the Swiss-Model server (http://swissmodel.expasy.org/) for structure assessment. All models were energy minimized by using GROMOS force field (http://www.gromacs.org/Documentation/Terminology/Force_Fields/GROMOS).

The subsequent table lists the sequences printed in the ensuing sequenc protocol. The leading number denotes the SEQ ID Nr. for the nucleotide sequence, the subsequent even number mssing number would denote the SEQ ID of the respective peptide sequence.
- 1: IMPIalpha (wild type or wtIMPIalpha)
- 3: IMPIbeta (wild type or wt IMPIbeta)
- 5: GST/IMPIalpha
- 7: GST/IMPIbeta
- 9 :: IMPIalpha Pos37 NnG Pos38 InL Pos39 RnA
- 11 :: GST/IMPIalpha Pos37 NnG Pos38 InL Pos39 RnA
- 13 :: IMPIalpha Pos52 RnK
- 15 :: GST/IMPIalpha Pos52 RnK
- 17 :: IMPI Pos35 InL
- 19 :: IMPI Pos35 InM
- 21 :: IMPI Pos35 InF
- 23 :: IMPI Pos35 InC
- 25 :: IMPI Pos35 InN
- 27 :: IMPI Pos35 InQ
- 29 :: IMPI Pos35 InH
- 31 :: IMPI Pos35 InK
- 33 :: IMPI Pos35 InR
- 35 :: IMPI Pos36 InV
- 37 :: IMPI Pos36 InM
- 39 :: IMPI Pos36 InF
- 41 :: IMPI Pos36 InW
- 43 :: IMPI Pos36 InY
- 45 :: IMPI Pos36 InS
- 47 :: IMPI Pos36 InT
- 49 :: IMPI Pos36 InN
- 51 :: IMPI Pos36 InQ
- 53 :: IMPI Pos36 InH
- 55 :: IMPI Pos36 InR
- 57 :: IMPI Pos36 InK
- 59 :: IMPI Pos39 InV
- 61 :: IMPI Pos39 InK
- 63 :: IMPI Pos35 InW
- 65 :: IMPI Pos35 InY
- 67 :: IMPI Pos39 RnA
- 69 :: IMPI Pos35 InC
- 71 :: IMPI Pos35 InK
- 73 :: IMPI Pos35 InR
- 75 :: IMPI Pos35 InL
- 77 :: IMPI Pos35 InM
- 79 :: IMPI Pos35 InF
- 81 :: IMPI Pos35 InQ
- 83 :: IMPI Pos35 InH
- 85 :: GST/IMPI Pos35 InN
- 87 :: GST/IMPI Pos36 InS
- 89 :: GST/IMPI Pos39 InK
- 91 :: GST/IMPI Pos35 InC
- 93 :: IMPI signal peptide,
- 95 :: IMPI like (Solenopsis, Peptide only, no nucleotide sequence provided)

### References

Adekoya O, Sylte I. The thermolysin family (M4) of enzymes: therapeutic and biotechnological potential. Chem Biol Drug Des 2009; 73:7-16.
Armstrong PB. Proteases and protease inhibitors: a balance of activities in host-pathogen interaction. Immunobiol 2006; 211:263-81.
Arnold F.H., Georgiou G. (Eds.) " Directed Enzyme Evolution: Screening and Selection Methods (Methods in Molecular Biology) Springer 2003, ISBN-13 978-1588292865
Arnold F.H., Georgiou G. (Eds.) " Directed Evolution Library Creation: Methods and Protocols (Methods in Molecular Biology) Springer 2003, ISBN-13 978-1588292858
Barret A. J., Rawling N.D. Woessner J.F. (Eds.) (2004) Handbook of Proteolytic Enzymes, Academic Press, Inc., ISBN-13 978-0120796106.
Biela A., Nasief N.N., Betz M., Heine A., Hangauer D., Klebe G. "Dissecting the Hydrophobic Effect on the Molecular Level: The Role of Water, Enthalpy, and Entropy in Ligand Binding to Thermolysin" Angew. Chem. Int. Ed. 2013, 52, 1822 - 1828 2013 Wiley-VCH Verlag GmbH & Co. KGaA, Weinheim
Cathcart G.R., Greer B., Harriott P., Lynas J.F., Gilmore B.F., Walker B. "Novel Inhibitors of the Pseudomonas aeruginosa Virulence Factor LasB: a Potential Therapeutic Approach for the Attenuation of Virulence Mechanisms in Pseudomonal Infection" Antimicrob Agents Chemother. 2011 Jun;55(6):2670-8
Ceroni A., Passerini A., Vullo A., Frasconi P." DISULFIND: a Disulfide Bonding State and Cysteine Connectivity Prediction Server", Nucleic Acids Research, 34 (2006) W177--W181
Chen, Ch.-H., Miller, M. A., Sarkar A.,Beste M. T., Isaacson K. B., Lauffenburger D. A., Griffith L. G. and Han J. "Multiplexed Protease Activity Assay for Low-Volume Clinical Samples Using Droplet-Based Microfluidics and Its Application to Endometriosis", JACS 2013, 135 (5), pp 1645-1648, DOI: 10.1021/ja307866z
Chung M., Popova T.G., Millis B.A., Mukherjee D.V., Zhou W., Liotta L.A., Petricoin E.F., Chandhoke V., Bailey Ch., Popov S.G., "Secreted Neutral Metalloproteases of Bacillus anthracis as Candidate Pathogenic Factors" JBC 281, 42, (2006) 31408-31418, DOI 10.1074/jbc.M605526200
Chung M., Popova T.G., Jorgensen S.C., Dong L., Chandhoke V., Bailey Ch. L.,Popov S.G., "Degradation of Circulating von Willebrand Factor and Its Regulator ADAMTS13 Implicates Secreted Bacillus anthracis Metalloproteases in Anthrax Consumptive Coagulopathy" JBC 283, 15, (2008). 9531-9542, DOI 10.1074/jbc.M705871200
Chung M., Jorgensen S.C., Tonry J.H., Kashanchi F.., Bailey Ch. L.,Popov S.G., "SecretedBacillus anthracis proteases target the host ¢brinolytic system" FEMS Immunol Med Microbiol 62 (2011) 1-9, DOI:10.1111/j.1574-695X.2011.00798.x Clermont A, Wedde M, Seitz V, Podsiadlowski L, Hummel M, Vilcinskas A. Cloning and expression of an inhibitor against microbial metalloproteinases from insects (IMPI) contributing to innate immunity. Biochem J 382 (2004);:315
Duthie E.S. and Lorenz L. "Protease Inhibitors 2.Bacterial Proteases and their inhibitors" Biochem J. 44(1949) 173 - 178
Fischer-Durand N., Salmain M., Rudolf B, Dai L., Jugé L., Guérineau V., Laprévote O., Vessières A, Jaouen G. "Site-specific conjugation of metal carbonyl dendrimer to antibody and its use as detection reagent in immunoassay" Analytical Biochemistry 407(2) (2010), 211-219, doi:10.1016/j.ab.2010.08.027
Handbook of Proteolytic Enzymes, 3rd Edn chapter 111, pp 540 - 553, 2013 Elsevier Ltd. ISBN: 978-0-12-382219-2 DOI: http://dx.doi.org/10.1016/B978-0-12-382219-2.00111-3
Hiraga K, Seeram SS, Tate S, Tanaka N, Kainosho M, Oda K Mutational analysis of the reactive site loop of Streptomyces metalloproteinase inhibitor, SMPI. J Biochem. 1999 Jan;125(1):202-9.
Holden H.M., Tronrud D.E., Monzingo A.R., Weaver L.H. and Matthews B.W. Slow- and Fast-Binding Inhibitors of Thermolysin Display Different Modes of Binding: Crystallographic Analysis of Extended Phosphonamidate Transition-State Analogues Biochemistry 26(1987) 8542-8553
Junutula J.R.,.Raab H., Clark S, Bhakta S. Leipold D.D. Weir S., Chen Y, Simpson M., Tsai S.P,. Dennis M.S., Lu Y., Meng Y.G., Ng C., Yang J., Lee Ch.C., Duenas E., Gorrell J., Katta V., Kim A., McDorman K., Flagella K., Venook R., Ross S., Spencer S.D., Wong W.L., Lowman H.B., Vandlen R., Sliwkowski M.X., Scheller R.H., Polakis P. Mallet W. "Site specific conjugation of a ytotoxic drug to an antibody improves the therapeutic index" Nature Biotechnology 26 (2008) 925 - 932 doi:10.1038/nbt.1480
Khan M.T.H., Fuskevaag O.M. and Sylte I. Discovery of Potent Thermolysin Inhibitors Using Structure Based Virtual Screening and Binding Assays. Journal of Medicinal Chemistry 52 (2009) 48-61
Khan M.T.H., Khan R., Wuxiuer Y., Arfan M., Ahmed M. and Sylte I. Identification of novel quinazolin-4(3H)-ones as inhibitors of thermolysin, the prototype of the M4 family of proteinases. Bioorganic & Medicinal Chemistry 18 (2010) 4317-4327
Oda k., Koyama T. and Murao S. Purification and properties of a proteinaceous metallo-proteinase inhibitor from Streptomyces nigrescens TK-23, Biochim. Bio-phys. Acta 571 (1979), pp. 147-156
Otto M. "Bacterial Sensing of Antimicrobial Peptides", in Collin M, Schuch R (eds): Bacterial Sensing and Signaling. Contrib Microbiol.16, Basel, Karger, 2009,136-149 DOI: 10.1159/00021937
Panchal R.G., Hermone A.R., Nguyen T.L., Wong T.Y., Schwarzenbacher R., Schmidt J., Lane D., McGrath C., Turk B.E., Burnett J., Aman M.J., Little S., Sausville E.A., Zaharevitz D.W., Cantley L.C., Liddington R.C., Gussio R., Bavari S., "Identification of small molecule inhibitors of anthrax lethal factor" Nature Structural & Molecular Biology 11, 67 - 72 (2004) doi: 10.1038/nsmb711
Peinado J.R., Kacprzak M.M., Leppla S.H., Lindberg I., "Cross-inhibition between furin and lethal factor inhibitors", Biochemical and Biophysical Research Communications (2004)321 (3), 601-605 DOI: 10.1016/j.bbrc.2004.07.012
Popov S.G., Popova T.G., Hopkins S., Weinstein R.S., MacAfee R., Fryxell K.J., Chandhoke V., Bailey Ch., Alibek K. "Effective antiprotease-antibiotic treatment of experimental Anthrax" BMC Infectious Diseases 2005, 5:25 doi:10.1186/1471-2334-5-25
Potempa J, Robert NP. Corruption of innate immunity by bacterial proteases. J Innate Immun 2009; 1:70-87
Roy J., Kumar U.Ch., Machiraju P.K., Muttineni R.K., Kumar S.B.V.S., Gundla R., Dayam R., Jagarlapudi S.A.R.P. "Insilico studies on anthrax lethal factor inhibitors: Pharmacophore modeling and virtual screening approaches towards designing of novel inhibitors for a killer" Journal of Molecular Graphics and Modelling 29, 2, 2010, 256-265 doi:10.1016/j.jmgm.2010.07.002
Schmidtchen A., Holst E., Tapper H., Bjorck, L. Elastase-producing Pseudomonas aeruginosa degrade plasma proteins and extracellular products of human skin and fibroblasts, and inhibit fibroblast growth Microb. Pathog. 34 (2003) 47-55
Smith A. W., Chahal B., French G. L. The human gastric pathogen Helicobacter pylori has a gene encoding an enzyme first classified as a mucinase in Vibrio cholerae Mol. Microbiol. 13 (1994) 153-160
Snell K., Holder I.A., Leppla S.A. and Saelinger C.B. Role of Exotoxin and Protease as Possible Virulence Factors in Experimental Infections with Pseudomonas aeruginosa Infection and Immunity 19 (1978) 839-845
Strohal R. Dissemond J., Hastermann G., Herberger K.,Läuchli S., Luch G., Mayer D.,Neubert T., Storck M. "The role of a point-of-care protease test in wound diagnostics", WundManagement 2012, pp 9-11.
Tonello F., Seveso M., Marin O., Mock M., Montecucco C., "Pharmacology: Screening Inhibitors of Anthrax Lethal Factor" Nature 418, 386 (July 2002) doi: 10.1038/418386a
Tronrud, D.E. , Holden, H.M., and Matthews, B.W. " Structures of Two Thermolysin-Inhibitor Complexes That Differ by a Single Hydrogen Bond" Science 235 (1987)571 - 574
Tsuru D, Fujita Y, Morikawa S, Ito K, Yoshimoto T. "Degradation of streptomyces metalloprotease inhibitor (SMPI) by neutral protease from Bacillus subtilis var. amylosacchariticus" Biosci Biotechnol Biochem. 1992 Aug;56(8):1275-8.
Turk B.E., Wong T.Y., Schwarzenbacher R.; Jarrell E.T., Leppla S.H., Collier R.J., Liddington R.C., Cantley L.C., "The structural basis for substrate and inhibitor selectivity of the anthrax lethal factor" Nature Structural & Molecular Biology 11, 60 - 66 (2003) doi: 10.1038/nsmb708
Vilcinskas A. Coevolution between pathogen-derived proteinases and proteinase inhibitors of host insects. Virulence 2010; 1(3): 206-14
Wedde M, Weise C, Kopacek P, Franke P, Vilcinskas A. Purification and characterization of an inducible metalloprotease inhibitor from the hemolymph of greater wax moth larvae, Galleria mellonella. Eur J Biochem 1998; 255:534-43
Xiong Y., Wiltsie J., Woods A., Guo J., Pivnichny J.V., Tang W., Bansal A., Cummings R.T., Cunningham B.R., Friedlander A.M., Douglas C.M., Salowe S.P., Zaller D.M., Scolnick E.M., Schmatz D.M., Bartizal K., Hermes J.D., MacCoss M., Chapman K.T., "The discovery of a potent and selective lethal factor inhibitor for adjunct therapy of anthrax infection", Bioorganic & Medicinal Chemistry Letters 16(4) 2006, 964-968, doi:10.1016/j.bmcl.2005.10.088

## Claims

1. A polypeptide having at least 70% homology, in particular 80%, 90% or 95% homology to the polypeptide of SEQ ID No 2 representing the wild-type of the protein insect metalloproteinase inhibitor IMPIα and having at least one mutation at position 35, 36 and/or 39 of the amino acid sequence of SEQ ID No 2 and the polypeptide having an IC₅₀ value to thermolysine of less than the IC₅₀ value of IMPIα wherein
- the nonpolar amino acid isoleucine at position 35 of IMPIα is replaced either by a nonpolar amino acid selected from the group consisting of leucine, methionine and phenylalanine or by polar amino acid selected from the group consisting of cysteine, asparagine, glutamine, histidine, lysine and arginine; and/or
- the nonpolar amino acid isoleucine at position 36 of IMPIα is replaced either by a nonpolar amino acid selected from the group consisting of valine, phenylalanine and tryptophan or by polar amino acid selected from the group consisting of tyrosine, serine, threonine, asparagine, glutamine, histidine, lysine and arginine; and/or
- the polar amino acid position 39 of IMPIα is replaced either by the nonpolar amino acid valine or by the polar amino acids histidine or lysine.

2. The polypeptide of claim 1 having the amino acid sequences of SEQ ID numbers 10, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84.

3. The polypeptide of claim 1 or 2 having chemical modifications in the side chain or at the N and/or C terminal for improving biological or chemical properties such as bio availability, stability, effectivity or comprising a detectable label.

4. A fusion polypeptide comprising a polypeptide of at least one of the claims 1 to 3 fused with at least one polypeptide having a physiological function selected from the group consisting of an antibody or antibody fragment, scaffolds such as lipocalin, ankyrin, fibronectin, transferrin, tetranectin, adnectin, albumin, uteroglobin, or protein A; functional peptides such as transferrin; peptides useful for diagnostic applications, such as green fluorescent protein; or peptide tags enabling immobilization on technical surfaces, such as hexahistidine; or a transferase such as glutathione-S-transferase.

5. The fusion polypeptide of claim 4 being linked by a peptide of 1 to 100 amino acids in length.

6. The fusion polypeptide of claim 4 wherein a chemical linking group conjugates the polypeptide of at least one of the claims 1 to 3 and the at least one polypeptide having a physiological function.

7. A polynucleotide coding for the polypeptide of at least one of the claims 1 or 2 or the fusion polypeptide of claim 5.

8. The polynucleotide of claim 7 operably linked to the heterologous promoter.

9. A host cell comprising the recombinant nucleic acid of claim 7 or 8.

10. A method of producing a polypeptide comprising culturing the host cell of claim 9, expression of said nucleic acid in the host cell and isolation of the polypeptide.

11. A kit for diagnosis comprising a peptide of claim 3 and/or a fusion polypeptide of anyone of the claims 4 to 6.

12. A pharmaceutical composition comprising a polypeptide according to claim 1 and/or the fusion polypeptide of at least one of the claims 4 to 6 or a polynucleotide comprising a section coding for at least one of the polypeptides of claim 1 to 6.

13. The polypeptide according to claim 1 or the fusion polypeptide of anyone of the claims 4 to 6 or the polynucleotide of claim 7 for use in the treatment of an animal or human infected by microorganisms secreting bacterial toxins of the M4 or Metzincin family of metalloproteinases, in particular thermolysine, aureolysin, bacillolysin, pseudolysin, vibriolysin or anthrax npr599.

14. Use of a polypeptide of claim 3 and/or a fusion polypeptide of anyone of the claims 4 to 6 for detection of the presence or activity of proteases belonging to the M4 family in a sample.

## Patentansprüche

1. Ein Polypeptid mit mindestens 70% Homologie, insbesondere 80%, 90% oder 95% Homologie zu dem Polypeptid der SEQ ID Nr. 2, das den Wildtyp des Protein-Insektenmetalloproteinase-Inhibitors IMPIα darstellt und mindestens eine Mutation an Position aufweist 35, 36 und / oder 39 der Aminosäuresequenz von SEQ ID Nr. 2 und das Polypeptid weist einen IC₅₀-Wert gegenüber Thermolysin von weniger als dem IC₅₀-Wert von IMPIα auf, wobei
- die unpolare Aminosäure Isoleucin an Position 35 von IMPIα entweder durch eine unpolare Aminosäure ausgewählt aus der Gruppe bestehend aus Leucin, Methionin und Phenylalanin oder durch eine polare Aminosäure ausgewählt aus der Gruppe bestehend aus Cystein, Asparagin, Glutamin, Histidin, Lysin und Arginin ersetzt wird; und/oder
- die unpolare Aminosäure Isoleucin an Position 36 von IMPIα entweder durch eine unpolare Aminosäure ausgewählt aus der Gruppe Valin, Phenylalanin und Tryptophan oder durch eine polare Aminosäure ausgewählt aus der Gruppe bestehend aus Tyrosin, Serin, Threonin, Asparagin, Glutamin, Histidin, Lysin und Arginin ersetzt wird; und/oder
- die polare Aminosäureposition 39 von IMPIα entweder durch die unpolare Aminosäure Valin oder durch die polaren Aminosäuren Histidin oder Lysin ersetzt wird.

2. Polypeptid nach Anspruch 1 mit den Aminosäuresequenzen von den SEQ ID Nummern 10, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84.

3. Polypeptid nach Anspruch 1 oder 2 mit chemischen Modifikationen in der Seitenkette oder am N- und / oder C-Terminus zur Verbesserung biologischer oder chemischer Eigenschaften wie Bioverfügbarkeit, Stabilität, Wirksamkeit oder umfassend einer nachweisbaren Markierung.

4. Fusionspolypeptid umfassend ein Polypeptid nach mindestens einem der Ansprüche 1 bis 3, fusioniert mit mindestens einem Polypeptid mit einer physiologischen Funktion ausgewählt aus der Gruppe bestehend aus einem Antikörper oder Antikörperfragment, Gerüsten wie Lipocalin, Ankyrin, Fibronektin, Transferrin Tetranectin, Adnectin, Albumin, Uteroglobin oder Protein A; funktionelle Peptide wie Transferrin; Peptide, die für diagnostische Anwendungen nützlich sind, wie grün fluoreszierendes Protein; oder Peptidmarker, wie Hexahistidin, die eine Immobilisierung auf technischen Oberflächen ermöglichen, oder eine Transferase wie Glutathion-S-Transferase.

5. Fusionspolypeptid nach Anspruch 4, das durch ein Peptid mit 1 bis 100 Aminosäuren Länge verbunden ist.

6. Fusionspolypeptid nach Anspruch 4, wobei eine chemische Bindungsgruppe das Polypeptid nach mindestens einem der Ansprüche 1 bis 3 und das mindestens eine Polypeptid mit einer physiologischen Funktion konjugiert.

7. Polynukleotid, das für das Polypeptid nach mindestens einem der Ansprüche 1 oder 2 oder das Fusionspolypeptid nach Anspruch 5 kodiert.

8. Polynukleotid nach Anspruch 7, operativ verknüpft mit dem heterologen Promotor.

9. Wirtszelle umfassend die rekombinante Nukleinsäure nach Anspruch 7 oder 8.

10. Verfahren zur Herstellung eines Polypeptids, umfassend das Kultivieren der Wirtszelle nach Anspruch 9, die Expression der Nukleinsäure in der Wirtszelle und die Isolierung des Polypeptids.

11. Kit zur Diagnose umfassend ein Peptid nach Anspruch 3 und/oder ein Fusionspolypeptid nach einem der Ansprüche 4 bis 6.

12. Pharmazeutische Zusammensetzung umfassend ein Polypeptid nach Anspruch 1 und/oder das Fusionspolypeptid nach mindestens einem der Ansprüche 4 bis 6 oder ein Polynukleotid, umfassend einen Abschnitt, der für mindestens eines der Polypeptide nach Anspruch 1 bis 6 codiert.

13. Polypeptid nach Anspruch 1 oder das Fusionspolypeptid nach einem der Ansprüche 4 bis 6 oder das Polynukleotid nach Anspruch 7 zur Verwendung bei der Behandlung eines Tieres oder Menschen, die mit Mikroorganismen infiziert sind, die bakterielle Toxine der M4 oder Metzincin Familie von Metalloproteinasen sezernieren insbesondere Thermolysin, Aureolysin, Bacillolysin, Pseudolysin, Vibriolysin oder Anthrax npr599.

14. Verwendung eines Polypeptids nach Anspruch 3 und/oder eines Fusionspolypeptids nach einem der Ansprüche 4 bis 6 zum Nachweis des Vorhandenseins oder der Aktivität von Proteasen, die zu der M4-Familie gehören, in einer Probe.

## Revendications

1. Polypeptide ayant au moins 70 % d'homologie, en particulier 80 %, 90 % ou 95 % d'homologie avec le polypeptide de SÉQ ID n° : 2 représentant le type sauvage de la protéine inhibitrice de la métalloprotéinase d'insecte IMPIα et ayant au moins une mutation en position 35, 36 et/ou 39 de la séquence d'acides aminés de SÉQ ID n° : 2 et le polypeptide ayant une valeur CI₅₀ vis-à-vis de la thermolysine inférieure à la valeur CI₅₀ de l'IMPIα, dans lequel
- l'acide aminé non polaire isoleucine en position 35 de l'IMPIα est remplacé soit par un acide aminé non polaire choisi dans le groupe constitué par la leucine, la méthionine et la phénylalanine soit par un acide aminé polaire choisi dans le groupe constitué par la cystéine, l'asparagine, la glutamine , l'histidine, la lysine et l'arginine ; et/ou
- l'acide aminé non polaire isoleucine en position 36 de l'IMPIα est remplacé soit par un acide aminé non polaire choisi dans le groupe constitué par la valine, la phénylalanine et le tryptophane soit par un acide aminé polaire choisi dans le groupe constitué par la tyrosine, la sérine, la thréonine, l'asparagine, la glutamine , l'histidine, la lysine et l'arginine ; et/ou
- l'acide aminé polaire en position 39 de l'IMPIα est remplacé soit par l'acide aminé non polaire valine soit par les acides aminés polaires histidine ou lysine.

2. Polypeptide selon la revendication 1 ayant les séquences d'acides aminés de SÉQ ID numéros 10, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84.

3. Polypeptide selon la revendication 1 ou 2 ayant des modifications chimiques dans la chaîne latérale ou au niveau du N et/ou C-terminal pour améliorer les propriétés biologiques ou chimiques telles que la biodisponibilité, la stabilité, l'efficacité, ou comprenant un marqueur détectable.

4. Polypeptide de fusion comprenant un polypeptide selon au moins l'une des revendications 1 à 3, fusionné à au moins un polypeptide ayant une fonction physiologique choisi dans le groupe constitué par un anticorps ou un fragment d'anticorps, des charpentes telles que la lipocaline, l'ankyrine, la fibronectine, la transferrine, la tétranectine, l'adnectine, l'albumine, l'utéroglobine ou la protéine A ; les peptides fonctionnels tels que la transferrine ; les peptides utiles pour les applications de diagnostic, tels que la protéine fluorescente verte ; ou les marqueurs peptidiques permettant l'immobilisation sur des surfaces techniques, tels que l'hexahistidine ; ou une transférase telle que la glutathione-S-transférase.

5. Polypeptide de fusion selon la revendication 4 étant lié par un peptide d'une longueur de 1 à 100 acides aminés.

6. Polypeptide de fusion selon la revendication 4 dans lequel un groupe lieur chimique conjugue le polypeptide selon au moins l'une des revendications 1 à 3 et l'au moins un polypeptide ayant une fonction physiologique.

7. Polynucléotide codant pour le polypeptide selon au moins l'une des revendications 1 ou 2 ou le polypeptide de fusion selon la revendication 5.

8. Polynucléotide selon la revendication 7 opérationnellement lié au promoteur hétérologue.

9. Cellule hôte comprenant l'acide nucléique recombinant selon la revendication 7 ou 8.

10. Procédé de production d'un polypeptide comprenant la mise en culture de la cellule hôte selon la revendication 9, l'expression dudit acide nucléique dans la cellule hôte et l'isolement du polypeptide.

11. Kit de diagnostic comprenant un peptide selon la revendication 3 et/ou un polypeptide de fusion selon l'une quelconque des revendications 4 à 6.

12. Composition pharmaceutique comprenant un polypeptide selon la revendication 1 et/ou le polypeptide de fusion selon au moins l'une des revendications 4 à 6 ou un polynucléotide comprenant une section codant pour au moins l'un des polypeptides selon les revendications 1 à 6.

13. Polypeptide selon la revendication 1 ou polypeptide de fusion selon l'une quelconque des revendications 4 à 6 ou polynucléotide selon la revendication 7 pour une utilisation dans le traitement d'un animal ou d'un être humain infecté par des micro-organismes sécrétant des toxines bactériennes de la famille M4 ou metzincine des métalloprotéinases, en particulier la thermolysine, l'auréolysine, la bacillolysine, la pseudolysine, la vibriolysine ou l'anthrax npr599.

14. Utilisation d'un polypeptide selon la revendication 3 et/ou d'un polypeptide de fusion selon l'une quelconque des revendications 4 à 6 pour la détection de la présence ou de l'activité de protéases appartenant à la famille M4 dans un échantillon.


### SEQUENCE LISTING

<110> Fraunhofer-Institut für Molekularbiologie und Angewandte Oekologie IME
<120> Insect Metalloproteinase Inhibitors
<130> 140330wo
<150> EP13178180
   <151> 2013-07-26
<160> 95
<170> PatentIn version 3.3
<210> 1
   <211> 207
   <212> DNA
   <213> Galleria mellonella
<220>
   <221> CDS
   <222> (1)..(207)
<400> 1
   ata gtc cta att tgt aac ggt gga cac gaa tac tac gag tgc ggt gga       48
   Ile Val Leu Ile Cys Asn Gly Gly His Glu Tyr Tyr Glu Cys Gly Gly
   1               5                   10                  15
   gcc tgc gat aat gta tgt gca gat tta cat ata cag aat aaa aca aac       96
   Ala Cys Asp Asn Val Cys Ala Asp Leu His Ile Gln Asn Lys Thr Asn
               20                  25                  30
   tgt ccc atc att aat ata aga tgt aat gac aag tgc tac tgt gaa gat      144
   Cys Pro Ile Ile Asn Ile Arg Cys Asn Asp Lys Cys Tyr Cys Glu Asp
           35                  40                  45
   ggc tat gca agg gat gtc aat ggc aaa tgt ata ccg ata aaa gac tgt      192
   Gly Tyr Ala Arg Asp Val Asn Gly Lys Cys Ile Pro Ile Lys Asp Cys
       50                  55                  60
   cct aaa ata cgt tcg                                                  207
   Pro Lys Ile Arg Ser
   65
<210> 2
   <211> 69
   <212> PRT
   <213> Galleria mellonella
<400> 2
   Ile Val Leu Ile Cys Asn Gly Gly His Glu Tyr Tyr Glu Cys Gly Gly
   1               5                   10                  15
   Ala Cys Asp Asn Val Cys Ala Asp Leu His Ile Gln Asn Lys Thr Asn
               20                  25                  30
   Cys Pro Ile Ile Asn Ile Arg Cys Asn Asp Lys Cys Tyr Cys Glu Asp
           35                  40                  45
   Gly Tyr Ala Arg Asp Val Asn Gly Lys Cys Ile Pro Ile Lys Asp Cys
       50                  55                  60
   Pro Lys Ile Arg Ser
   65
<210> 3
   <211> 249
   <212> DNA
   <213> Galleria mellonella
<220>
   <221> CDS
   <222> (1)..(249)
<400> 3
   cgt cgt tcc att ggg ata cca gtc gac aag aaa tgc tgc aca ggt cct       48
   Arg Arg Ser Ile Gly Ile Pro Val Asp Lys Lys Cys Cys Thr Gly Pro
   1               5                   10                  15
   aac gaa cac tat gac gaa gag aaa gta agc tgt cct cca gaa acc tgt       96
   Asn Glu His Tyr Asp Glu Glu Lys Val Ser Cys Pro Pro Glu Thr Cys
               20                  25                  30
   atc tcc ctt gtg gct aag ttt tcc tgc att gac tcc cct cca ccg tcg      144
   Ile Ser Leu Val Ala Lys Phe Ser Cys Ile Asp Ser Pro Pro Pro Ser
           35                  40                  45
   cca ggg tgt tct tgc aat tca gga tac tta aga ctt aac cta act tca      192
   Pro Gly Cys Ser Cys Asn Ser Gly Tyr Leu Arg Leu Asn Leu Thr Ser
       50                  55                  60
   cca tgc ata cca att tgc gat tgt cca caa atg caa cat tcc cct gat      240
   Pro Cys Ile Pro Ile Cys Asp Cys Pro Gln Met Gln His Ser Pro Asp
   65                  70                  75                  80
   tgt caa taa                                                          249
   Cys Gln
<210> 4
   <211> 82
   <212> PRT
   <213> Galleria mellonella
<400> 4
   Arg Arg Ser Ile Gly Ile Pro Val Asp Lys Lys Cys Cys Thr Gly Pro
   1               5                   10                  15
   Asn Glu His Tyr Asp Glu Glu Lys Val Ser Cys Pro Pro Glu Thr Cys
               20                  25                  30
   Ile Ser Leu Val Ala Lys Phe Ser Cys Ile Asp Ser Pro Pro Pro Ser
           35                  40                  45
   Pro Gly Cys Ser Cys Asn Ser Gly Tyr Leu Arg Leu Asn Leu Thr Ser
       50                  55                  60
   Pro Cys Ile Pro Ile Cys Asp Cys Pro Gln Met Gln His Ser Pro Asp
   65                  70                  75                  80
   Cys Gln
<210> 5
   <211> 1041
   <212> DNA
   <213> Galleria mellonella
<220>
   <221> CDS
   <222> (1)..(1041)
<400> 5
   atg tcc cct ata cta ggt tat tgg aaa att aag ggc ctt gtg caa ccc       48
   Met Ser Pro Ile Leu Gly Tyr Trp Lys Ile Lys Gly Leu Val Gln Pro
   1               5                   10                  15
   act cga ctt ctt ttg gaa tat ctt gaa gaa aaa tat gaa gag cat ttg       96
   Thr Arg Leu Leu Leu Glu Tyr Leu Glu Glu Lys Tyr Glu Glu His Leu
               20                  25                  30
   tat gag cgc gat gaa ggt gat aaa tgg cga aac aaa aag ttt gaa ttg      144
   Tyr Glu Arg Asp Glu Gly Asp Lys Trp Arg Asn Lys Lys Phe Glu Leu
           35                  40                  45
   ggt ttg gag ttt ccc aat ctt cct tat tat att gat ggt gat gtt aaa      192
   Gly Leu Glu Phe Pro Asn Leu Pro Tyr Tyr Ile Asp Gly Asp Val Lys
       50                  55                  60
   tta aca cag tct atg gcc atc ata cgt tat ata gct gac aag cac aac      240
   Leu Thr Gln Ser Met Ala Ile Ile Arg Tyr Ile Ala Asp Lys His Asn
   65                  70                  75                  80
   atg ttg ggt ggt tgt cca aaa gag cgt gca gag att tca atg ctt gaa      288
   Met Leu Gly Gly Cys Pro Lys Glu Arg Ala Glu Ile Ser Met Leu Glu
                   85                  90                  95
   gga gcg gtt ttg gat att aga tac ggt gtt tcg aga att gca tat agt      336
   Gly Ala Val Leu Asp Ile Arg Tyr Gly Val Ser Arg Ile Ala Tyr Ser
               100                 105                 110
   aaa gac ttt gaa act ctc aaa gtt gat ttt ctt agc aag cta cct gaa      384
   Lys Asp Phe Glu Thr Leu Lys Val Asp Phe Leu Ser Lys Leu Pro Glu
           115                 120                 125
   atg ctg aaa atg ttc gaa gat cgt tta tgt cat aaa aca tat tta aat      432
   Met Leu Lys Met Phe Glu Asp Arg Leu Cys His Lys Thr Tyr Leu Asn
       130                 135                 140
   ggt gat cat gta acc cat cct gac ttc atg ttg tat gac gct ctt gat        480
   Gly Asp His Val Thr His Pro Asp Phe Met Leu Tyr Asp Ala Leu Asp
   145                 150                 155                 160
   gtt gtt tta tac atg gac cca atg tgc ctg gat gcg ttc cca aaa tta        528
   Val Val Leu Tyr Met Asp Pro Met Cys Leu Asp Ala Phe Pro Lys Leu
                   165                 170                 175
   gtt tgt ttt aaa aaa cgt att gaa gct atc cca caa att gat aag tac        576
   Val Cys Phe Lys Lys Arg Ile Glu Ala Ile Pro Gln Ile Asp Lys Tyr
               180                 185                 190
   ttg aaa tcc agc aag tat ata gca tgg cct ttg cag ggc tgg caa gcc        624
   Leu Lys Ser Ser Lys Tyr Ile Ala Trp Pro Leu Gln Gly Trp Gln Ala
           195                 200                 205
   acg ttt ggt ggt ggc gac cat cct cca aaa tcg gat ggt tca act agt        672
   Thr Phe Gly Gly Gly Asp His Pro Pro Lys Ser Asp Gly Ser Thr Ser
       210                 215                 220
   ggt tct ggt cat cac cat cac cat cac tcc gcg ggt ctg gtg cca cgc        720
   Gly Ser Gly His His His His His His Ser Ala Gly Leu Val Pro Arg
   225                 230                 235                 240
   ggt agt act gca att ggt atg aaa gaa acc gct gct gct aaa ttc gaa        768
   Gly Ser Thr Ala Ile Gly Met Lys Glu Thr Ala Ala Ala Lys Phe Glu
                   245                 250                 255
   cgc cag cac atg gac agc cca gat ctg ggt acc ggt ggt ggc tcc ggt        816
   Arg Gln His Met Asp Ser Pro Asp Leu Gly Thr Gly Gly Gly Ser Gly
               260                 265                 270
   gat gac gac gac aag ata gtc cta att tgt aac ggt gga cac gaa tac        864
   Asp Asp Asp Asp Lys Ile Val Leu Ile Cys Asn Gly Gly His Glu Tyr
           275                 280                 285
   tac gag tgc ggt gga gcc tgc gat aat gta tgt gca gat tta cat ata        912
   Tyr Glu Cys Gly Gly Ala Cys Asp Asn Val Cys Ala Asp Leu His Ile
       290                 295                 300
   cag aat aaa aca aac tgt ccc atc att aat ata aga tgt aat gac aag        960
   Gln Asn Lys Thr Asn Cys Pro Ile Ile Asn Ile Arg Cys Asn Asp Lys
   305                 310                 315                 320
   tgc tac tgt gaa gat ggc tat gca agg gat gtc aat ggc aaa tgt ata       1008
   Cys Tyr Cys Glu Asp Gly Tyr Ala Arg Asp Val Asn Gly Lys Cys Ile
                   325                 330                 335
   ccg ata aaa gac tgt cct aaa ata cgt tcg tag                           1041
   Pro Ile Lys Asp Cys Pro Lys Ile Arg Ser
               340                 345
<210> 6
   <211> 346
   <212> PRT
   <213> Galleria mellonella
<400> 6
   Met Ser Pro Ile Leu Gly Tyr Trp Lys Ile Lys Gly Leu Val Gln Pro
   1               5                   10                  15
   Thr Arg Leu Leu Leu Glu Tyr Leu Glu Glu Lys Tyr Glu Glu His Leu
               20                  25                  30
   Tyr Glu Arg Asp Glu Gly Asp Lys Trp Arg Asn Lys Lys Phe Glu Leu
           35                  40                  45
   Gly Leu Glu Phe Pro Asn Leu Pro Tyr Tyr Ile Asp Gly Asp Val Lys
       50                  55                  60
   Leu Thr Gln Ser Met Ala Ile Ile Arg Tyr Ile Ala Asp Lys His Asn
   65                  70                  75                  80
   Met Leu Gly Gly Cys Pro Lys Glu Arg Ala Glu Ile Ser Met Leu Glu
                   85                  90                  95
   Gly Ala Val Leu Asp Ile Arg Tyr Gly Val Ser Arg Ile Ala Tyr Ser
               100                 105                 110
   Lys Asp Phe Glu Thr Leu Lys Val Asp Phe Leu Ser Lys Leu Pro Glu
           115                 120                 125
   Met Leu Lys Met Phe Glu Asp Arg Leu Cys His Lys Thr Tyr Leu Asn
       130                 135                 140
   Gly Asp His Val Thr His Pro Asp Phe Met Leu Tyr Asp Ala Leu Asp
   145                 150                 155                 160
   Val Val Leu Tyr Met Asp Pro Met Cys Leu Asp Ala Phe Pro Lys Leu
                   165                 170                 175
   Val Cys Phe Lys Lys Arg Ile Glu Ala Ile Pro Gln Ile Asp Lys Tyr
               180                 185                 190
   Leu Lys Ser Ser Lys Tyr Ile Ala Trp Pro Leu Gln Gly Trp Gln Ala
           195                 200                 205
   Thr Phe Gly Gly Gly Asp His Pro Pro Lys Ser Asp Gly Ser Thr Ser
       210                 215                 220
   Gly Ser Gly His His His His His His Ser Ala Gly Leu Val Pro Arg
   225                 230                 235                 240
   Gly Ser Thr Ala Ile Gly Met Lys Glu Thr Ala Ala Ala Lys Phe Glu
                   245                 250                 255
   Arg Gln His Met Asp Ser Pro Asp Leu Gly Thr Gly Gly Gly Ser Gly
               260                 265                 270
   Asp Asp Asp Asp Lys Ile Val Leu Ile Cys Asn Gly Gly His Glu Tyr
               275                 280                 285
   Tyr Glu Cys Gly Gly Ala Cys Asp Asn Val Cys Ala Asp Leu His Ile
           290                 295                 300
   Gln Asn Lys Thr Asn Cys Pro Ile Ile Asn Ile Arg Cys Asn Asp Lys
   305                 310                 315                 320
   Cys Tyr Cys Glu Asp Gly Tyr Ala Arg Asp Val Asn Gly Lys Cys Ile
                   325                 330                 335
   Pro Ile Lys Asp Cys Pro Lys Ile Arg Ser
               340                 345
<210> 7
   <211> 1080
   <212> DNA
   <213> Galleria mellonella
<220>
   <221> CDS
   <222> (1)..(1080)
<400> 7
   atg tcc cct ata cta ggt tat tgg aaa att aag ggc ctt gtg caa ccc       48
   Met Ser Pro Ile Leu Gly Tyr Trp Lys Ile Lys Gly Leu Val Gln Pro
   1               5                   10                  15
   act cga ctt ctt ttg gaa tat ctt gaa gaa aaa tat gaa gag cat ttg       96
   Thr Arg Leu Leu Leu Glu Tyr Leu Glu Glu Lys Tyr Glu Glu His Leu
               20                  25                  30
   tat gag cgc gat gaa ggt gat aaa tgg cga aac aaa aag ttt gaa ttg      144
   Tyr Glu Arg Asp Glu Gly Asp Lys Trp Arg Asn Lys Lys Phe Glu Leu
           35                  40                  45
   ggt ttg gag ttt ccc aat ctt cct tat tat att gat ggt gat gtt aaa      192
   Gly Leu Glu Phe Pro Asn Leu Pro Tyr Tyr Ile Asp Gly Asp Val Lys
       50                  55                  60
   tta aca cag tct atg gcc atc ata cgt tat ata gct gac aag cac aac      240
   Leu Thr Gln Ser Met Ala Ile Ile Arg Tyr Ile Ala Asp Lys His Asn
   65                  70                  75                  80
   atg ttg ggt ggt tgt cca aaa gag cgt gca gag att tca atg ctt gaa      288
   Met Leu Gly Gly Cys Pro Lys Glu Arg Ala Glu Ile Ser Met Leu Glu
                   85                  90                  95
   gga gcg gtt ttg gat att aga tac ggt gtt tcg aga att gca tat agt      336
   Gly Ala Val Leu Asp Ile Arg Tyr Gly Val Ser Arg Ile Ala Tyr Ser
               100                 105                 110
   aaa gac ttt gaa act ctc aaa gtt gat ttt ctt agc aag cta cct gaa      384
   Lys Asp Phe Glu Thr Leu Lys Val Asp Phe Leu Ser Lys Leu Pro Glu
           115                 120                 125
   atg ctg aaa atg ttc gaa gat cgt tta tgt cat aaa aca tat tta aat      432
   Met Leu Lys Met Phe Glu Asp Arg Leu Cys His Lys Thr Tyr Leu Asn
       130                 135                 140
   ggt gat cat gta acc cat cct gac ttc atg ttg tat gac gct ctt gat      480
   Gly Asp His Val Thr His Pro Asp Phe Met Leu Tyr Asp Ala Leu Asp
   145                 150                 155                 160
   gtt gtt tta tac atg gac cca atg tgc ctg gat gcg ttc cca aaa tta      528
   Val Val Leu Tyr Met Asp Pro Met Cys Leu Asp Ala Phe Pro Lys Leu
                   165                 170                 175
   gtt tgt ttt aaa aaa cgt att gaa gct atc cca caa att gat aag tac      576
   Val Cys Phe Lys Lys Arg Ile Glu Ala Ile Pro Gln Ile Asp Lys Tyr
               180                 185                 190
   ttg aaa tcc agc aag tat ata gca tgg cct ttg cag ggc tgg caa gcc      624
   Leu Lys Ser Ser Lys Tyr Ile Ala Trp Pro Leu Gln Gly Trp Gln Ala
           195                 200                 205
   acg ttt ggt ggt ggc gac cat cct cca aaa tcg gat ggt tca act agt      672
   Thr Phe Gly Gly Gly Asp His Pro Pro Lys Ser Asp Gly Ser Thr Ser
       210                 215                 220
   ggt tct ggt cat cac cat cac cat cac tcc gcg ggt ctg gtg cca cgc      720
   Gly Ser Gly His His His His His His Ser Ala Gly Leu Val Pro Arg
   225                 230                 235                 240
   ggt agt act gca att ggt atg aaa gaa acc gct gct gct aaa ttc gaa      768
   Gly Ser Thr Ala Ile Gly Met Lys Glu Thr Ala Ala Ala Lys Phe Glu
                   245                 250                 255
   cgc cag cac atg gac agc cca gat ctg ggt acc ggt ggt ggc tcc ggt      816
   Arg Gln His Met Asp Ser Pro Asp Leu Gly Thr Gly Gly Gly Ser Gly
               260                 265                 270
   gat gac gac gac aag cgt cgt tcc att ggg ata cca gtc gac aag aaa      864
   Asp Asp Asp Asp Lys Arg Arg Ser Ile Gly Ile Pro Val Asp Lys Lys
           275                 280                 285
   tgc tgc aca ggt cct aac gaa cac tat gac gaa gag aaa gta agc tgt      912
   Cys Cys Thr Gly Pro Asn Glu His Tyr Asp Glu Glu Lys Val Ser Cys
       290                 295                 300
   cct cca gaa acc tgt atc tcc ctt gtg gct aag ttt tcc tgc att gac      960
   Pro Pro Glu Thr Cys Ile Ser Leu Val Ala Lys Phe Ser Cys Ile Asp
   305                 310                 315                 320
   tcc cct cca ccg tcg cca ggg tgt tct tgc aat tca gga tac tta aga     1008
   Ser Pro Pro Pro Ser Pro Gly Cys Ser Cys Asn Ser Gly Tyr Leu Arg
                   325                 330                 335
   ctt aac cta act tca cca tgc ata cca att tgc gat tgt cca caa atg     1056
   Leu Asn Leu Thr Ser Pro Cys Ile Pro Ile Cys Asp Cys Pro Gln Met
               340                 345                 350
   caa cat tcc cct gat tgt caa tag                                     1080
   Gln His Ser Pro Asp Cys Gln
           355
<210> 8
   <211> 359
   <212> PRT
   <213> Galleria mellonella
<400> 8
   Met Ser Pro Ile Leu Gly Tyr Trp Lys Ile Lys Gly Leu Val Gln Pro
   1               5                   10                  15
   Thr Arg Leu Leu Leu Glu Tyr Leu Glu Glu Lys Tyr Glu Glu His Leu
               20                  25                  30
   Tyr Glu Arg Asp Glu Gly Asp Lys Trp Arg Asn Lys Lys Phe Glu Leu
           35                  40                  45
   Gly Leu Glu Phe Pro Asn Leu Pro Tyr Tyr Ile Asp Gly Asp Val Lys
       50                  55                  60
   Leu Thr Gln Ser Met Ala Ile Ile Arg Tyr Ile Ala Asp Lys His Asn
   65                  70                  75                  80
   Met Leu Gly Gly Cys Pro Lys Glu Arg Ala Glu Ile Ser Met Leu Glu
                   85                  90                  95
   Gly Ala Val Leu Asp Ile Arg Tyr Gly Val Ser Arg Ile Ala Tyr Ser
               100                 105                 110
   Lys Asp Phe Glu Thr Leu Lys Val Asp Phe Leu Ser Lys Leu Pro Glu
           115                 120                 125
   Met Leu Lys Met Phe Glu Asp Arg Leu Cys His Lys Thr Tyr Leu Asn
       130                 135                 140
   Gly Asp His Val Thr His Pro Asp Phe Met Leu Tyr Asp Ala Leu Asp
   145                 150                 155                 160
   Val Val Leu Tyr Met Asp Pro Met Cys Leu Asp Ala Phe Pro Lys Leu
                   165                 170                 175
   Val Cys Phe Lys Lys Arg Ile Glu Ala Ile Pro Gln Ile Asp Lys Tyr
               180                 185                 190
   Leu Lys Ser Ser Lys Tyr Ile Ala Trp Pro Leu Gln Gly Trp Gln Ala
           195                 200                 205
   Thr Phe Gly Gly Gly Asp His Pro Pro Lys Ser Asp Gly Ser Thr Ser
       210                 215                 220
   Gly Ser Gly His His His His His His Ser Ala Gly Leu Val Pro Arg
   225 230 235 240
   Gly Ser Thr Ala Ile Gly Met Lys Glu Thr Ala Ala Ala Lys Phe Glu
   245 250 255
   Arg Gln His Met Asp Ser Pro Asp Leu Gly Thr Gly Gly Gly Ser Gly
   260 265 270
   Asp Asp Asp Asp Lys Arg Arg Ser Ile Gly Ile Pro Val Asp Lys Lys
   275 280 285
   Cys Cys Thr Gly Pro Asn Glu His Tyr Asp Glu Glu Lys Val Ser Cys
   290 295 300
   Pro Pro Glu Thr Cys Ile Ser Leu Val Ala Lys Phe Ser Cys Ile Asp
   305 310 315 320
   Ser Pro Pro Pro Ser Pro Gly Cys Ser Cys Asn Ser Gly Tyr Leu Arg
   325 330 335
   Leu Asn Leu Thr Ser Pro Cys Ile Pro Ile Cys Asp Cys Pro Gln Met
   340 345 350
   Gln His Ser Pro Asp Cys Gln
   355
<210> 9
   <211> 207
   <212> DNA
   <213> Galleria mellonella
<220>
   <221> CDS
   <222> (1)..(207)
<400> 9
   ata gtc cta att tgt aac ggt gga cac gaa tac tac gag tgc ggt gga       48
   Ile Val Leu Ile Cys Asn Gly Gly His Glu Tyr Tyr Glu Cys Gly Gly
   1               5                   10                  15
   gcc tgc gat aat gta tgt gca gat tta cat ata cag aat aaa aca aac       96
   Ala Cys Asp Asn Val Cys Ala Asp Leu His Ile Gln Asn Lys Thr Asn
               20                  25                  30
   tgt ccc atc att ggt ctg gct tgt aat gac aag tgc tac tgt gaa gat      144
   Cys Pro Ile Ile Gly Leu Ala Cys Asn Asp Lys Cys Tyr Cys Glu Asp
           35                  40                  45
   ggc tat gca agg gat gtc aat ggc aaa tgt ata ccg ata aaa gac tgt      192
   Gly Tyr Ala Arg Asp Val Asn Gly Lys Cys Ile Pro Ile Lys Asp Cys
       50                  55                  60
   cct aaa ata cgt tcg                                                  207
   Pro Lys Ile Arg Ser
   65
<210> 10
   <211> 69
   <212> PRT
   <213> Galleria mellonella
<400> 10
   Ile Val Leu Ile Cys Asn Gly Gly His Glu Tyr Tyr Glu Cys Gly Gly
   1               5                   10                  15
   Ala Cys Asp Asn Val Cys Ala Asp Leu His Ile Gln Asn Lys Thr Asn
               20                  25                  30
   Cys Pro Ile Ile Gly Leu Ala Cys Asn Asp Lys Cys Tyr Cys Glu Asp
           35                  40                  45
   Gly Tyr Ala Arg Asp Val Asn Gly Lys Cys Ile Pro Ile Lys Asp Cys
       50                  55                  60
   Pro Lys Ile Arg Ser
   65
<210> 11
   <211> 1041
   <212> DNA
   <213> Galleria mellonella
<220>
   <221> CDS
   <222> (1)..(1041)
<400> 11
   atg tcc cct ata cta ggt tat tgg aaa att aag ggc ctt gtg caa ccc       48
   Met Ser Pro Ile Leu Gly Tyr Trp Lys Ile Lys Gly Leu Val Gln Pro
   1               5                   10                  15
   act cga ctt ctt ttg gaa tat ctt gaa gaa aaa tat gaa gag cat ttg       96
   Thr Arg Leu Leu Leu Glu Tyr Leu Glu Glu Lys Tyr Glu Glu His Leu
               20                  25                  30
   tat gag cgc gat gaa ggt gat aaa tgg cga aac aaa aag ttt gaa ttg      144
   Tyr Glu Arg Asp Glu Gly Asp Lys Trp Arg Asn Lys Lys Phe Glu Leu
           35                  40                  45
   ggt ttg gag ttt ccc aat ctt cct tat tat att gat ggt gat gtt aaa      192
   Gly Leu Glu Phe Pro Asn Leu Pro Tyr Tyr Ile Asp Gly Asp Val Lys
       50                  55                  60
   tta aca cag tct atg gcc atc ata cgt tat ata gct gac aag cac aac      240
   Leu Thr Gln Ser Met Ala Ile Ile Arg Tyr Ile Ala Asp Lys His Asn
   65                  70                  75                  80
   atg ttg ggt ggt tgt cca aaa gag cgt gca gag att tca atg ctt gaa        288
   Met Leu Gly Gly Cys Pro Lys Glu Arg Ala Glu Ile Ser Met Leu Glu
                   85                  90                  95
   gga gcg gtt ttg gat att aga tac ggt gtt tcg aga att gca tat agt        336
   Gly Ala Val Leu Asp Ile Arg Tyr Gly Val Ser Arg Ile Ala Tyr Ser
               100                 105                 110
   aaa gac ttt gaa act ctc aaa gtt gat ttt ctt agc aag cta cct gaa        384
   Lys Asp Phe Glu Thr Leu Lys Val Asp Phe Leu Ser Lys Leu Pro Glu
           115                 120                 125
   atg ctg aaa atg ttc gaa gat cgt tta tgt cat aaa aca tat tta aat        432
   Met Leu Lys Met Phe Glu Asp Arg Leu Cys His Lys Thr Tyr Leu Asn
       130                 135                 140
   ggt gat cat gta acc cat cct gac ttc atg ttg tat gac gct ctt gat        480
   Gly Asp His Val Thr His Pro Asp Phe Met Leu Tyr Asp Ala Leu Asp
   145                 150                 155                 160
   gtt gtt tta tac atg gac cca atg tgc ctg gat gcg ttc cca aaa tta        528
   Val Val Leu Tyr Met Asp Pro Met Cys Leu Asp Ala Phe Pro Lys Leu
                   165                 170                 175
   gtt tgt ttt aaa aaa cgt att gaa gct atc cca caa att gat aag tac        576
   Val Cys Phe Lys Lys Arg Ile Glu Ala Ile Pro Gln Ile Asp Lys Tyr
               180                 185                 190
   ttg aaa tcc agc aag tat ata gca tgg cct ttg cag ggc tgg caa gcc        624
   Leu Lys Ser Ser Lys Tyr Ile Ala Trp Pro Leu Gln Gly Trp Gln Ala
           195                 200                 205
   acg ttt ggt ggt ggc gac cat cct cca aaa tcg gat ggt tca act agt        672
   Thr Phe Gly Gly Gly Asp His Pro Pro Lys Ser Asp Gly Ser Thr Ser
       210                 215                 220
   ggt tct ggt cat cac cat cac cat cac tcc gcg ggt ctg gtg cca cgc        720
   Gly Ser Gly His His His His His His Ser Ala Gly Leu Val Pro Arg
   225                 230                 235                 240
   ggt agt act gca att ggt atg aaa gaa acc gct gct gct aaa ttc gaa        768
   Gly Ser Thr Ala Ile Gly Met Lys Glu Thr Ala Ala Ala Lys Phe Glu
                   245                 250                 255
   cgc cag cac atg gac agc cca gat ctg ggt acc ggt ggt ggc tcc ggt        816
   Arg Gln His Met Asp Ser Pro Asp Leu Gly Thr Gly Gly Gly Ser Gly
               260                 265                 270
   gat gac gac gac aag ata gtc cta att tgt aac ggt gga cac gaa tac        864
   Asp Asp Asp Asp Lys Ile Val Leu Ile Cys Asn Gly Gly His Glu Tyr
           275                 280                 285
   tac gag tgc ggt gga gcc tgc gat aat gta tgt gca gat tta cat ata        912
   Tyr Glu Cys Gly Gly Ala Cys Asp Asn Val Cys Ala Asp Leu His Ile
       290                 295                 300
   cag aat aaa aca aac tgt ccc atc att ggt ctg gct tgt aat gac aag        960
   Gln Asn Lys Thr Asn Cys Pro Ile Ile Gly Leu Ala Cys Asn Asp Lys
   305                 310                 315                 320
   tgc tac tgt gaa gat ggc tat gca agg gat gtc aat ggc aaa tgt ata       1008
   Cys Tyr Cys Glu Asp Gly Tyr Ala Arg Asp Val Asn Gly Lys Cys Ile
                   325                 330                 335
   ccg ata aaa gac tgt cct aaa ata cgt tcg tag                      1041
   Pro Ile Lys Asp Cys Pro Lys Ile Arg Ser
               340                 345
<210> 12
   <211> 346
   <212> PRT
   <213> Galleria mellonella
<400> 12
   Met Ser Pro Ile Leu Gly Tyr Trp Lys Ile Lys Gly Leu Val Gln Pro
   1               5                   10                  15
   Thr Arg Leu Leu Leu Glu Tyr Leu Glu Glu Lys Tyr Glu Glu His Leu
               20                  25                  30
   Tyr Glu Arg Asp Glu Gly Asp Lys Trp Arg Asn Lys Lys Phe Glu Leu
           35                  40                  45
   Gly Leu Glu Phe Pro Asn Leu Pro Tyr Tyr Ile Asp Gly Asp Val Lys
       50                  55                  60
   Leu Thr Gln Ser Met Ala Ile Ile Arg Tyr Ile Ala Asp Lys His Asn
   65                  70                  75                  80
   Met Leu Gly Gly Cys Pro Lys Glu Arg Ala Glu Ile Ser Met Leu Glu
                   85                  90                  95
   Gly Ala Val Leu Asp Ile Arg Tyr Gly Val Ser Arg Ile Ala Tyr Ser
               100                 105                 110
   Lys Asp Phe Glu Thr Leu Lys Val Asp Phe Leu Ser Lys Leu Pro Glu
           115                 120                 125
   Met Leu Lys Met Phe Glu Asp Arg Leu Cys His Lys Thr Tyr Leu Asn
       130                 135                 140
   Gly Asp His Val Thr His Pro Asp Phe Met Leu Tyr Asp Ala Leu Asp
   145                 150                 155                 160
   Val Val Leu Tyr Met Asp Pro Met Cys Leu Asp Ala Phe Pro Lys Leu
                   165                 170                 175
   Val Cys Phe Lys Lys Arg Ile Glu Ala Ile Pro Gln Ile Asp Lys Tyr
               180                 185                 190
   Leu Lys Ser Ser Lys Tyr Ile Ala Trp Pro Leu Gln Gly Trp Gln Ala
           195                 200                 205
   Thr Phe Gly Gly Gly Asp His Pro Pro Lys Ser Asp Gly Ser Thr Ser
       210                 215                 220
   Gly Ser Gly His His His His His His Ser Ala Gly Leu Val Pro Arg
   225                 230                 235                 240
   Gly Ser Thr Ala Ile Gly Met Lys Glu Thr Ala Ala Ala Lys Phe Glu
                   245                 250                 255
   Arg Gln His Met Asp Ser Pro Asp Leu Gly Thr Gly Gly Gly Ser Gly
               260                 265                 270
   Asp Asp Asp Asp Lys Ile Val Leu Ile Cys Asn Gly Gly His Glu Tyr
           275                 280                 285
   Tyr Glu Cys Gly Gly Ala Cys Asp Asn Val Cys Ala Asp Leu His Ile
       290                 295                 300
   Gln Asn Lys Thr Asn Cys Pro Ile Ile Gly Leu Ala Cys Asn Asp Lys
   305                 310                 315                 320
   Cys Tyr Cys Glu Asp Gly Tyr Ala Arg Asp Val Asn Gly Lys Cys Ile
                   325                 330                 335
   Pro Ile Lys Asp Cys Pro Lys Ile Arg Ser
               340                 345
<210> 13
   <211> 207
   <212> DNA
   <213> Galleria mellonella
<220>
   <221> CDS
   <222> (1)..(207)
<400> 13
   ata gtc cta att tgt aac ggt gga cac gaa tac tac gag tgc ggt gga      48
   Ile Val Leu Ile Cys Asn Gly Gly His Glu Tyr Tyr Glu Cys Gly Gly
   1               5                   10                  15
   gcc tgc gat aat gta tgt gca gat tta cat ata cag aat aaa aca aac      96
   Ala Cys Asp Asn Val Cys Ala Asp Leu His Ile Gln Asn Lys Thr Asn
               20                  25                  30
   tgt ccc atc att aat ata aga tgt aat gac aag tgc tac tgt gaa gat     144
   Cys Pro Ile Ile Asn Ile Arg Cys Asn Asp Lys Cys Tyr Cys Glu Asp
           35                  40                  45
   ggc tat gca aag gat gtc aat ggc aaa tgt ata ccg ata aaa gac tgt     192
   Gly Tyr Ala Lys Asp Val Asn Gly Lys Cys Ile Pro Ile Lys Asp Cys
       50                  55                  60
   cct aaa ata cgt tcg                                                     207
   Pro Lys Ile Arg Ser
   65
<210> 14
   <211> 69
   <212> PRT
   <213> Galleria mellonella
<400> 14
   Ile Val Leu Ile Cys Asn Gly Gly His Glu Tyr Tyr Glu Cys Gly Gly
   1               5                   10                  15
   Ala Cys Asp Asn Val Cys Ala Asp Leu His Ile Gln Asn Lys Thr Asn
               20                  25                  30
   Cys Pro Ile Ile Asn Ile Arg Cys Asn Asp Lys Cys Tyr Cys Glu Asp
           35                  40                  45
   Gly Tyr Ala Lys Asp Val Asn Gly Lys Cys Ile Pro Ile Lys Asp Cys
       50                  55                  60
   Pro Lys Ile Arg Ser
   65
<210> 15
   <211> 1041
   <212> DNA
   <213> Galleria mellonella
<220>
   <221> CDS
   <222> (1)..(1041)
<400> 15
   atg tcc cct ata cta ggt tat tgg aaa att aag ggc ctt gtg caa ccc       48
   Met Ser Pro Ile Leu Gly Tyr Trp Lys Ile Lys Gly Leu Val Gln Pro
   1               5                   10                  15
   act cga ctt ctt ttg gaa tat ctt gaa gaa aaa tat gaa gag cat ttg       96
   Thr Arg Leu Leu Leu Glu Tyr Leu Glu Glu Lys Tyr Glu Glu His Leu
               20                  25                  30
   tat gag cgc gat gaa ggt gat aaa tgg cga aac aaa aag ttt gaa ttg      144
   Tyr Glu Arg Asp Glu Gly Asp Lys Trp Arg Asn Lys Lys Phe Glu Leu
           35                  40                  45
   ggt ttg gag ttt ccc aat ctt cct tat tat att gat ggt gat gtt aaa      192
   Gly Leu Glu Phe Pro Asn Leu Pro Tyr Tyr Ile Asp Gly Asp Val Lys
       50                  55                  60
   tta aca cag tct atg gcc atc ata cgt tat ata gct gac aag cac aac      240
   Leu Thr Gln Ser Met Ala Ile Ile Arg Tyr Ile Ala Asp Lys His Asn
   65                  70                  75                  80
   atg ttg ggt ggt tgt cca aaa gag cgt gca gag att tca atg ctt gaa      288
   Met Leu Gly Gly Cys Pro Lys Glu Arg Ala Glu Ile Ser Met Leu Glu
                   85                  90                  95
   gga gcg gtt ttg gat att aga tac ggt gtt tcg aga att gca tat agt      336
   Gly Ala Val Leu Asp Ile Arg Tyr Gly Val Ser Arg Ile Ala Tyr Ser
               100                 105                 110
   aaa gac ttt gaa act ctc aaa gtt gat ttt ctt agc aag cta cct gaa      384
   Lys Asp Phe Glu Thr Leu Lys Val Asp Phe Leu Ser Lys Leu Pro Glu
           115                 120                 125
   atg ctg aaa atg ttc gaa gat cgt tta tgt cat aaa aca tat tta aat      432
   Met Leu Lys Met Phe Glu Asp Arg Leu Cys His Lys Thr Tyr Leu Asn
       130                 135                 140
   ggt gat cat gta acc cat cct gac ttc atg ttg tat gac gct ctt gat      480
   Gly Asp His Val Thr His Pro Asp Phe Met Leu Tyr Asp Ala Leu Asp
   145                 150                 155                 160
   gtt gtt tta tac atg gac cca atg tgc ctg gat gcg ttc cca aaa tta      528
   Val Val Leu Tyr Met Asp Pro Met Cys Leu Asp Ala Phe Pro Lys Leu
                   165                 170                 175
   gtt tgt ttt aaa aaa cgt att gaa gct atc cca caa att gat aag tac      576
   Val Cys Phe Lys Lys Arg Ile Glu Ala Ile Pro Gln Ile Asp Lys Tyr
               180                 185                 190
   ttg aaa tcc agc aag tat ata gca tgg cct ttg cag ggc tgg caa gcc      624
   Leu Lys Ser Ser Lys Tyr Ile Ala Trp Pro Leu Gln Gly Trp Gln Ala
           195                 200                 205
   acg ttt ggt ggt ggc gac cat cct cca aaa tcg gat ggt tca act agt      672
   Thr Phe Gly Gly Gly Asp His Pro Pro Lys Ser Asp Gly Ser Thr Ser
       210                 215                 220
   ggt tct ggt cat cac cat cac cat cac tcc gcg ggt ctg gtg cca cgc      720
   Gly Ser Gly His His His His His His Ser Ala Gly Leu Val Pro Arg
   225                 230                 235                 240
   ggt agt act gca att ggt atg aaa gaa acc gct gct gct aaa ttc gaa      768
   Gly Ser Thr Ala Ile Gly Met Lys Glu Thr Ala Ala Ala Lys Phe Glu
                   245                 250                 255
   cgc cag cac atg gac agc cca gat ctg ggt acc ggt ggt ggc tcc ggt      816
   Arg Gln His Met Asp Ser Pro Asp Leu Gly Thr Gly Gly Gly Ser Gly
               260                 265                 270
   gat gac gac gac aag ata gtc cta att tgt aac ggt gga cac gaa tac      864
   Asp Asp Asp Asp Lys Ile Val Leu Ile Cys Asn Gly Gly His Glu Tyr
           275                 280                 285
   tac gag tgc ggt gga gcc tgc gat aat gta tgt gca gat tta cat ata      912
   Tyr Glu Cys Gly Gly Ala Cys Asp Asn Val Cys Ala Asp Leu His Ile
       290                 295                 300
   cag aat aaa aca aac tgt ccc atc att aat ata aga tgt aat gac aag      960
   Gln Asn Lys Thr Asn Cys Pro Ile Ile Asn Ile Arg Cys Asn Asp Lys
   305                 310                 315                 320
   tgc tac tgt gaa gat ggc tat gca aag gat gtc aat ggc aaa tgt ata     1008
   Cys Tyr Cys Glu Asp Gly Tyr Ala Lys Asp Val Asn Gly Lys Cys Ile
               325                 330                 335
   ccg ata aaa gac tgt cct aaa ata cgt tcg tag                         1041
   Pro Ile Lys Asp Cys Pro Lys Ile Arg Ser
           340                 345
<210> 16
   <211> 346
   <212> PRT
   <213> Galleria mellonella
<400> 16
   Met Ser Pro Ile Leu Gly Tyr Trp Lys Ile Lys Gly Leu Val Gln Pro
   1               5                   10                  15
   Thr Arg Leu Leu Leu Glu Tyr Leu Glu Glu Lys Tyr Glu Glu His Leu
               20                  25                  30
   Tyr Glu Arg Asp Glu Gly Asp Lys Trp Arg Asn Lys Lys Phe Glu Leu
           35                  40                  45
   Gly Leu Glu Phe Pro Asn Leu Pro Tyr Tyr Ile Asp Gly Asp Val Lys
       50                  55                  60
   Leu Thr Gln Ser Met Ala Ile Ile Arg Tyr Ile Ala Asp Lys His Asn
   65                  70                  75                  80
   Met Leu Gly Gly Cys Pro Lys Glu Arg Ala Glu Ile Ser Met Leu Glu
                   85                  90                  95
   Gly Ala Val Leu Asp Ile Arg Tyr Gly Val Ser Arg Ile Ala Tyr Ser
               100                 105                 110
   Lys Asp Phe Glu Thr Leu Lys Val Asp Phe Leu Ser Lys Leu Pro Glu
           115                 120                 125
   Met Leu Lys Met Phe Glu Asp Arg Leu Cys His Lys Thr Tyr Leu Asn
       130                 135                 140
   Gly Asp His Val Thr His Pro Asp Phe Met Leu Tyr Asp Ala Leu Asp
   145                 150                 155                 160
   Val Val Leu Tyr Met Asp Pro Met Cys Leu Asp Ala Phe Pro Lys Leu
                   165                 170                 175
   Val Cys Phe Lys Lys Arg Ile Glu Ala Ile Pro Gln Ile Asp Lys Tyr
               180                 185                 190
   Leu Lys Ser Ser Lys Tyr Ile Ala Trp Pro Leu Gln Gly Trp Gln Ala
           195                 200                 205
   Thr Phe Gly Gly Gly Asp His Pro Pro Lys Ser Asp Gly Ser Thr Ser
       210                 215                 220
   Gly Ser Gly His His His His His His Ser Ala Gly Leu Val Pro Arg
   225                 230                 235                 240
   Gly Ser Thr Ala Ile Gly Met Lys Glu Thr Ala Ala Ala Lys Phe Glu
                   245                 250                 255
   Arg Gln His Met Asp Ser Pro Asp Leu Gly Thr Gly Gly Gly Ser Gly
               260                 265                 270
   Asp Asp Asp Asp Lys Ile Val Leu Ile Cys Asn Gly Gly His Glu Tyr
           275                 280                 285
   Tyr Glu Cys Gly Gly Ala Cys Asp Asn Val Cys Ala Asp Leu His Ile
       290                 295                 300
   Gln Asn Lys Thr Asn Cys Pro Ile Ile Asn Ile Arg Cys Asn Asp Lys
   305                 310                 315                 320
   Cys Tyr Cys Glu Asp Gly Tyr Ala Lys Asp Val Asn Gly Lys Cys Ile
                   325                 330                 335
   Pro Ile Lys Asp Cys Pro Lys Ile Arg Ser
               340                 345
<210> 17
   <211> 207
   <212> DNA
   <213> Galleria mellonella
<220>
   <221> CDS
   <222> (1)..(207)
<220>
   <221> misc_feature
   <222> (185)..(185)
   <223> n is a, c, g, or t
<400> 17
   ata gtc cta att tgt aac ggt gga cac gaa tac tac gag tgc ggt gga        48
   Ile Val Leu Ile Cys Asn Gly Gly His Glu Tyr Tyr Glu Cys Gly Gly
   1               5                   10                  15
   gcc tgc gat aat gta tgt gca gat tta cat ata cag aat aaa aca aac        96
   Ala Cys Asp Asn Val Cys Ala Asp Leu His Ile Gln Asn Lys Thr Asn
               20                  25                  30
   tgt ccc ttg att aat ata aga tgt aat gac aag tgc tac tgt gaa gat       144
   Cys Pro Leu Ile Asn Ile Arg Cys Asn Asp Lys Cys Tyr Cys Glu Asp
           35                  40                  45
   ggc tat gca agg gat gtc aat ggc aaa tgt ata ccg ata ana gac tgt       192
   Gly Tyr Ala Arg Asp Val Asn Gly Lys Cys Ile Pro Ile Xaa Asp Cys
       50                  55                  60
   cct aaa ata cgt tcg                                                   207
   Pro Lys Ile Arg Ser
   65
<210> 18
   <211> 69
   <212> PRT
   <213> Galleria mellonella
<220>
   <221> misc_feature
   <222> (62)..(62)
   <223> The 'Xaa' at location 62 stands for Lys, Arg, Thr, or Ile.
<400> 18
   Ile Val Leu Ile Cys Asn Gly Gly His Glu Tyr Tyr Glu Cys Gly Gly
   1               5                   10                  15
   Ala Cys Asp Asn Val Cys Ala Asp Leu His Ile Gln Asn Lys Thr Asn
               20                  25                  30
   Cys Pro Leu Ile Asn Ile Arg Cys Asn Asp Lys Cys Tyr Cys Glu Asp
           35                  40                  45
   Gly Tyr Ala Arg Asp Val Asn Gly Lys Cys Ile Pro Ile Xaa Asp Cys
       50                  55                  60
   Pro Lys Ile Arg Ser
   65
<210> 19
   <211> 207
   <212> DNA
   <213> Galleria mellonella
<220>
   <221> CDS
   <222> (1)..(207)
<400> 19
   ata gtc cta att tgt aac ggt gga cac gaa tac tac gag tgc ggt gga        48
   Ile Val Leu Ile Cys Asn Gly Gly His Glu Tyr Tyr Glu Cys Gly Gly
   1               5                   10                  15
   gcc tgc gat aat gta tgt gca gat tta cat ata cag aat aaa aca aac        96
   Ala Cys Asp Asn Val Cys Ala Asp Leu His Ile Gln Asn Lys Thr Asn
               20                  25                  30
   tgt ccc atg att aat ata aga tgt aat gac aag tgc tac tgt gaa gat       144
   Cys Pro Met Ile Asn Ile Arg Cys Asn Asp Lys Cys Tyr Cys Glu Asp
           35                  40                  45
   ggc tat gca agg gat gtc aat ggc aaa tgt ata ccg ata aaa gac tgt       192
   Gly Tyr Ala Arg Asp Val Asn Gly Lys Cys Ile Pro Ile Lys Asp Cys
       50                  55                  60
   cct aaa ata cgt tcg                                                   207
   Pro Lys Ile Arg Ser
   65
<210> 20
   <211> 69
   <212> PRT
   <213> Galleria mellonella
<400> 20
   Ile Val Leu Ile Cys Asn Gly Gly His Glu Tyr Tyr Glu Cys Gly Gly
   1               5                   10                  15
   Ala Cys Asp Asn Val Cys Ala Asp Leu His Ile Gln Asn Lys Thr Asn
               20                  25                  30
   Cys Pro Met Ile Asn Ile Arg Cys Asn Asp Lys Cys Tyr Cys Glu Asp
           35                  40                  45
   Gly Tyr Ala Arg Asp Val Asn Gly Lys Cys Ile Pro Ile Lys Asp Cys
       50                  55                  60
   Pro Lys Ile Arg Ser
   65
<210> 21
   <211> 207
   <212> DNA
   <213> Galleria mellonella
<220>
   <221> CDS
   <222> (1)..(207)
<400> 21
   ata gtc cta att tgt aac ggt gga cac gaa tac tac gag tgc ggt gga        48
   Ile Val Leu Ile Cys Asn Gly Gly His Glu Tyr Tyr Glu Cys Gly Gly
   1               5                   10                  15
   gcc tgc gat aat gta tgt gca gat tta cat ata cag aat aaa aca aac        96
   Ala Cys Asp Asn Val Cys Ala Asp Leu His Ile Gln Asn Lys Thr Asn
               20                  25                  30
   tgt ccc ttc att aat ata aga tgt aat gac aag tgc tac tgt gaa gat       144
   Cys Pro Phe Ile Asn Ile Arg Cys Asn Asp Lys Cys Tyr Cys Glu Asp
           35                  40                  45
   ggc tat gca agg gat gtc aat ggc aaa tgt ata ccg ata aaa gac tgt       192
   Gly Tyr Ala Arg Asp Val Asn Gly Lys Cys Ile Pro Ile Lys Asp Cys
       50                  55                  60
   cct aaa ata cgt tcg                                                   207
   Pro Lys Ile Arg Ser
   65
<210> 22
   <211> 69
   <212> PRT
   <213> Galleria mellonella
<400> 22
   Ile Val Leu Ile Cys Asn Gly Gly His Glu Tyr Tyr Glu Cys Gly Gly
   1               5                   10                  15
   Ala Cys Asp Asn Val Cys Ala Asp Leu His Ile Gln Asn Lys Thr Asn
               20                  25                  30
   Cys Pro Phe Ile Asn Ile Arg Cys Asn Asp Lys Cys Tyr Cys Glu Asp
           35                  40                  45
   Gly Tyr Ala Arg Asp Val Asn Gly Lys Cys Ile Pro Ile Lys Asp Cys
       50                  55                  60
   Pro Lys Ile Arg Ser
   65
<210> 23
   <211> 207
   <212> DNA
   <213> Galleria mellonella
<220>
   <221> CDS
   <222> (1)..(207)
<400> 23
   ata gtc cta att tgt aac ggt gga cac gaa tac tac gag tgc ggt gga       48
   Ile Val Leu Ile Cys Asn Gly Gly His Glu Tyr Tyr Glu Cys Gly Gly
   1               5                   10                  15
   gcc tgc gat aat gta tgt gca gat tta cat ata cag aat aaa aca aac       96
   Ala Cys Asp Asn Val Cys Ala Asp Leu His Ile Gln Asn Lys Thr Asn
               20                  25                  30
   tgt ccc tgt att aat ata aga tgt aat gac aag tgc tac tgt gaa gat      144
   Cys Pro Cys Ile Asn Ile Arg Cys Asn Asp Lys Cys Tyr Cys Glu Asp
           35                  40                  45
   ggc tat gca agg gat gtc aat ggc aaa tgt ata ccg ata aaa gac tgt      192
   Gly Tyr Ala Arg Asp Val Asn Gly Lys Cys Ile Pro Ile Lys Asp Cys
       50                  55                  60
   cct aaa ata cgt tcg                                                  207
   Pro Lys Ile Arg Ser
   65
<210> 24
   <211> 69
   <212> PRT
   <213> Galleria mellonella
<400> 24
   Ile Val Leu Ile Cys Asn Gly Gly His Glu Tyr Tyr Glu Cys Gly Gly
   1               5                   10                  15
   Ala Cys Asp Asn Val Cys Ala Asp Leu His Ile Gln Asn Lys Thr Asn
               20                  25                  30
   Cys Pro Cys Ile Asn Ile Arg Cys Asn Asp Lys Cys Tyr Cys Glu Asp
           35                  40                  45
   Gly Tyr Ala Arg Asp Val Asn Gly Lys Cys Ile Pro Ile Lys Asp Cys
       50                  55                  60
   Pro Lys Ile Arg Ser
   65
<210> 25
   <211> 207
   <212> DNA
   <213> Galleria mellonella
<220>
   <221> CDS
   <222> (1)..(207)
<400> 25
   ata gtc cta att tgt aac ggt gga cac gaa tac tac gag tgc ggt gga        48
   Ile Val Leu Ile Cys Asn Gly Gly His Glu Tyr Tyr Glu Cys Gly Gly
   1               5                   10                  15
   gcc tgc gat aat gta tgt gca gat tta cat ata cag aat aaa aca aac        96
   Ala Cys Asp Asn Val Cys Ala Asp Leu His Ile Gln Asn Lys Thr Asn
               20                  25                  30
   tgt ccc aat att aat ata aga tgt aat gac aag tgc tac tgt gaa gat       144
   Cys Pro Asn Ile Asn Ile Arg Cys Asn Asp Lys Cys Tyr Cys Glu Asp
           35                  40                  45
   ggc tat gca agg gat gtc aat ggc aaa tgt ata ccg ata aaa gac tgt       192
   Gly Tyr Ala Arg Asp Val Asn Gly Lys Cys Ile Pro Ile Lys Asp Cys
       50                  55                  60
   cct aaa ata cgt tcg                                                   207
   Pro Lys Ile Arg Ser
                                           65
<210> 26
   <211> 69
   <212> PRT
   <213> Galleria mellonella
<400> 26
   Ile Val Leu Ile Cys Asn Gly Gly His Glu Tyr Tyr Glu Cys Gly Gly
   1               5                   10                  15
   Ala Cys Asp Asn Val Cys Ala Asp Leu His Ile Gln Asn Lys Thr Asn
               20                  25                  30
   Cys Pro Asn Ile Asn Ile Arg Cys Asn Asp Lys Cys Tyr Cys Glu Asp
           35                  40                  45
   Gly Tyr Ala Arg Asp Val Asn Gly Lys Cys Ile Pro Ile Lys Asp Cys
       50                  55                  60
   Pro Lys Ile Arg Ser
   65
<210> 27
   <211> 207
   <212> DNA
   <213> Galleria mellonella
<220>
   <221> CDS
   <222> (1)..(207)
<400> 27
   ata gtc cta att tgt aac ggt gga cac gaa tac tac gag tgc ggt gga        48
   Ile Val Leu Ile Cys Asn Gly Gly His Glu Tyr Tyr Glu Cys Gly Gly
   1               5                   10                  15
   gcc tgc gat aat gta tgt gca gat tta cat ata cag aat aaa aca aac        96
   Ala Cys Asp Asn Val Cys Ala Asp Leu His Ile Gln Asn Lys Thr Asn
               20                  25                  30
   tgt ccc cag att aat ata aga tgt aat gac aag tgc tac tgt gaa gat       144
   Cys Pro Gln Ile Asn Ile Arg Cys Asn Asp Lys Cys Tyr Cys Glu Asp
           35                  40                  45
   ggc tat gca agg gat gtc aat ggc aaa tgt ata ccg ata aaa gac tgt       192
   Gly Tyr Ala Arg Asp Val Asn Gly Lys Cys Ile Pro Ile Lys Asp Cys
       50                  55                  60
   cct aaa ata cgt tcg                                                   207
   Pro Lys Ile Arg Ser
   65
<210> 28
   <211> 69
   <212> PRT
   <213> Galleria mellonella
<400> 28
   Ile Val Leu Ile Cys Asn Gly Gly His Glu Tyr Tyr Glu Cys Gly Gly
   1               5                   10                  15
   Ala Cys Asp Asn Val Cys Ala Asp Leu His Ile Gln Asn Lys Thr Asn
               20                  25                  30
   Cys Pro Gln Ile Asn Ile Arg Cys Asn Asp Lys Cys Tyr Cys Glu Asp
           35                  40                  45
   Gly Tyr Ala Arg Asp Val Asn Gly Lys Cys Ile Pro Ile Lys Asp Cys
       50                  55                  60
   Pro Lys Ile Arg Ser
   65
<210> 29
   <211> 207
   <212> DNA
   <213> Galleria mellonella
<220>
   <221> CDS
   <222> (1)..(207)
<400> 29
   ata gtc cta att tgt aac ggt gga cac gaa tac tac gag tgc ggt gga       48
   Ile Val Leu Ile Cys Asn Gly Gly His Glu Tyr Tyr Glu Cys Gly Gly
   1               5                   10                  15
   gcc tgc gat aat gta tgt gca gat tta cat ata cag aat aaa aca aac       96
   Ala Cys Asp Asn Val Cys Ala Asp Leu His Ile Gln Asn Lys Thr Asn
               20                  25                  30
   tgt ccc cac att aat ata aga tgt aat gac aag tgc tac tgt gaa gat      144
   Cys Pro His Ile Asn Ile Arg Cys Asn Asp Lys Cys Tyr Cys Glu Asp
           35                  40                  45
   ggc tat gca agg gat gtc aat ggc aaa tgt ata ccg ata aaa gac tgt      192
   Gly Tyr Ala Arg Asp Val Asn Gly Lys Cys Ile Pro Ile Lys Asp Cys
       50                  55                  60
   cct aaa ata cgt tcg                                                  207
   Pro Lys Ile Arg Ser
   65
<210> 30
   <211> 69
   <212> PRT
   <213> Galleria mellonella
<400> 30
   Ile Val Leu Ile Cys Asn Gly Gly His Glu Tyr Tyr Glu Cys Gly Gly
   1               5                   10                  15
   Ala Cys Asp Asn Val Cys Ala Asp Leu His Ile Gln Asn Lys Thr Asn
               20                  25                  30
   Cys Pro His Ile Asn Ile Arg Cys Asn Asp Lys Cys Tyr Cys Glu Asp
           35                  40                  45
   Gly Tyr Ala Arg Asp Val Asn Gly Lys Cys Ile Pro Ile Lys Asp Cys
       50                  55                  60
   Pro Lys Ile Arg Ser
   65
<210> 31
   <211> 207
   <212> DNA
   <213> Galleria mellonella
<220>
   <221> CDS
   <222> (1)..(207)
<400> 31
   ata gtc cta att tgt aac ggt gga cac gaa tac tac gag tgc ggt gga       48
   Ile Val Leu Ile Cys Asn Gly Gly His Glu Tyr Tyr Glu Cys Gly Gly
   1               5                   10                  15
   gcc tgc gat aat gta tgt gca gat tta cat ata cag aat aaa aca aac       96
   Ala Cys Asp Asn Val Cys Ala Asp Leu His Ile Gln Asn Lys Thr Asn
               20                  25                  30
   tgt ccc aag att aat ata aga tgt aat gac aag tgc tac tgt gaa gat      144
   Cys Pro Lys Ile Asn Ile Arg Cys Asn Asp Lys Cys Tyr Cys Glu Asp
           35                  40                  45
   ggc tat gca agg gat gtc aat ggc aaa tgt ata ccg ata aaa gac tgt      192
   Gly Tyr Ala Arg Asp Val Asn Gly Lys Cys Ile Pro Ile Lys Asp Cys
       50                  55                  60
   cct aaa ata cgt tcg                                                  207
   Pro Lys Ile Arg Ser
   65
<210> 32
   <211> 69
   <212> PRT
   <213> Galleria mellonella
<400> 32
   Ile Val Leu Ile Cys Asn Gly Gly His Glu Tyr Tyr Glu Cys Gly Gly
   1               5                   10                  15
   Ala Cys Asp Asn Val Cys Ala Asp Leu His Ile Gln Asn Lys Thr Asn
               20                  25                  30
   Cys Pro Lys Ile Asn Ile Arg Cys Asn Asp Lys Cys Tyr Cys Glu Asp
           35                  40                  45
   Gly Tyr Ala Arg Asp Val Asn Gly Lys Cys Ile Pro Ile Lys Asp Cys
       50                  55                  60
   Pro Lys Ile Arg Ser
   65
<210> 33
   <211> 207
   <212> DNA
   <213> Galleria mellonella
<220>
   <221> CDS
   <222> (1)..(207)
<400> 33
   ata gtc cta att tgt aac ggt gga cac gaa tac tac gag tgc ggt gga        48
   Ile Val Leu Ile Cys Asn Gly Gly His Glu Tyr Tyr Glu Cys Gly Gly
   1               5                   10                  15
   gcc tgc gat aat gta tgt gca gat tta cat ata cag aat aaa aca aac        96
   Ala Cys Asp Asn Val Cys Ala Asp Leu His Ile Gln Asn Lys Thr Asn
               20                  25                  30
   tgt ccc cgt att aat ata aga tgt aat gac aag tgc tac tgt gaa gat       144
   Cys Pro Arg Ile Asn Ile Arg Cys Asn Asp Lys Cys Tyr Cys Glu Asp
           35                  40                  45
   ggc tat gca agg gat gtc aat ggc aaa tgt ata ccg ata aaa gac tgt       192
   Gly Tyr Ala Arg Asp Val Asn Gly Lys Cys Ile Pro Ile Lys Asp Cys
       50                  55                  60
   cct aaa ata cgt tcg                                                   207
   Pro Lys Ile Arg Ser
   65
<210> 34
   <211> 69
   <212> PRT
   <213> Galleria mellonella
<400> 34
   Ile Val Leu Ile Cys Asn Gly Gly His Glu Tyr Tyr Glu Cys Gly Gly
   1               5                   10                  15
   Ala Cys Asp Asn Val Cys Ala Asp Leu His Ile Gln Asn Lys Thr Asn
               20                  25                  30
   Cys Pro Arg Ile Asn Ile Arg Cys Asn Asp Lys Cys Tyr Cys Glu Asp
           35                  40                  45
   Gly Tyr Ala Arg Asp Val Asn Gly Lys Cys Ile Pro Ile Lys Asp Cys
       50                  55                  60
   Pro Lys Ile Arg Ser
   65
<210> 35
   <211> 207
   <212> DNA
   <213> Galleria mellonella
<220>
   <221> CDS
   <222> (1)..(207)
<400> 35
   ata gtc cta att tgt aac ggt gga cac gaa tac tac gag tgc ggt gga        48
   Ile Val Leu Ile Cys Asn Gly Gly His Glu Tyr Tyr Glu Cys Gly Gly
   1               5                   10                  15
   gcc tgc gat aat gta tgt gca gat tta cat ata cag aat aaa aca aac        96
   Ala Cys Asp Asn Val Cys Ala Asp Leu His Ile Gln Asn Lys Thr Asn
               20                  25                  30
   tgt ccc atc gtt aat ata aga tgt aat gac aag tgc tac tgt gaa gat       144
   Cys Pro Ile Val Asn Ile Arg Cys Asn Asp Lys Cys Tyr Cys Glu Asp
           35                  40                  45
   ggc tat gca agg gat gtc aat ggc aaa tgt ata ccg ata aaa gac tgt       192
   Gly Tyr Ala Arg Asp Val Asn Gly Lys Cys Ile Pro Ile Lys Asp Cys
       50                  55                  60
   cct aaa ata cgt tcg                                                   207
   Pro Lys Ile Arg Ser
   65
<210> 36
   <211> 69
   <212> PRT
   <213> Galleria mellonella
<400> 36
   Ile Val Leu Ile Cys Asn Gly Gly His Glu Tyr Tyr Glu Cys Gly Gly
   1               5                   10                  15
   Ala Cys Asp Asn Val Cys Ala Asp Leu His Ile Gln Asn Lys Thr Asn
               20                  25                  30
   Cys Pro Ile Val Asn Ile Arg Cys Asn Asp Lys Cys Tyr Cys Glu Asp
           35                  40                  45
   Gly Tyr Ala Arg Asp Val Asn Gly Lys Cys Ile Pro Ile Lys Asp Cys
       50                  55                  60
   Pro Lys Ile Arg Ser
   65
<210> 37
   <211> 207
   <212> DNA
   <213> Galleria mellonella
<220>
   <221> CDS
   <222> (1)..(207)
<400> 37
   ata gtc cta att tgt aac ggt gga cac gaa tac tac gag tgc ggt gga        48
   Ile Val Leu Ile Cys Asn Gly Gly His Glu Tyr Tyr Glu Cys Gly Gly
   1               5                   10                  15
   gcc tgc gat aat gta tgt gca gat tta cat ata cag aat aaa aca aac        96
   Ala Cys Asp Asn Val Cys Ala Asp Leu His Ile Gln Asn Lys Thr Asn
               20                  25                  30
   tgt ccc atc atg aat ata aga tgt aat gac aag tgc tac tgt gaa gat       144
   Cys Pro Ile Met Asn Ile Arg Cys Asn Asp Lys Cys Tyr Cys Glu Asp
           35                  40                  45
   ggc tat gca agg gat gtc aat ggc aaa tgt ata ccg ata aaa gac tgt       192
   Gly Tyr Ala Arg Asp Val Asn Gly Lys Cys Ile Pro Ile Lys Asp Cys
       50                  55                  60
   cct aaa ata cgt tcg                                                   207
   Pro Lys Ile Arg Ser
   65
<210> 38
   <211> 69
   <212> PRT
   <213> Galleria mellonella
<400> 38
   Ile Val Leu Ile Cys Asn Gly Gly His Glu Tyr Tyr Glu Cys Gly Gly
   1               5                   10                  15
   Ala Cys Asp Asn Val Cys Ala Asp Leu His Ile Gln Asn Lys Thr Asn
               20                  25                  30
   Cys Pro Ile Met Asn Ile Arg Cys Asn Asp Lys Cys Tyr Cys Glu Asp
           35                  40                  45
   Gly Tyr Ala Arg Asp Val Asn Gly Lys Cys Ile Pro Ile Lys Asp Cys
       50                  55                  60
   Pro Lys Ile Arg Ser
   65
<210> 39
   <211> 207
   <212> DNA
   <213> Galleria mellonella
<220>
   <221> CDS
   <222> (1)..(207)
<400> 39
   ata gtc cta att tgt aac ggt gga cac gaa tac tac gag tgc ggt gga        48
   Ile Val Leu Ile Cys Asn Gly Gly His Glu Tyr Tyr Glu Cys Gly Gly
   1               5                   10                  15
   gcc tgc gat aat gta tgt gca gat tta cat ata cag aat aaa aca aac        96
   Ala Cys Asp Asn Val Cys Ala Asp Leu His Ile Gln Asn Lys Thr Asn
               20                  25                  30
   tgt ccc atc ttc aat ata aga tgt aat gac aag tgc tac tgt gaa gat       144
   Cys Pro Ile Phe Asn Ile Arg Cys Asn Asp Lys Cys Tyr Cys Glu Asp
           35                  40                  45
   ggc tat gca agg gat gtc aat ggc aaa tgt ata ccg ata aaa gac tgt       192
   Gly Tyr Ala Arg Asp Val Asn Gly Lys Cys Ile Pro Ile Lys Asp Cys
       50                  55                  60
   cct aaa ata cgt tcg                                                   207
   Pro Lys Ile Arg Ser
   65
<210> 40
   <211> 69
   <212> PRT
   <213> Galleria mellonella
<400> 40
   Ile Val Leu Ile Cys Asn Gly Gly His Glu Tyr Tyr Glu Cys Gly Gly
   1               5                   10                  15
   Ala Cys Asp Asn Val Cys Ala Asp Leu His Ile Gln Asn Lys Thr Asn
               20                  25                  30
   Cys Pro Ile Phe Asn Ile Arg Cys Asn Asp Lys Cys Tyr Cys Glu Asp
           35                  40                  45
   Gly Tyr Ala Arg Asp Val Asn Gly Lys Cys Ile Pro Ile Lys Asp Cys
       50                  55                  60
   Pro Lys Ile Arg Ser
   65
<210> 41
   <211> 207
   <212> DNA
   <213> Galleria mellonella
<220>
   <221> CDS
   <222> (1)..(207)
<220>
   <221> misc_feature
   <222> (7)..(7)
   <223> n is a, c, g, or t
<220>
   <221> misc_feature
   <222> (17)..(17)
   <223> n is a, c, g, or t
<220>
   <221> misc_feature
   <222> (33)..(33)
   <223> n is a, c, g, or t
<220>
   <221> misc_feature
   <222> (35)..(36)
   <223> n is a, c, g, or t
<220>
   <221> misc_feature
   <222> (38)..(38)
   <223> n is a, c, g, or t
<220>
   <221> misc_feature
   <222> (87)..(88)
   <223> n is a, c, g, or t
<220>
   <221> misc_feature
   <222> (186)..(186)
   <223> n is a, c, g, or t
<220>
   <221> misc_feature
   <222> (188)..(189)
   <223> n is a, c, g, or t
<400> 41
   ata gtc nta att tgt anc ggt gga cac gaa tan tnn gng tgc ggt gga       48
   Ile Val Xaa Ile Cys Xaa Gly Gly His Glu Xaa Xaa Xaa Cys Gly Gly
   1               5                   10                  15
   gcc tgc gat aat gta tgt gca gat tta cat ata cag aan naa aca aac       96
   Ala Cys Asp Asn Val Cys Ala Asp Leu His Ile Gln Xaa Xaa Thr Asn
               20                  25                  30
   tgt ccc atc tgg aat ata aga tgt aat gac aag tgc tac tgt gaa gat      144
   Cys Pro Ile Trp Asn Ile Arg Cys Asn Asp Lys Cys Tyr Cys Glu Asp
           35                  40                  45
   ggc tat gca agg gat gtc aat ggc aaa tgt ata ccg att aan gnn tgt      192
   Gly Tyr Ala Arg Asp Val Asn Gly Lys Cys Ile Pro Ile Xaa Xaa Cys
       50                  55                  60
   cct aaa ata cgt tcg                                                  207
   Pro Lys Ile Arg Ser
   65
<210> 42
   <211> 69
   <212> PRT
   <213> Galleria mellonella
<220>
   <221> misc_feature
   <222> (3)..(3)
   <223> The 'Xaa' at location 3 stands for Ile, Val, or Leu.
<220>
   <221> misc_feature
   <222> (6)..(6)
   <223> The 'Xaa' at location 6 stands for Asn, Ser, Thr, or Ile.
<220>
   <221> misc_feature
   <222> (11)..(11)
   <223> The 'Xaa' at location 11 stands for Tyr.
<220>
   <221> misc_feature
   <222> (12)..(12)
   <223> The 'Xaa' at location 12 stands for Tyr, Trp, Cys, Ser, Leu, or Phe.
<220>
   <221> misc_feature
   <222> (13)..(13)
   <223> The 'Xaa' at location 13 stands for Glu, Gly, Ala, or Val.
<220>
   <221> misc_feature
   <222> (29)..(29)
   <223> The 'Xaa' at location 29 stands for Lys, or Asn.
<220>
   <221> misc_feature
   <222> (30)..(30)
   <223> The 'Xaa' at location 30 stands for Lys, Glu, or Gln.
<220>
   <221> misc_feature
   <222> (62)..(62)
   <223> The 'Xaa' at location 62 stands for Lys, or Asn.
<220>
   <221> misc_feature
   <222> (63)..(63)
   <223> The 'Xaa' at location 63 stands for Glu, Asp, Gly, Ala, or Val.
<400> 42
   Ile Val Xaa Ile Cys Xaa Gly Gly His Glu Xaa Xaa Xaa Cys Gly Gly
   1               5                   10                  15
   Ala Cys Asp Asn Val Cys Ala Asp Leu His Ile Gln Xaa Xaa Thr Asn
               20                  25                  30
   Cys Pro Ile Trp Asn Ile Arg Cys Asn Asp Lys Cys Tyr Cys Glu Asp
           35                  40                  45
   Gly Tyr Ala Arg Asp Val Asn Gly Lys Cys Ile Pro Ile Xaa Xaa Cys
       50                  55                  60
   Pro Lys Ile Arg Ser
   65
<210> 43
   <211> 207
   <212> DNA
   <213> Galleria mellonella
<220>
   <221> CDS
   <222> (1)..(207)
<400> 43
   ata gtc cta att tgt aac ggt gga cac gaa tac tac gag tgc ggt gga      48
   Ile Val Leu Ile Cys Asn Gly Gly His Glu Tyr Tyr Glu Cys Gly Gly
   1               5                   10                  15
   gcc tgc gat aat gta tgt gca gat tta cat ata cag aat aaa aca aac      96
   Ala Cys Asp Asn Val Cys Ala Asp Leu His Ile Gln Asn Lys Thr Asn
               20                  25                  30
   tgt ccc atc tac aat ata aga tgt aat gac aag tgc tac tgt gaa gat     144
   Cys Pro Ile Tyr Asn Ile Arg Cys Asn Asp Lys Cys Tyr Cys Glu Asp
           35                  40                  45
   ggc tat gca agg gat gtc aat ggc aaa tgt ata ccg ata aaa gac tgt     192
   Gly Tyr Ala Arg Asp Val Asn Gly Lys Cys Ile Pro Ile Lys Asp Cys
       50                  55                  60
   cct aaa ata cgt tcg                                                  207
   Pro Lys Ile Arg Ser
   65
<210> 44
   <211> 69
   <212> PRT
   <213> Galleria mellonella
<400> 44
   Ile Val Leu Ile Cys Asn Gly Gly His Glu Tyr Tyr Glu Cys Gly Gly
   1               5                   10                  15
   Ala Cys Asp Asn Val Cys Ala Asp Leu His Ile Gln Asn Lys Thr Asn
               20                  25                  30
   Cys Pro Ile Tyr Asn Ile Arg Cys Asn Asp Lys Cys Tyr Cys Glu Asp
           35                  40                  45
   Gly Tyr Ala Arg Asp Val Asn Gly Lys Cys Ile Pro Ile Lys Asp Cys
       50                  55                  60
   Pro Lys Ile Arg Ser
   65
<210> 45
   <211> 207
   <212> DNA
   <213> Galleria mellonella
<220>
   <221> CDS
   <222> (1)..(207)
<400> 45
   ata gtc cta att tgt aac ggt gga cac gaa tac tac gag tgc ggt gga       48
   Ile Val Leu Ile Cys Asn Gly Gly His Glu Tyr Tyr Glu Cys Gly Gly
   1               5                   10                  15
   gcc tgc gat aat gta tgt gca gat tta cat ata cag aat aaa aca aac       96
   Ala Cys Asp Asn Val Cys Ala Asp Leu His Ile Gln Asn Lys Thr Asn
               20                  25                  30
   tgt ccc atc agt aat ata aga tgt aat gac aag tgc tac tgt gaa gat      144
   Cys Pro Ile Ser Asn Ile Arg Cys Asn Asp Lys Cys Tyr Cys Glu Asp
           35                  40                  45
   ggc tat gca agg gat gtc aat ggc aaa tgt ata ccg ata aaa gac tgt      192
   Gly Tyr Ala Arg Asp Val Asn Gly Lys Cys Ile Pro Ile Lys Asp Cys
       50                  55                  60
   cct aaa ata cgt tcg                                                  207
   Pro Lys Ile Arg Ser
   65
<210> 46
   <211> 69
   <212> PRT
   <213> Galleria mellonella
<400> 46
   Ile Val Leu Ile Cys Asn Gly Gly His Glu Tyr Tyr Glu Cys Gly Gly
   1               5                   10                  15
   Ala Cys Asp Asn Val Cys Ala Asp Leu His Ile Gln Asn Lys Thr Asn
               20                  25                  30
   Cys Pro Ile Ser Asn Ile Arg Cys Asn Asp Lys Cys Tyr Cys Glu Asp
   35 40 45
   Gly Tyr Ala Arg Asp Val Asn Gly Lys Cys Ile Pro Ile Lys Asp Cys
   50 55 60
   Pro Lys Ile Arg Ser
   65
<210> 47
   <211> 207
   <212> DNA
   <213> Galleria mellonella
<220>
   <221> CDS
   <222> (1)..(207)
<400> 47
   ata gtc cta att tgt aac ggt gga cac gaa tac tac gag tgc ggt gga 48
   Ile Val Leu Ile Cys Asn Gly Gly His Glu Tyr Tyr Glu Cys Gly Gly
   1 5 10 15
   gcc tgc gat aat gta tgt gca gat tta cat ata cag aat aaa aca aac 96
   Ala Cys Asp Asn Val Cys Ala Asp Leu His Ile Gln Asn Lys Thr Asn
   20 25 30
   tgt ccc atc act aat ata aga tgt aat gac aag tgc tac tgt gaa gat 144
   Cys Pro Ile Thr Asn Ile Arg Cys Asn Asp Lys Cys Tyr Cys Glu Asp
   35 40 45
   ggc tat gca agg gat gtc aat ggc aaa tgt ata ccg ata aaa gac tgt 192
   Gly Tyr Ala Arg Asp Val Asn Gly Lys Cys Ile Pro Ile Lys Asp Cys
   50 55 60
   cct aaa ata cgt tcg 207
   Pro Lys Ile Arg Ser
   65
<210> 48
   <211> 69
   <212> PRT
   <213> Galleria mellonella
<400> 48
   Ile Val Leu Ile Cys Asn Gly Gly His Glu Tyr Tyr Glu Cys Gly Gly
   1 5 10 15
   Ala Cys Asp Asn Val Cys Ala Asp Leu His Ile Gln Asn Lys Thr Asn
   20 25 30
   Cys Pro Ile Thr Asn Ile Arg Cys Asn Asp Lys Cys Tyr Cys Glu Asp
   35 40 45
   Gly Tyr Ala Arg Asp Val Asn Gly Lys Cys Ile Pro Ile Lys Asp Cys
       50                  55                  60
   Pro Lys Ile Arg Ser
   65
<210> 49
   <211> 207
   <212> DNA
   <213> Galleria mellonella
<220>
   <221> CDS
   <222> (1)..(207)
<400> 49
   ata gtc cta att tgt aac ggt gga cac gaa tac tac gag tgc ggt gga       48
   Ile Val Leu Ile Cys Asn Gly Gly His Glu Tyr Tyr Glu Cys Gly Gly
   1               5                   10                  15
   gcc tgc gat aat gta tgt gca gat tta cat ata cag aat aaa aca aac       96
   Ala Cys Asp Asn Val Cys Ala Asp Leu His Ile Gln Asn Lys Thr Asn
               20                  25                  30
   tgt ccc atc aat aat ata aga tgt aat gac aag tgc tac tgt gaa gat      144
   Cys Pro Ile Asn Asn Ile Arg Cys Asn Asp Lys Cys Tyr Cys Glu Asp
           35                  40                  45
   ggc tat gca agg gat gtc aat ggc aaa tgt ata ccg ata aaa gac tgt      192
   Gly Tyr Ala Arg Asp Val Asn Gly Lys Cys Ile Pro Ile Lys Asp Cys
       50                  55                  60
   cct aaa ata cgt tcg                                                  207
   Pro Lys Ile Arg Ser
   65
<210> 50
   <211> 69
   <212> PRT
   <213> Galleria mellonella
<400> 50
   Ile Val Leu Ile Cys Asn Gly Gly His Glu Tyr Tyr Glu Cys Gly Gly
   1               5                   10                  15
   Ala Cys Asp Asn Val Cys Ala Asp Leu His Ile Gln Asn Lys Thr Asn
               20                  25                  30
   Cys Pro Ile Asn Asn Ile Arg Cys Asn Asp Lys Cys Tyr Cys Glu Asp
           35                  40                  45
   Gly Tyr Ala Arg Asp Val Asn Gly Lys Cys Ile Pro Ile Lys Asp Cys
       50                  55                  60
   Pro Lys Ile Arg Ser
   65
<210> 51
   <211> 207
   <212> DNA
   <213> Galleria mellonella
<220>
   <221> CDS
   <222> (1)..(207)
<400> 51
   ata gtc cta att tgt aac ggt gga cac gaa tac tac gag tgc ggt gga       48
   Ile Val Leu Ile Cys Asn Gly Gly His Glu Tyr Tyr Glu Cys Gly Gly
   1               5                   10                  15
   gcc tgc gat aat gta tgt gca gat tta cat ata cag aat aaa aca aac       96
   Ala Cys Asp Asn Val Cys Ala Asp Leu His Ile Gln Asn Lys Thr Asn
               20                  25                  30
   tgt ccc atc cag aat ata aga tgt aat gac aag tgc tac tgt gaa gat      144
   Cys Pro Ile Gln Asn Ile Arg Cys Asn Asp Lys Cys Tyr Cys Glu Asp
           35                  40                  45
   ggc tat gca agg gat gtc aat ggc aaa tgt ata ccg ata aaa gac tgt      192
   Gly Tyr Ala Arg Asp Val Asn Gly Lys Cys Ile Pro Ile Lys Asp Cys
       50                  55                  60
   cct aaa ata cgt tcg                                                  207
   Pro Lys Ile Arg Ser
   65
<210> 52
   <211> 69
   <212> PRT
   <213> Galleria mellonella
<400> 52
   Ile Val Leu Ile Cys Asn Gly Gly His Glu Tyr Tyr Glu Cys Gly Gly
   1               5                   10                  15
   Ala Cys Asp Asn Val Cys Ala Asp Leu His Ile Gln Asn Lys Thr Asn
               20                  25                  30
   Cys Pro Ile Gln Asn Ile Arg Cys Asn Asp Lys Cys Tyr Cys Glu Asp
           35                  40                  45
   Gly Tyr Ala Arg Asp Val Asn Gly Lys Cys Ile Pro Ile Lys Asp Cys
       50                  55                  60
   Pro Lys Ile Arg Ser
   65
<210> 53
   <211> 207
   <212> DNA
   <213> Galleria mellonella
<220>
   <221> CDS
   <222> (1)..(207)
<400> 53
   ata gtc cta att tgt aac ggt gga cac gaa tac tac gag tgc ggt gga       48
   Ile Val Leu Ile Cys Asn Gly Gly His Glu Tyr Tyr Glu Cys Gly Gly
   1               5                   10                  15
   gcc tgc gat aat gta tgt gca gat tta cat ata cag aat aaa aca aac       96
   Ala Cys Asp Asn Val Cys Ala Asp Leu His Ile Gln Asn Lys Thr Asn
               20                  25                  30
   tgt ccc atc cat aat ata aga tgt aat gac aag tgc tac tgt gaa gat      144
   Cys Pro Ile His Asn Ile Arg Cys Asn Asp Lys Cys Tyr Cys Glu Asp
           35                  40                  45
   ggc tat gca agg gat gtc aat ggc aaa tgt ata ccg ata aaa gac tgt      192
   Gly Tyr Ala Arg Asp Val Asn Gly Lys Cys Ile Pro Ile Lys Asp Cys
       50                  55                  60
   cct aaa ata cgt tcg                                                  207
   Pro Lys Ile Arg Ser
   65
<210> 54
   <211> 69
   <212> PRT
   <213> Galleria mellonella
<400> 54
   Ile Val Leu Ile Cys Asn Gly Gly His Glu Tyr Tyr Glu Cys Gly Gly
   1               5                   10                  15
   Ala Cys Asp Asn Val Cys Ala Asp Leu His Ile Gln Asn Lys Thr Asn
               20                  25                  30
   Cys Pro Ile His Asn Ile Arg Cys Asn Asp Lys Cys Tyr Cys Glu Asp
           35                  40                  45
   Gly Tyr Ala Arg Asp Val Asn Gly Lys Cys Ile Pro Ile Lys Asp Cys
       50                  55                  60
   Pro Lys Ile Arg Ser
   65
<210> 55
   <211> 207
   <212> DNA
   <213> Galleria mellonella
<220>
   <221> CDS
   <222> (1)..(207)
<400> 55
   ata gtc cta att tgt aac ggt gga cac gaa tac tac gag tgc ggt gga      48
   Ile Val Leu Ile Cys Asn Gly Gly His Glu Tyr Tyr Glu Cys Gly Gly
   1               5                   10                  15
   gcc tgc gat aat gta tgt gca gat tta cat ata cag aat aaa aca aac      96
   Ala Cys Asp Asn Val Cys Ala Asp Leu His Ile Gln Asn Lys Thr Asn
               20                  25                  30
   tgt ccc atc cgt aat ata aga tgt aat gac aag tgc tac tgt gaa gat      144
   Cys Pro Ile Arg Asn Ile Arg Cys Asn Asp Lys Cys Tyr Cys Glu Asp
           35                  40                  45
   ggc tat gca agg gat gtc aat ggc aaa tgt ata ccg ata aaa gac tgt      192
   Gly Tyr Ala Arg Asp Val Asn Gly Lys Cys Ile Pro Ile Lys Asp Cys
       50                  55                  60
   cct aaa ata cgt tcg                                                  207
   Pro Lys Ile Arg Ser
   65
<210> 56
   <211> 69
   <212> PRT
   <213> Galleria mellonella
<400> 56
   Ile Val Leu Ile Cys Asn Gly Gly His Glu Tyr Tyr Glu Cys Gly Gly
   1               5                   10                  15
   Ala Cys Asp Asn Val Cys Ala Asp Leu His Ile Gln Asn Lys Thr Asn
               20                  25                  30
   Cys Pro Ile Arg Asn Ile Arg Cys Asn Asp Lys Cys Tyr Cys Glu Asp
           35                  40                  45
   Gly Tyr Ala Arg Asp Val Asn Gly Lys Cys Ile Pro Ile Lys Asp Cys
       50                  55                  60
   Pro Lys Ile Arg Ser
   65
<210> 57
   <211> 207
   <212> DNA
   <213> Galleria mellonella
<220>
   <221> CDS
   <222> (1)..(207)
<400> 57
   ata gtc cta att tgt aac ggt gga cac gaa tac tac gag tgc ggt gga       48
   Ile Val Leu Ile Cys Asn Gly Gly His Glu Tyr Tyr Glu Cys Gly Gly
   1               5                   10                  15
   gcc tgc gat aat gta tgt gca gat tta cat ata cag aat aaa aca aac       96
   Ala Cys Asp Asn Val Cys Ala Asp Leu His Ile Gln Asn Lys Thr Asn
               20                  25                  30
   tgt ccc atc aag aat ata aga tgt aat gac aag tgc tac tgt gaa gat      144
   Cys Pro Ile Lys Asn Ile Arg Cys Asn Asp Lys Cys Tyr Cys Glu Asp
           35                  40                  45
   ggc tat gca agg gat gtc aat ggc aaa tgt ata ccg ata aaa gac tgt      192
   Gly Tyr Ala Arg Asp Val Asn Gly Lys Cys Ile Pro Ile Lys Asp Cys
       50                  55                  60
   cct aaa ata cgt tcg                                                  207
   Pro Lys Ile Arg Ser
   65
<210> 58
   <211> 69
   <212> PRT
   <213> Galleria mellonella
<400> 58
   Ile Val Leu Ile Cys Asn Gly Gly His Glu Tyr Tyr Glu Cys Gly Gly
   1               5                   10                  15
   Ala Cys Asp Asn Val Cys Ala Asp Leu His Ile Gln Asn Lys Thr Asn
               20                  25                  30
   Cys Pro Ile Lys Asn Ile Arg Cys Asn Asp Lys Cys Tyr Cys Glu Asp
           35                  40                  45
   Gly Tyr Ala Arg Asp Val Asn Gly Lys Cys Ile Pro Ile Lys Asp Cys
       50                  55                  60
   Pro Lys Ile Arg Ser
   65
<210> 59
   <211> 207
   <212> DNA
   <213> Galleria mellonella
<220>
   <221> CDS
   <222> (1)..(207)
<400> 59
   ata gtc cta att tgt aac ggt gga cac gaa tac tac gag tgc ggt gga       48
   Ile Val Leu Ile Cys Asn Gly Gly His Glu Tyr Tyr Glu Cys Gly Gly
   1               5                   10                  15
   gcc tgc gat aat gta tgt gca gat tta cat ata cag aat aaa aca aac       96
   Ala Cys Asp Asn Val Cys Ala Asp Leu His Ile Gln Asn Lys Thr Asn
               20                  25                  30
   tgt ccc atc att aat ata gta tgt aat gac aag tgc tac tgt gaa gat      144
   Cys Pro Ile Ile Asn Ile Val Cys Asn Asp Lys Cys Tyr Cys Glu Asp
           35                  40                  45
   ggc tat gca agg gat gtc aat ggc aaa tgt ata ccg ata aaa gac tgt      192
   Gly Tyr Ala Arg Asp Val Asn Gly Lys Cys Ile Pro Ile Lys Asp Cys
       50                  55                  60
   cct aaa ata cgt tcg                                                  207
   Pro Lys Ile Arg Ser
   65
<210> 60
   <211> 69
   <212> PRT
   <213> Galleria mellonella
<400> 60
   Ile Val Leu Ile Cys Asn Gly Gly His Glu Tyr Tyr Glu Cys Gly Gly
   1               5                   10                  15
   Ala Cys Asp Asn Val Cys Ala Asp Leu His Ile Gln Asn Lys Thr Asn
               20                  25                  30
   Cys Pro Ile Ile Asn Ile Val Cys Asn Asp Lys Cys Tyr Cys Glu Asp
           35                  40                  45
   Gly Tyr Ala Arg Asp Val Asn Gly Lys Cys Ile Pro Ile Lys Asp Cys
       50                  55                  60
   Pro Lys Ile Arg Ser
   65
<210> 61
   <211> 207
   <212> DNA
   <213> Galleria mellonella
<220>
   <221> CDS
   <222> (1)..(207)
<400> 61
   ata gtc cta att tgt aac ggt gga cac gaa tac tac gag tgc ggt gga       48
   Ile Val Leu Ile Cys Asn Gly Gly His Glu Tyr Tyr Glu Cys Gly Gly
   1               5                   10                  15
   gcc tgc gat aat gta tgt gca gat tta cat ata cag aat aaa aca aac       96
   Ala Cys Asp Asn Val Cys Ala Asp Leu His Ile Gln Asn Lys Thr Asn
               20                  25                  30
   tgt ccc atc att aat ata aaa tgt aat gac aag tgc tac tgt gaa gat      144
   Cys Pro Ile Ile Asn Ile Lys Cys Asn Asp Lys Cys Tyr Cys Glu Asp
           35                  40                  45
   ggc tat gca agg gat gtc aat ggc aaa tgt ata ccg ata aaa gac tgt      192
   Gly Tyr Ala Arg Asp Val Asn Gly Lys Cys Ile Pro Ile Lys Asp Cys
       50                  55                  60
   cct aaa ata cgt tcg                                                  207
   Pro Lys Ile Arg Ser
   65
<210> 62
   <211> 69
   <212> PRT
   <213> Galleria mellonella
<400> 62
   Ile Val Leu Ile Cys Asn Gly Gly His Glu Tyr Tyr Glu Cys Gly Gly
   1               5                   10                  15
   Ala Cys Asp Asn Val Cys Ala Asp Leu His Ile Gln Asn Lys Thr Asn
               20                  25                  30
   Cys Pro Ile Ile Asn Ile Lys Cys Asn Asp Lys Cys Tyr Cys Glu Asp
           35                  40                  45
   Gly Tyr Ala Arg Asp Val Asn Gly Lys Cys Ile Pro Ile Lys Asp Cys
       50                  55                  60
   Pro Lys Ile Arg Ser
   65
<210> 63
   <211> 207
   <212> DNA
   <213> Galleria mellonella
<220>
   <221> CDS
   <222> (1)..(207)
<400> 63
   ata gtc cta att tgt aac ggt gga cac gaa tac tac gag tgc ggt gga       48
   Ile Val Leu Ile Cys Asn Gly Gly His Glu Tyr Tyr Glu Cys Gly Gly
   1               5                   10                  15
   gcc tgc gat aat gta tgt gca gat tta cat ata cag aat aaa aca aac       96
   Ala Cys Asp Asn Val Cys Ala Asp Leu His Ile Gln Asn Lys Thr Asn
               20                  25                  30
   tgt ccc tgg att aat ata aga tgt aat gac aag tgc tac tgt gaa gat      144
   Cys Pro Trp Ile Asn Ile Arg Cys Asn Asp Lys Cys Tyr Cys Glu Asp
           35                  40                  45
   ggc tat gca agg gat gtc aat ggc aaa tgt ata ccg ata aaa gac tgt      192
   Gly Tyr Ala Arg Asp Val Asn Gly Lys Cys Ile Pro Ile Lys Asp Cys
       50                  55                  60
   cct aaa ata cgt tcg                                                  207
   Pro Lys Ile Arg Ser
   65
<210> 64
   <211> 69
   <212> PRT
   <213> Galleria mellonella
<400> 64
   Ile Val Leu Ile Cys Asn Gly Gly His Glu Tyr Tyr Glu Cys Gly Gly
   1               5                   10                  15
   Ala Cys Asp Asn Val Cys Ala Asp Leu His Ile Gln Asn Lys Thr Asn
               20                  25                  30
   Cys Pro Trp Ile Asn Ile Arg Cys Asn Asp Lys Cys Tyr Cys Glu Asp
           35                  40                  45
   Gly Tyr Ala Arg Asp Val Asn Gly Lys Cys Ile Pro Ile Lys Asp Cys
       50                  55                  60
   Pro Lys Ile Arg Ser
   65
<210> 65
   <211> 207
   <212> DNA
   <213> Galleria mellonella
<220>
   <221> CDS
   <222> (1)..(207)
<400> 65
   ata gtc cta att tgt aac ggt gga cac gaa tac tac gag tgc ggt gga        48
   Ile Val Leu Ile Cys Asn Gly Gly His Glu Tyr Tyr Glu Cys Gly Gly
   1               5                   10                  15
   gcc tgc gat aat gta tgt gca gat tta cat ata cag aat aaa aca aac        96
   Ala Cys Asp Asn Val Cys Ala Asp Leu His Ile Gln Asn Lys Thr Asn
               20                  25                  30
   tgt ccc tac att aat ata aga tgt aat gac aag tgc tac tgt gaa gat       144
   Cys Pro Tyr Ile Asn Ile Arg Cys Asn Asp Lys Cys Tyr Cys Glu Asp
           35                  40                  45
   ggc tat gca agg gat gtc aat ggc aaa tgt ata ccg ata aaa gac tgt       192
   Gly Tyr Ala Arg Asp Val Asn Gly Lys Cys Ile Pro Ile Lys Asp Cys
       50                  55                  60
   cct aaa ata cgt tcg                                                   207
   Pro Lys Ile Arg Ser
   65
<210> 66
   <211> 69
   <212> PRT
   <213> Galleria mellonella
<400> 66
   Ile Val Leu Ile Cys Asn Gly Gly His Glu Tyr Tyr Glu Cys Gly Gly
   1               5                   10                  15
   Ala Cys Asp Asn Val Cys Ala Asp Leu His Ile Gln Asn Lys Thr Asn
               20                  25                  30
   Cys Pro Tyr Ile Asn Ile Arg Cys Asn Asp Lys Cys Tyr Cys Glu Asp
           35                  40                  45
   Gly Tyr Ala Arg Asp Val Asn Gly Lys Cys Ile Pro Ile Lys Asp Cys
       50                  55                  60
   Pro Lys Ile Arg Ser
   65
<210> 67
   <211> 207
   <212> DNA
   <213> Galleria mellonella
<220>
   <221> CDS
   <222> (1)..(207)
<400> 67
   ata gtc cta att tgt aac ggt gga cac gaa tac tac gag tgc ggt gga        48
   Ile Val Leu Ile Cys Asn Gly Gly His Glu Tyr Tyr Glu Cys Gly Gly
   1               5                   10                  15
   gcc tgc gat aat gta tgt gca gat tta cat ata cag aat aaa aca aac        96
   Ala Cys Asp Asn Val Cys Ala Asp Leu His Ile Gln Asn Lys Thr Asn
               20                  25                  30
   tgt ccc atc att aat ata gca tgt aat gac aag tgc tac tgt gaa gat        144
   Cys Pro Ile Ile Asn Ile Ala Cys Asn Asp Lys Cys Tyr Cys Glu Asp
           35                  40                  45
   ggc tat gca agg gat gtc aat ggc aaa tgt ata ccg ata aaa gac tgt        192
   Gly Tyr Ala Arg Asp Val Asn Gly Lys Cys Ile Pro Ile Lys Asp Cys
       50                  55                  60
   cct aaa ata cgt tcg                                                    207
   Pro Lys Ile Arg Ser
   65
<210> 68
   <211> 69
   <212> PRT
   <213> Galleria mellonella
<400> 68
   Ile Val Leu Ile Cys Asn Gly Gly His Glu Tyr Tyr Glu Cys Gly Gly
   1               5                   10                  15
   Ala Cys Asp Asn Val Cys Ala Asp Leu His Ile Gln Asn Lys Thr Asn
               20                  25                  30
   Cys Pro Ile Ile Asn Ile Ala Cys Asn Asp Lys Cys Tyr Cys Glu Asp
           35                  40                  45
   Gly Tyr Ala Arg Asp Val Asn Gly Lys Cys Ile Pro Ile Lys Asp Cys
       50                  55                  60
   Pro Lys Ile Arg Ser
   65
<210> 69
   <211> 207
   <212> DNA
   <213> Galleria mellonella
<220>
   <221> CDS
   <222> (1)..(207)
<400> 69
   ata gtc cta att tgt aac ggt gga cac gaa tac tac gag tgc ggt gga       48
   Ile Val Leu Ile Cys Asn Gly Gly His Glu Tyr Tyr Glu Cys Gly Gly
   1               5                   10                  15
   gcc tgc gat aat gta tgt gca gat tta cat ata cag aat aaa aca aac       96
   Ala Cys Asp Asn Val Cys Ala Asp Leu His Ile Gln Asn Lys Thr Asn
               20                  25                  30
   tgt ccc tgt att aat ata aga tgt aat gac aag tgc tac tgt gaa gat      144
   Cys Pro Cys Ile Asn Ile Arg Cys Asn Asp Lys Cys Tyr Cys Glu Asp
           35                  40                  45
   ggc tat gca agg gat gtc aat ggc aaa tgt ata ccg ata aaa gac tgt      192
   Gly Tyr Ala Arg Asp Val Asn Gly Lys Cys Ile Pro Ile Lys Asp Cys
       50                  55                  60
   cct aaa ata cgt tcg                                                  207
   Pro Lys Ile Arg Ser
   65
<210> 70
   <211> 69
   <212> PRT
   <213> Galleria mellonella
<400> 70
   Ile Val Leu Ile Cys Asn Gly Gly His Glu Tyr Tyr Glu Cys Gly Gly
   1               5                   10                  15
   Ala Cys Asp Asn Val Cys Ala Asp Leu His Ile Gln Asn Lys Thr Asn
               20                  25                  30
   Cys Pro Cys Ile Asn Ile Arg Cys Asn Asp Lys Cys Tyr Cys Glu Asp
           35                  40                  45
   Gly Tyr Ala Arg Asp Val Asn Gly Lys Cys Ile Pro Ile Lys Asp Cys
       50                  55                  60
   Pro Lys Ile Arg Ser
   65
<210> 71
   <211> 207
   <212> DNA
   <213> Galleria mellonella
<220>
   <221> CDS
   <222> (1)..(207)
<400> 71
   ata gtc cta att tgt aac ggt gga cac gaa tac tac gag tgc ggt gga       48
   Ile Val Leu Ile Cys Asn Gly Gly His Glu Tyr Tyr Glu Cys Gly Gly
   1               5                   10                  15
   gcc tgc gat aat gta tgt gca gat tta cat ata cag aat aaa aca aac       96
   Ala Cys Asp Asn Val Cys Ala Asp Leu His Ile Gln Asn Lys Thr Asn
               20                  25                  30
   tgt ccc aag att aat ata aga tgt aat gac aag tgc tac tgt gaa gat      144
   Cys Pro Lys Ile Asn Ile Arg Cys Asn Asp Lys Cys Tyr Cys Glu Asp
           35                  40                  45
   ggc tat gca agg gat gtc aat ggc aaa tgt ata ccg ata aaa gac tgt      192
   Gly Tyr Ala Arg Asp Val Asn Gly Lys Cys Ile Pro Ile Lys Asp Cys
       50                  55                  60
   cct aaa ata cgt tcg                                                  207
   Pro Lys Ile Arg Ser
   65
<210> 72
   <211> 69
   <212> PRT
   <213> Galleria mellonella
<400> 72
   Ile Val Leu Ile Cys Asn Gly Gly His Glu Tyr Tyr Glu Cys Gly Gly
   1               5                   10                  15
   Ala Cys Asp Asn Val Cys Ala Asp Leu His Ile Gln Asn Lys Thr Asn
               20                  25                  30
   Cys Pro Lys Ile Asn Ile Arg Cys Asn Asp Lys Cys Tyr Cys Glu Asp
           35                  40                  45
   Gly Tyr Ala Arg Asp Val Asn Gly Lys Cys Ile Pro Ile Lys Asp Cys
       50                  55                  60
   Pro Lys Ile Arg Ser
   65
<210> 73
   <211> 207
   <212> DNA
   <213> Galleria mellonella
<220>
   <221> CDS
   <222> (1)..(207)
<400> 73
   ata gtc cta att tgt aac ggt gga cac gaa tac tac gag tgc ggt gga       48
   Ile Val Leu Ile Cys Asn Gly Gly His Glu Tyr Tyr Glu Cys Gly Gly
   1               5                   10                  15
   gcc tgc gat aat gta tgt gca gat tta cat ata cag aat aaa aca aac       96
   Ala Cys Asp Asn Val Cys Ala Asp Leu His Ile Gln Asn Lys Thr Asn
               20                  25                  30
   tgt ccc cgt att aat ata aga tgt aat gac aag tgc tac tgt gaa gat      144
   Cys Pro Arg Ile Asn Ile Arg Cys Asn Asp Lys Cys Tyr Cys Glu Asp
           35                  40                  45
   ggc tat gca agg gat gtc aat ggc aaa tgt ata ccg ata aaa gac tgt      192
   Gly Tyr Ala Arg Asp Val Asn Gly Lys Cys Ile Pro Ile Lys Asp Cys
       50                  55                  60
   cct aaa ata cgt tcg                                                  207
   Pro Lys Ile Arg Ser
   65
<210> 74
   <211> 69
   <212> PRT
   <213> Galleria mellonella
<400> 74
   Ile Val Leu Ile Cys Asn Gly Gly His Glu Tyr Tyr Glu Cys Gly Gly
   1               5                   10                  15
   Ala Cys Asp Asn Val Cys Ala Asp Leu His Ile Gln Asn Lys Thr Asn
               20                  25                  30
   Cys Pro Arg Ile Asn Ile Arg Cys Asn Asp Lys Cys Tyr Cys Glu Asp
           35                  40                  45
   Gly Tyr Ala Arg Asp Val Asn Gly Lys Cys Ile Pro Ile Lys Asp Cys
       50                  55                  60
   Pro Lys Ile Arg Ser
   65
<210> 75
   <211> 207
   <212> DNA
   <213> Galleria mellonella
<220>
   <221> CDS
   <222> (1)..(207)
<400> 75
   ata gtc cta att tgt aac ggt gga cac gaa tac tac gag tgc ggt gga       48
   Ile Val Leu Ile Cys Asn Gly Gly His Glu Tyr Tyr Glu Cys Gly Gly
   1               5                   10                  15
   gcc tgc gat aat gta tgt gca gat tta cat ata cag aat aaa aca aac       96
   Ala Cys Asp Asn Val Cys Ala Asp Leu His Ile Gln Asn Lys Thr Asn
               20                  25                  30
   tgt ccc ttg att aat ata aga tgt aat gac aag tgc tac tgt gaa gat      144
   Cys Pro Leu Ile Asn Ile Arg Cys Asn Asp Lys Cys Tyr Cys Glu Asp
           35                  40                  45
   ggc tat gca agg gat gtc aat ggc aaa tgt ata ccg ata aaa gac tgt      192
   Gly Tyr Ala Arg Asp Val Asn Gly Lys Cys Ile Pro Ile Lys Asp Cys
       50                  55                  60
   cct aaa ata cgt tcg                                                  207
   Pro Lys Ile Arg Ser
   65
<210> 76
   <211> 69
   <212> PRT
   <213> Galleria mellonella
<400> 76
   Ile Val Leu Ile Cys Asn Gly Gly His Glu Tyr Tyr Glu Cys Gly Gly
   1               5                   10                  15
   Ala Cys Asp Asn Val Cys Ala Asp Leu His Ile Gln Asn Lys Thr Asn
               20                  25                  30
   Cys Pro Leu Ile Asn Ile Arg Cys Asn Asp Lys Cys Tyr Cys Glu Asp
           35                  40                  45
   Gly Tyr Ala Arg Asp Val Asn Gly Lys Cys Ile Pro Ile Lys Asp Cys
       50                  55                  60
   Pro Lys Ile Arg Ser
   65
<210> 77
   <211> 207
   <212> DNA
   <213> Galleria mellonella
<220>
   <221> CDS
   <222> (1)..(207)
<400> 77
   ata gtc cta att tgt aac ggt gga cac gaa tac tac gag tgc ggt gga        48
   Ile Val Leu Ile Cys Asn Gly Gly His Glu Tyr Tyr Glu Cys Gly Gly
   1               5                   10                  15
   gcc tgc gat aat gta tgt gca gat tta cat ata cag aat aaa aca aac        96
   Ala Cys Asp Asn Val Cys Ala Asp Leu His Ile Gln Asn Lys Thr Asn
               20                  25                  30
   tgt ccc atg att aat ata aga tgt aat gac aag tgc tac tgt gaa gat       144
   Cys Pro Met Ile Asn Ile Arg Cys Asn Asp Lys Cys Tyr Cys Glu Asp
           35                  40                  45
   ggc tat gca agg gat gtc aat ggc aaa tgt ata ccg ata aaa gac tgt       192
   Gly Tyr Ala Arg Asp Val Asn Gly Lys Cys Ile Pro Ile Lys Asp Cys
       50                  55                  60
   cct aaa ata cgt tcg                                                   207
   Pro Lys Ile Arg Ser
   65
<210> 78
   <211> 69
   <212> PRT
   <213> Galleria mellonella
<400> 78
   Ile Val Leu Ile Cys Asn Gly Gly His Glu Tyr Tyr Glu Cys Gly Gly
   1               5                   10                  15
   Ala Cys Asp Asn Val Cys Ala Asp Leu His Ile Gln Asn Lys Thr Asn
               20                  25                  30
   Cys Pro Met Ile Asn Ile Arg Cys Asn Asp Lys Cys Tyr Cys Glu Asp
           35                  40                  45
   Gly Tyr Ala Arg Asp Val Asn Gly Lys Cys Ile Pro Ile Lys Asp Cys
       50                  55                  60
   Pro Lys Ile Arg Ser
   65
<210> 79
   <211> 207
   <212> DNA
   <213> Galleria mellonella
<220>
   <221> CDS
   <222> (1)..(207)
<400> 79
   ata gtc cta att tgt aac ggt gga cac gaa tac tac gag tgc ggt gga        48
   Ile Val Leu Ile Cys Asn Gly Gly His Glu Tyr Tyr Glu Cys Gly Gly
   1               5                   10                  15
   gcc tgc gat aat gta tgt gca gat tta cat ata cag aat aaa aca aac        96
   Ala Cys Asp Asn Val Cys Ala Asp Leu His Ile Gln Asn Lys Thr Asn
               20                  25                  30
   tgt ccc ttc att aat ata aga tgt aat gac aag tgc tac tgt gaa gat       144
   Cys Pro Phe Ile Asn Ile Arg Cys Asn Asp Lys Cys Tyr Cys Glu Asp
           35                  40                  45
   ggc tat gca agg gat gtc aat ggc aaa tgt ata ccg ata aaa gac tgt       192
   Gly Tyr Ala Arg Asp Val Asn Gly Lys Cys Ile Pro Ile Lys Asp Cys
       50                  55                  60
   cct aaa ata cgt tcg                                                   207
   Pro Lys Ile Arg Ser
   65
<210> 80
   <211> 69
   <212> PRT
   <213> Galleria mellonella
<400> 80
   Ile Val Leu Ile Cys Asn Gly Gly His Glu Tyr Tyr Glu Cys Gly Gly
   1               5                   10                  15
   Ala Cys Asp Asn Val Cys Ala Asp Leu His Ile Gln Asn Lys Thr Asn
               20                  25                  30
   Cys Pro Phe Ile Asn Ile Arg Cys Asn Asp Lys Cys Tyr Cys Glu Asp
           35                  40                  45
   Gly Tyr Ala Arg Asp Val Asn Gly Lys Cys Ile Pro Ile Lys Asp Cys
       50                  55                  60
   Pro Lys Ile Arg Ser
   65
<210> 81
   <211> 207
   <212> DNA
   <213> Galleria mellonella
<220>
   <221> CDS
   <222> (1)..(207)
<400> 81
   ata gtc cta att tgt aac ggt gga cac gaa tac tac gag tgc ggt gga        48
   Ile Val Leu Ile Cys Asn Gly Gly His Glu Tyr Tyr Glu Cys Gly Gly
   1               5                   10                  15
   gcc tgc gat aat gta tgt gca gat tta cat ata cag aat aaa aca aac        96
   Ala Cys Asp Asn Val Cys Ala Asp Leu His Ile Gln Asn Lys Thr Asn
               20                  25                  30
   tgt ccc cag att aat ata aga tgt aat gac aag tgc tac tgt gaa gat       144
   Cys Pro Gln Ile Asn Ile Arg Cys Asn Asp Lys Cys Tyr Cys Glu Asp
           35                  40                  45
   ggc tat gca agg gat gtc aat ggc aaa tgt ata ccg ata aaa gac tgt       192
   Gly Tyr Ala Arg Asp Val Asn Gly Lys Cys Ile Pro Ile Lys Asp Cys
       50                  55                  60
   cct aaa ata cgt tcg                                                   207
   Pro Lys Ile Arg Ser
   65
<210> 82
   <211> 69
   <212> PRT
   <213> Galleria mellonella
<400> 82
   Ile Val Leu Ile Cys Asn Gly Gly His Glu Tyr Tyr Glu Cys Gly Gly
   1               5                   10                  15
   Ala Cys Asp Asn Val Cys Ala Asp Leu His Ile Gln Asn Lys Thr Asn
               20                  25                  30
   Cys Pro Gln Ile Asn Ile Arg Cys Asn Asp Lys Cys Tyr Cys Glu Asp
           35                  40                  45
   Gly Tyr Ala Arg Asp Val Asn Gly Lys Cys Ile Pro Ile Lys Asp Cys
       50                  55                  60
   Pro Lys Ile Arg Ser
   65
<210> 83
   <211> 207
   <212> DNA
   <213> Galleria mellonella
<220>
   <221> CDS
   <222> (1)..(207)
<400> 83
   ata gtc cta att tgt aac ggt gga cac gaa tac tac gag tgc ggt gga       48
   Ile Val Leu Ile Cys Asn Gly Gly His Glu Tyr Tyr Glu Cys Gly Gly
   1               5                   10                  15
   gcc tgc gat aat gta tgt gca gat tta cat ata cag aat aaa aca aac       96
   Ala Cys Asp Asn Val Cys Ala Asp Leu His Ile Gln Asn Lys Thr Asn
               20                  25                  30
   tgt ccc cac att aat ata aga tgt aat gac aag tgc tac tgt gaa gat      144
   Cys Pro His Ile Asn Ile Arg Cys Asn Asp Lys Cys Tyr Cys Glu Asp
           35                  40                  45
   ggc tat gca agg gat gtc aat ggc aaa tgt ata ccg ata aaa gac tgt      192
   Gly Tyr Ala Arg Asp Val Asn Gly Lys Cys Ile Pro Ile Lys Asp Cys
       50                  55                  60
   cct aaa ata cgt tcg                                                  207
   Pro Lys Ile Arg Ser
   65
<210> 84
   <211> 69
   <212> PRT
   <213> Galleria mellonella
<400> 84
   Ile Val Leu Ile Cys Asn Gly Gly His Glu Tyr Tyr Glu Cys Gly Gly
   1               5                   10                  15
   Ala Cys Asp Asn Val Cys Ala Asp Leu His Ile Gln Asn Lys Thr Asn
               20                  25                  30
   Cys Pro His Ile Asn Ile Arg Cys Asn Asp Lys Cys Tyr Cys Glu Asp
           35                  40                  45
   Gly Tyr Ala Arg Asp Val Asn Gly Lys Cys Ile Pro Ile Lys Asp Cys
       50                  55                  60
   Pro Lys Ile Arg Ser
   65
<210> 85
   <211> 1041
   <212> DNA
   <213> Galleria mellonella
<220>
   <221> CDS
   <222> (1)..(1041)
<400> 85
   atg tcc cct ata cta ggt tat tgg aaa att aag ggc ctt gtg caa ccc      48
   Met Ser Pro Ile Leu Gly Tyr Trp Lys Ile Lys Gly Leu Val Gln Pro
   1               5                   10                  15
   act cga ctt ctt ttg gaa tat ctt gaa gaa aaa tat gaa gag cat ttg      96
   Thr Arg Leu Leu Leu Glu Tyr Leu Glu Glu Lys Tyr Glu Glu His Leu
               20                  25                  30
   tat gag cgc gat gaa ggt gat aaa tgg cga aac aaa aag ttt gaa ttg     144
   Tyr Glu Arg Asp Glu Gly Asp Lys Trp Arg Asn Lys Lys Phe Glu Leu
           35                  40                  45
   ggt ttg gag ttt ccc aat ctt cct tat tat att gat ggt gat gtt aaa     192
   Gly Leu Glu Phe Pro Asn Leu Pro Tyr Tyr Ile Asp Gly Asp Val Lys
       50                  55                  60
   tta aca cag tct atg gcc atc ata cgt tat ata gct gac aag cac aac     240
   Leu Thr Gln Ser Met Ala Ile Ile Arg Tyr Ile Ala Asp Lys His Asn
   65                  70                  75                  80
   atg ttg ggt ggt tgt cca aaa gag cgt gca gag att tca atg ctt gaa     288
   Met Leu Gly Gly Cys Pro Lys Glu Arg Ala Glu Ile Ser Met Leu Glu
                   85                  90                  95
   gga gcg gtt ttg gat att aga tac ggt gtt tcg aga att gca tat agt     336
   Gly Ala Val Leu Asp Ile Arg Tyr Gly Val Ser Arg Ile Ala Tyr Ser
               100                 105                 110
   aaa gac ttt gaa act ctc aaa gtt gat ttt ctt agc aag cta cct gaa     384
   Lys Asp Phe Glu Thr Leu Lys Val Asp Phe Leu Ser Lys Leu Pro Glu
           115                 120                 125
   atg ctg aaa atg ttc gaa gat cgt tta tgt cat aaa aca tat tta aat     432
   Met Leu Lys Met Phe Glu Asp Arg Leu Cys His Lys Thr Tyr Leu Asn
       130                 135                 140
   ggt gat cat gta acc cat cct gac ttc atg ttg tat gac gct ctt gat     480
   Gly Asp His Val Thr His Pro Asp Phe Met Leu Tyr Asp Ala Leu Asp
   145                 150                 155                 160
   gtt gtt tta tac atg gac cca atg tgc ctg gat gcg ttc cca aaa tta     528
   Val Val Leu Tyr Met Asp Pro Met Cys Leu Asp Ala Phe Pro Lys Leu
                   165                 170                 175
   gtt tgt ttt aaa aaa cgt att gaa gct atc cca caa att gat aag tac     576
   Val Cys Phe Lys Lys Arg Ile Glu Ala Ile Pro Gln Ile Asp Lys Tyr
               180                 185                 190
   ttg aaa tcc agc aag tat ata gca tgg cct ttg cag ggc tgg caa gcc     624
   Leu Lys Ser Ser Lys Tyr Ile Ala Trp Pro Leu Gln Gly Trp Gln Ala
           195                 200                 205
   acg ttt ggt ggt ggc gac cat cct cca aaa tcg gat ggt tca act agt       672
   Thr Phe Gly Gly Gly Asp His Pro Pro Lys Ser Asp Gly Ser Thr Ser
       210                 215                 220
   ggt tct ggt cat cac cat cac cat cac tcc gcg ggt ctg gtg cca cgc       720
   Gly Ser Gly His His His His His His Ser Ala Gly Leu Val Pro Arg
   225                 230                 235                 240
   ggt agt act gca att ggt atg aaa gaa acc gct gct gct aaa ttc gaa       768
   Gly Ser Thr Ala Ile Gly Met Lys Glu Thr Ala Ala Ala Lys Phe Glu
                   245                 250                 255
   cgc cag cac atg gac agc cca gat ctg ggt acc ggt ggt ggc tcc ggt       816
   Arg Gln His Met Asp Ser Pro Asp Leu Gly Thr Gly Gly Gly Ser Gly
               260                 265                 270
   gat gac gac gac aag ata gtc cta att tgt aac ggt gga cac gaa tac       864
   Asp Asp Asp Asp Lys Ile Val Leu Ile Cys Asn Gly Gly His Glu Tyr
           275                 280                 285
   tac gag tgc ggt gga gcc tgc gat aat gta tgt gca gat tta cat ata       912
   Tyr Glu Cys Gly Gly Ala Cys Asp Asn Val Cys Ala Asp Leu His Ile
       290                 295                 300
   cag aat aaa aca aac tgt ccc aat att aat ata aga tgt aat gac aag       960
   Gln Asn Lys Thr Asn Cys Pro Asn Ile Asn Ile Arg Cys Asn Asp Lys
   305                 310                 315                 320
   tgc tac tgt gaa gat ggc tat gca agg gat gtc aat ggc aaa tgt ata      1008
   Cys Tyr Cys Glu Asp Gly Tyr Ala Arg Asp Val Asn Gly Lys Cys Ile
                   325                 330                 335
   ccg ata aaa gac tgt cct aaa ata cgt tcg tag                          1041
   Pro Ile Lys Asp Cys Pro Lys Ile Arg Ser
               340                 345
<210> 86
   <211> 346
   <212> PRT
   <213> Galleria mellonella
<400> 86
   Met Ser Pro Ile Leu Gly Tyr Trp Lys Ile Lys Gly Leu Val Gln Pro
   1               5                   10                  15
   Thr Arg Leu Leu Leu Glu Tyr Leu Glu Glu Lys Tyr Glu Glu His Leu
               20                  25                  30
   Tyr Glu Arg Asp Glu Gly Asp Lys Trp Arg Asn Lys Lys Phe Glu Leu
           35                  40                  45
   Gly Leu Glu Phe Pro Asn Leu Pro Tyr Tyr Ile Asp Gly Asp Val Lys
       50                  55                  60
   Leu Thr Gln Ser Met Ala Ile Ile Arg Tyr Ile Ala Asp Lys His Asn
   65                  70                  75                  80
   Met Leu Gly Gly Cys Pro Lys Glu Arg Ala Glu Ile Ser Met Leu Glu
                   85                  90                  95
   Gly Ala Val Leu Asp Ile Arg Tyr Gly Val Ser Arg Ile Ala Tyr Ser
               100                 105                 110
   Lys Asp Phe Glu Thr Leu Lys Val Asp Phe Leu Ser Lys Leu Pro Glu
           115                 120                 125
   Met Leu Lys Met Phe Glu Asp Arg Leu Cys His Lys Thr Tyr Leu Asn
       130                 135                 140
   Gly Asp His Val Thr His Pro Asp Phe Met Leu Tyr Asp Ala Leu Asp
   145                 150                 155                 160
   Val Val Leu Tyr Met Asp Pro Met Cys Leu Asp Ala Phe Pro Lys Leu
                   165                 170                 175
   Val Cys Phe Lys Lys Arg Ile Glu Ala Ile Pro Gln Ile Asp Lys Tyr
               180                 185                 190
   Leu Lys Ser Ser Lys Tyr Ile Ala Trp Pro Leu Gln Gly Trp Gln Ala
           195                 200                 205
   Thr Phe Gly Gly Gly Asp His Pro Pro Lys Ser Asp Gly Ser Thr Ser
       210                 215                 220
   Gly Ser Gly His His His His His His Ser Ala Gly Leu Val Pro Arg
   225                 230                 235                 240
   Gly Ser Thr Ala Ile Gly Met Lys Glu Thr Ala Ala Ala Lys Phe Glu
                   245                 250                 255
   Arg Gln His Met Asp Ser Pro Asp Leu Gly Thr Gly Gly Gly Ser Gly
               260                 265                 270
   Asp Asp Asp Asp Lys Ile Val Leu Ile Cys Asn Gly Gly His Glu Tyr
           275                 280                 285
   Tyr Glu Cys Gly Gly Ala Cys Asp Asn Val Cys Ala Asp Leu His Ile
       290                 295                 300
   Gln Asn Lys Thr Asn Cys Pro Asn Ile Asn Ile Arg Cys Asn Asp Lys
   305                 310                 315                 320
   Cys Tyr Cys Glu Asp Gly Tyr Ala Arg Asp Val Asn Gly Lys Cys Ile
                   325                 330                 335
   Pro Ile Lys Asp Cys Pro Lys Ile Arg Ser
               340                 345
<210> 87
   <211> 1041
   <212> DNA
   <213> Galleria mellonella
<220>
   <221> CDS
   <222> (1)..(1041)
<400> 87
   atg tcc cct ata cta ggt tat tgg aaa att aag ggc ctt gtg caa ccc        48
   Met Ser Pro Ile Leu Gly Tyr Trp Lys Ile Lys Gly Leu Val Gln Pro
   1               5                   10                  15
   act cga ctt ctt ttg gaa tat ctt gaa gaa aaa tat gaa gag cat ttg        96
   Thr Arg Leu Leu Leu Glu Tyr Leu Glu Glu Lys Tyr Glu Glu His Leu
               20                  25                  30
   tat gag cgc gat gaa ggt gat aaa tgg cga aac aaa aag ttt gaa ttg       144
   Tyr Glu Arg Asp Glu Gly Asp Lys Trp Arg Asn Lys Lys Phe Glu Leu
           35                  40                  45
   ggt ttg gag ttt ccc aat ctt cct tat tat att gat ggt gat gtt aaa       192
   Gly Leu Glu Phe Pro Asn Leu Pro Tyr Tyr Ile Asp Gly Asp Val Lys
       50                  55                  60
   tta aca cag tct atg gcc atc ata cgt tat ata gct gac aag cac aac       240
   Leu Thr Gln Ser Met Ala Ile Ile Arg Tyr Ile Ala Asp Lys His Asn
   65                  70                  75                  80
   atg ttg ggt ggt tgt cca aaa gag cgt gca gag att tca atg ctt gaa       288
   Met Leu Gly Gly Cys Pro Lys Glu Arg Ala Glu Ile Ser Met Leu Glu
                   85                  90                  95
   gga gcg gtt ttg gat att aga tac ggt gtt tcg aga att gca tat agt       336
   Gly Ala Val Leu Asp Ile Arg Tyr Gly Val Ser Arg Ile Ala Tyr Ser
               100                 105                 110
   aaa gac ttt gaa act ctc aaa gtt gat ttt ctt agc aag cta cct gaa       384
   Lys Asp Phe Glu Thr Leu Lys Val Asp Phe Leu Ser Lys Leu Pro Glu
           115                 120                 125
   atg ctg aaa atg ttc gaa gat cgt tta tgt cat aaa aca tat tta aat       432
   Met Leu Lys Met Phe Glu Asp Arg Leu Cys His Lys Thr Tyr Leu Asn
       130                 135                 140
   ggt gat cat gta acc cat cct gac ttc atg ttg tat gac gct ctt gat       480
   Gly Asp His Val Thr His Pro Asp Phe Met Leu Tyr Asp Ala Leu Asp
   145                 150                 155                 160
   gtt gtt tta tac atg gac cca atg tgc ctg gat gcg ttc cca aaa tta       528
   Val Val Leu Tyr Met Asp Pro Met Cys Leu Asp Ala Phe Pro Lys Leu
                   165                 170                 175
   gtt tgt ttt aaa aaa cgt att gaa gct atc cca caa att gat aag tac       576
   Val Cys Phe Lys Lys Arg Ile Glu Ala Ile Pro Gln Ile Asp Lys Tyr
               180                 185                 190
   ttg aaa tcc agc aag tat ata gca tgg cct ttg cag ggc tgg caa gcc      624
   Leu Lys Ser Ser Lys Tyr Ile Ala Trp Pro Leu Gln Gly Trp Gln Ala
           195                 200                 205
   acg ttt ggt ggt ggc gac cat cct cca aaa tcg gat ggt tca act agt      672
   Thr Phe Gly Gly Gly Asp His Pro Pro Lys Ser Asp Gly Ser Thr Ser
       210                 215                 220
   ggt tct ggt cat cac cat cac cat cac tcc gcg ggt ctg gtg cca cgc      720
   Gly Ser Gly His His His His His His Ser Ala Gly Leu Val Pro Arg
   225                 230                 235                 240
   ggt agt act gca att ggt atg aaa gaa acc gct gct gct aaa ttc gaa      768
   Gly Ser Thr Ala Ile Gly Met Lys Glu Thr Ala Ala Ala Lys Phe Glu
                   245                 250                 255
   cgc cag cac atg gac agc cca gat ctg ggt acc ggt ggt ggc tcc ggt      816
   Arg Gln His Met Asp Ser Pro Asp Leu Gly Thr Gly Gly Gly Ser Gly
               260                 265                 270
   gat gac gac gac aag ata gtc cta att tgt aac ggt gga cac gaa tac      864
   Asp Asp Asp Asp Lys Ile Val Leu Ile Cys Asn Gly Gly His Glu Tyr
           275                 280                 285
   tac gag tgc ggt gga gcc tgc gat aat gta tgt gca gat tta cat ata      912
   Tyr Glu Cys Gly Gly Ala Cys Asp Asn Val Cys Ala Asp Leu His Ile
       290                 295                 300
   cag aat aaa aca aac tgt ccc atc agt aat ata aga tgt aat gac aag      960
   Gln Asn Lys Thr Asn Cys Pro Ile Ser Asn Ile Arg Cys Asn Asp Lys
   305                 310                 315                 320
   tgc tac tgt gaa gat ggc tat gca agg gat gtc aat ggc aaa tgt ata     1008
   Cys Tyr Cys Glu Asp Gly Tyr Ala Arg Asp Val Asn Gly Lys Cys Ile
                   325                 330                 335
   ccg ata aaa gac tgt cct aaa ata cgt tcg tag                         1041
   Pro Ile Lys Asp Cys Pro Lys Ile Arg Ser
               340                 345
<210> 88
   <211> 346
   <212> PRT
   <213> Galleria mellonella
<400> 88
   Met Ser Pro Ile Leu Gly Tyr Trp Lys Ile Lys Gly Leu Val Gln Pro
   1               5                   10                  15
   Thr Arg Leu Leu Leu Glu Tyr Leu Glu Glu Lys Tyr Glu Glu His Leu
               20                  25                  30
   Tyr Glu Arg Asp Glu Gly Asp Lys Trp Arg Asn Lys Lys Phe Glu Leu
           35                  40                  45
   Gly Leu Glu Phe Pro Asn Leu Pro Tyr Tyr Ile Asp Gly Asp Val Lys
       50                  55                  60
   Leu Thr Gln Ser Met Ala Ile Ile Arg Tyr Ile Ala Asp Lys His Asn
   65                  70                  75                  80
   Met Leu Gly Gly Cys Pro Lys Glu Arg Ala Glu Ile Ser Met Leu Glu
                   85                  90                  95
   Gly Ala Val Leu Asp Ile Arg Tyr Gly Val Ser Arg Ile Ala Tyr Ser
               100                 105                 110
   Lys Asp Phe Glu Thr Leu Lys Val Asp Phe Leu Ser Lys Leu Pro Glu
           115                 120                 125
   Met Leu Lys Met Phe Glu Asp Arg Leu Cys His Lys Thr Tyr Leu Asn
       130                 135                 140
   Gly Asp His Val Thr His Pro Asp Phe Met Leu Tyr Asp Ala Leu Asp
   145                 150                 155                 160
   Val Val Leu Tyr Met Asp Pro Met Cys Leu Asp Ala Phe Pro Lys Leu
                   165                 170                 175
   Val Cys Phe Lys Lys Arg Ile Glu Ala Ile Pro Gln Ile Asp Lys Tyr
               180                 185                 190
   Leu Lys Ser Ser Lys Tyr Ile Ala Trp Pro Leu Gln Gly Trp Gln Ala
           195                 200                 205
   Thr Phe Gly Gly Gly Asp His Pro Pro Lys Ser Asp Gly Ser Thr Ser
       210                 215                 220
   Gly Ser Gly His His His His His His Ser Ala Gly Leu Val Pro Arg
   225                 230                 235                 240
   Gly Ser Thr Ala Ile Gly Met Lys Glu Thr Ala Ala Ala Lys Phe Glu
                   245                 250                 255
   Arg Gln His Met Asp Ser Pro Asp Leu Gly Thr Gly Gly Gly Ser Gly
               260                 265                 270
   Asp Asp Asp Asp Lys Ile Val Leu Ile Cys Asn Gly Gly His Glu Tyr
           275                 280                 285
   Tyr Glu Cys Gly Gly Ala Cys Asp Asn Val Cys Ala Asp Leu His Ile
       290                 295                 300
   Gln Asn Lys Thr Asn Cys Pro Ile Ser Asn Ile Arg Cys Asn Asp Lys
   305                 310                 315                 320
   Cys Tyr Cys Glu Asp Gly Tyr Ala Arg Asp Val Asn Gly Lys Cys Ile
                   325                 330                 335
   Pro Ile Lys Asp Cys Pro Lys Ile Arg Ser
               340                 345
<210> 89
   <211> 1041
   <212> DNA
   <213> Galleria mellonella
<220>
   <221> CDS
   <222> (1)..(1041)
<400> 89
   atg tcc cct ata cta ggt tat tgg aaa att aag ggc ctt gtg caa ccc      48
   Met Ser Pro Ile Leu Gly Tyr Trp Lys Ile Lys Gly Leu Val Gln Pro
   1               5                   10                  15
   act cga ctt ctt ttg gaa tat ctt gaa gaa aaa tat gaa gag cat ttg      96
   Thr Arg Leu Leu Leu Glu Tyr Leu Glu Glu Lys Tyr Glu Glu His Leu
               20                  25                  30
   tat gag cgc gat gaa ggt gat aaa tgg cga aac aaa aag ttt gaa ttg     144
   Tyr Glu Arg Asp Glu Gly Asp Lys Trp Arg Asn Lys Lys Phe Glu Leu
           35                  40                  45
   ggt ttg gag ttt ccc aat ctt cct tat tat att gat ggt gat gtt aaa     192
   Gly Leu Glu Phe Pro Asn Leu Pro Tyr Tyr Ile Asp Gly Asp Val Lys
       50                  55                  60
   tta aca cag tct atg gcc atc ata cgt tat ata gct gac aag cac aac     240
   Leu Thr Gln Ser Met Ala Ile Ile Arg Tyr Ile Ala Asp Lys His Asn
   65                  70                  75                  80
   atg ttg ggt ggt tgt cca aaa gag cgt gca gag att tca atg ctt gaa     288
   Met Leu Gly Gly Cys Pro Lys Glu Arg Ala Glu Ile Ser Met Leu Glu
                   85                  90                  95
   gga gcg gtt ttg gat att aga tac ggt gtt tcg aga att gca tat agt     336
   Gly Ala Val Leu Asp Ile Arg Tyr Gly Val Ser Arg Ile Ala Tyr Ser
               100                 105                 110
   aaa gac ttt gaa act ctc aaa gtt gat ttt ctt agc aag cta cct gaa     384
   Lys Asp Phe Glu Thr Leu Lys Val Asp Phe Leu Ser Lys Leu Pro Glu
           115                 120                 125
   atg ctg aaa atg ttc gaa gat cgt tta tgt cat aaa aca tat tta aat     432
   Met Leu Lys Met Phe Glu Asp Arg Leu Cys His Lys Thr Tyr Leu Asn
       130                 135                 140
   ggt gat cat gta acc cat cct gac ttc atg ttg tat gac gct ctt gat     480
   Gly Asp His Val Thr His Pro Asp Phe Met Leu Tyr Asp Ala Leu Asp
   145                 150                 155                 160
   gtt gtt tta tac atg gac cca atg tgc ctg gat gcg ttc cca aaa tta      528
   Val Val Leu Tyr Met Asp Pro Met Cys Leu Asp Ala Phe Pro Lys Leu
                   165                 170                 175
   gtt tgt ttt aaa aaa cgt att gaa gct atc cca caa att gat aag tac      576
   Val Cys Phe Lys Lys Arg Ile Glu Ala Ile Pro Gln Ile Asp Lys Tyr
               180                 185                 190
   ttg aaa tcc agc aag tat ata gca tgg cct ttg cag ggc tgg caa gcc      624
   Leu Lys Ser Ser Lys Tyr Ile Ala Trp Pro Leu Gln Gly Trp Gln Ala
           195                 200                 205
   acg ttt ggt ggt ggc gac cat cct cca aaa tcg gat ggt tca act agt      672
   Thr Phe Gly Gly Gly Asp His Pro Pro Lys Ser Asp Gly Ser Thr Ser
       210                 215                 220
   ggt tct ggt cat cac cat cac cat cac tcc gcg ggt ctg gtg cca cgc      720
   Gly Ser Gly His His His His His His Ser Ala Gly Leu Val Pro Arg
   225                 230                 235                 240
   ggt agt act gca att ggt atg aaa gaa acc gct gct gct aaa ttc gaa      768
   Gly Ser Thr Ala Ile Gly Met Lys Glu Thr Ala Ala Ala Lys Phe Glu
                   245                 250                 255
   cgc cag cac atg gac agc cca gat ctg ggt acc ggt ggt ggc tcc ggt      816
   Arg Gln His Met Asp Ser Pro Asp Leu Gly Thr Gly Gly Gly Ser Gly
               260                 265                 270
   gat gac gac gac aag ata gtc cta att tgt aac ggt gga cac gaa tac      864
   Asp Asp Asp Asp Lys Ile Val Leu Ile Cys Asn Gly Gly His Glu Tyr
           275                 280                 285
   tac gag tgc ggt gga gcc tgc gat aat gta tgt gca gat tta cat ata      912
   Tyr Glu Cys Gly Gly Ala Cys Asp Asn Val Cys Ala Asp Leu His Ile
       290                 295                 300
   cag aat aaa aca aac tgt ccc atc att aat ata aaa tgt aat gac aag      960
   Gln Asn Lys Thr Asn Cys Pro Ile Ile Asn Ile Lys Cys Asn Asp Lys
   305                 310                 315                 320
   tgc tac tgt gaa gat ggc tat gca agg gat gtc aat ggc aaa tgt ata     1008
   Cys Tyr Cys Glu Asp Gly Tyr Ala Arg Asp Val Asn Gly Lys Cys Ile
                   325                 330                 335
   ccg ata aaa gac tgt cct aaa ata cgt tcg tag                         1041
   Pro Ile Lys Asp Cys Pro Lys Ile Arg Ser
               340                 345
<210> 90
   <211> 346
   <212> PRT
   <213> Galleria mellonella
<400> 90
   Met Ser Pro Ile Leu Gly Tyr Trp Lys Ile Lys Gly Leu Val Gln Pro
   1               5                   10                  15
   Thr Arg Leu Leu Leu Glu Tyr Leu Glu Glu Lys Tyr Glu Glu His Leu
               20                  25                  30
   Tyr Glu Arg Asp Glu Gly Asp Lys Trp Arg Asn Lys Lys Phe Glu Leu
           35                  40                  45
   Gly Leu Glu Phe Pro Asn Leu Pro Tyr Tyr Ile Asp Gly Asp Val Lys
       50                  55                  60
   Leu Thr Gln Ser Met Ala Ile Ile Arg Tyr Ile Ala Asp Lys His Asn
   65                  70                  75                  80
   Met Leu Gly Gly Cys Pro Lys Glu Arg Ala Glu Ile Ser Met Leu Glu
                   85                  90                  95
   Gly Ala Val Leu Asp Ile Arg Tyr Gly Val Ser Arg Ile Ala Tyr Ser
               100                 105                 110
   Lys Asp Phe Glu Thr Leu Lys Val Asp Phe Leu Ser Lys Leu Pro Glu
           115                 120                 125
   Met Leu Lys Met Phe Glu Asp Arg Leu Cys His Lys Thr Tyr Leu Asn
       130                 135                 140
   Gly Asp His Val Thr His Pro Asp Phe Met Leu Tyr Asp Ala Leu Asp
   145                 150                 155                 160
   Val Val Leu Tyr Met Asp Pro Met Cys Leu Asp Ala Phe Pro Lys Leu
                   165                 170                 175
   Val Cys Phe Lys Lys Arg Ile Glu Ala Ile Pro Gln Ile Asp Lys Tyr
               180                 185                 190
   Leu Lys Ser Ser Lys Tyr Ile Ala Trp Pro Leu Gln Gly Trp Gln Ala
           195                 200                 205
   Thr Phe Gly Gly Gly Asp His Pro Pro Lys Ser Asp Gly Ser Thr Ser
       210                 215                 220
   Gly Ser Gly His His His His His His Ser Ala Gly Leu Val Pro Arg
   225                 230                 235                 240
   Gly Ser Thr Ala Ile Gly Met Lys Glu Thr Ala Ala Ala Lys Phe Glu
                   245                 250                 255
   Arg Gln His Met Asp Ser Pro Asp Leu Gly Thr Gly Gly Gly Ser Gly
               260                 265                 270
   Asp Asp Asp Asp Lys Ile Val Leu Ile Cys Asn Gly Gly His Glu Tyr
           275                 280                 285
   Tyr Glu Cys Gly Gly Ala Cys Asp Asn Val Cys Ala Asp Leu His Ile
       290                 295                 300
   Gln Asn Lys Thr Asn Cys Pro Ile Ile Asn Ile Lys Cys Asn Asp Lys
   305                 310                 315                 320
   Cys Tyr Cys Glu Asp Gly Tyr Ala Arg Asp Val Asn Gly Lys Cys Ile
                   325                 330                 335
   Pro Ile Lys Asp Cys Pro Lys Ile Arg Ser
               340                 345
<210> 91
   <211> 1041
   <212> DNA
   <213> Galleria mellonella
<220>
   <221> CDS
   <222> (1)..(1041)
<400> 91
   atg tcc cct ata cta ggt tat tgg aaa att aag ggc ctt gtg caa ccc       48
   Met Ser Pro Ile Leu Gly Tyr Trp Lys Ile Lys Gly Leu Val Gln Pro
   1               5                   10                  15
   act cga ctt ctt ttg gaa tat ctt gaa gaa aaa tat gaa gag cat ttg       96
   Thr Arg Leu Leu Leu Glu Tyr Leu Glu Glu Lys Tyr Glu Glu His Leu
               20                  25                  30
   tat gag cgc gat gaa ggt gat aaa tgg cga aac aaa aag ttt gaa ttg      144
   Tyr Glu Arg Asp Glu Gly Asp Lys Trp Arg Asn Lys Lys Phe Glu Leu
           35                  40                  45
   ggt ttg gag ttt ccc aat ctt cct tat tat att gat ggt gat gtt aaa      192
   Gly Leu Glu Phe Pro Asn Leu Pro Tyr Tyr Ile Asp Gly Asp Val Lys
       50                  55                  60
   tta aca cag tct atg gcc atc ata cgt tat ata gct gac aag cac aac      240
   Leu Thr Gln Ser Met Ala Ile Ile Arg Tyr Ile Ala Asp Lys His Asn
   65                  70                  75                  80
   atg ttg ggt ggt tgt cca aaa gag cgt gca gag att tca atg ctt gaa      288
   Met Leu Gly Gly Cys Pro Lys Glu Arg Ala Glu Ile Ser Met Leu Glu
                   85                  90                  95
   gga gcg gtt ttg gat att aga tac ggt gtt tcg aga att gca tat agt      336
   Gly Ala Val Leu Asp Ile Arg Tyr Gly Val Ser Arg Ile Ala Tyr Ser
               100                 105                 110
   aaa gac ttt gaa act ctc aaa gtt gat ttt ctt agc aag cta cct gaa      384
   Lys Asp Phe Glu Thr Leu Lys Val Asp Phe Leu Ser Lys Leu Pro Glu
           115                 120                 125
   atg ctg aaa atg ttc gaa gat cgt tta tgt cat aaa aca tat tta aat      432
   Met Leu Lys Met Phe Glu Asp Arg Leu Cys His Lys Thr Tyr Leu Asn
       130                 135                 140
   ggt gat cat gta acc cat cct gac ttc atg ttg tat gac gct ctt gat      480
   Gly Asp His Val Thr His Pro Asp Phe Met Leu Tyr Asp Ala Leu Asp
   145                 150                 155                 160
   gtt gtt tta tac atg gac cca atg tgc ctg gat gcg ttc cca aaa tta      528
   Val Val Leu Tyr Met Asp Pro Met Cys Leu Asp Ala Phe Pro Lys Leu
                   165                 170                 175
   gtt tgt ttt aaa aaa cgt att gaa gct atc cca caa att gat aag tac      576
   Val Cys Phe Lys Lys Arg Ile Glu Ala Ile Pro Gln Ile Asp Lys Tyr
               180                 185                 190
   ttg aaa tcc agc aag tat ata gca tgg cct ttg cag ggc tgg caa gcc      624
   Leu Lys Ser Ser Lys Tyr Ile Ala Trp Pro Leu Gln Gly Trp Gln Ala
           195                 200                 205
   acg ttt ggt ggt ggc gac cat cct cca aaa tcg gat ggt tca act agt      672
   Thr Phe Gly Gly Gly Asp His Pro Pro Lys Ser Asp Gly Ser Thr Ser
       210                 215                 220
   ggt tct ggt cat cac cat cac cat cac tcc gcg ggt ctg gtg cca cgc      720
   Gly Ser Gly His His His His His His Ser Ala Gly Leu Val Pro Arg
   225                 230                 235                 240
   ggt agt act gca att ggt atg aaa gaa acc gct gct gct aaa ttc gaa      768
   Gly Ser Thr Ala Ile Gly Met Lys Glu Thr Ala Ala Ala Lys Phe Glu
                   245                 250                 255
   cgc cag cac atg gac agc cca gat ctg ggt acc ggt ggt ggc tcc ggt      816
   Arg Gln His Met Asp Ser Pro Asp Leu Gly Thr Gly Gly Gly Ser Gly
               260                 265                 270
   gat gac gac gac aag ata gtc cta att tgt aac ggt gga cac gaa tac      864
   Asp Asp Asp Asp Lys Ile Val Leu Ile Cys Asn Gly Gly His Glu Tyr
           275                 280                 285
   tac gag tgc ggt gga gcc tgc gat aat gta tgt gca gat tta cat ata      912
   Tyr Glu Cys Gly Gly Ala Cys Asp Asn Val Cys Ala Asp Leu His Ile
       290                 295                 300
   cag aat aaa aca aac tgt ccc tgt att aat ata aga tgt aat gac aag      960
   Gln Asn Lys Thr Asn Cys Pro Cys Ile Asn Ile Arg Cys Asn Asp Lys
   305                 310                 315                 320
   tgc tac tgt gaa gat ggc tat gca agg gat gtc aat ggc aaa tgt ata     1008
   Cys Tyr Cys Glu Asp Gly Tyr Ala Arg Asp Val Asn Gly Lys Cys Ile
                   325                 330                 335
   ccg ata aaa gac tgt cct aaa ata cgt tcg tag                         1041
   Pro Ile Lys Asp Cys Pro Lys Ile Arg Ser
               340                 345
<210> 92
   <211> 346
   <212> PRT
   <213> Galleria mellonella
<400> 92
   Met Ser Pro Ile Leu Gly Tyr Trp Lys Ile Lys Gly Leu Val Gln Pro
   1               5                   10                  15
   Thr Arg Leu Leu Leu Glu Tyr Leu Glu Glu Lys Tyr Glu Glu His Leu
               20                  25                  30
   Tyr Glu Arg Asp Glu Gly Asp Lys Trp Arg Asn Lys Lys Phe Glu Leu
           35                  40                  45
   Gly Leu Glu Phe Pro Asn Leu Pro Tyr Tyr Ile Asp Gly Asp Val Lys
       50                  55                  60
   Leu Thr Gln Ser Met Ala Ile Ile Arg Tyr Ile Ala Asp Lys His Asn
   65                  70                  75                  80
   Met Leu Gly Gly Cys Pro Lys Glu Arg Ala Glu Ile Ser Met Leu Glu
                   85                  90                  95
   Gly Ala Val Leu Asp Ile Arg Tyr Gly Val Ser Arg Ile Ala Tyr Ser
               100                 105                 110
   Lys Asp Phe Glu Thr Leu Lys Val Asp Phe Leu Ser Lys Leu Pro Glu
           115                 120                 125
   Met Leu Lys Met Phe Glu Asp Arg Leu Cys His Lys Thr Tyr Leu Asn
       130                 135                 140
   Gly Asp His Val Thr His Pro Asp Phe Met Leu Tyr Asp Ala Leu Asp
   145                 150                 155                 160
   Val Val Leu Tyr Met Asp Pro Met Cys Leu Asp Ala Phe Pro Lys Leu
                   165                 170                 175
   Val Cys Phe Lys Lys Arg Ile Glu Ala Ile Pro Gln Ile Asp Lys Tyr
               180                 185                 190
   Leu Lys Ser Ser Lys Tyr Ile Ala Trp Pro Leu Gln Gly Trp Gln Ala
           195                 200                 205
   Thr Phe Gly Gly Gly Asp His Pro Pro Lys Ser Asp Gly Ser Thr Ser
       210                 215                 220
   Gly Ser Gly His His His His His His Ser Ala Gly Leu Val Pro Arg
   225                 230                 235                 240
   Gly Ser Thr Ala Ile Gly Met Lys Glu Thr Ala Ala Ala Lys Phe Glu
                   245                 250                 255
   Arg Gln His Met Asp Ser Pro Asp Leu Gly Thr Gly Gly Gly Ser Gly
               260                 265                 270
   Asp Asp Asp Asp Lys Ile Val Leu Ile Cys Asn Gly Gly His Glu Tyr
           275                 280                 285
   Tyr Glu Cys Gly Gly Ala Cys Asp Asn Val Cys Ala Asp Leu His Ile
       290                 295                 300
   Gln Asn Lys Thr Asn Cys Pro Cys Ile Asn Ile Arg Cys Asn Asp Lys
   305                 310                 315                 320
   Cys Tyr Cys Glu Asp Gly Tyr Ala Arg Asp Val Asn Gly Lys Cys Ile
                   325                 330                 335
   Pro Ile Lys Asp Cys Pro Lys Ile Arg Ser
               340                 345
<210> 93
   <211> 57
   <212> DNA
   <213> Galleria mellonella
<220>
   <221> CDS
   <222> (1)..(57)
<400> 93
   atg aag tgc tta tta tat tta tgt cta tgg tgt tat tgt gta cta gta        48
   Met Lys Cys Leu Leu Tyr Leu Cys Leu Trp Cys Tyr Cys Val Leu Val
   1               5                   10                  15
   agc agt agc                                                            57
   Ser Ser Ser
<210> 94
   <211> 19
   <212> PRT
   <213> Galleria mellonella
<400> 94
   Met Lys Cys Leu Leu Tyr Leu Cys Leu Trp Cys Tyr Cys Val Leu Val
   1               5                   10                  15
   Ser Ser Ser
<210> 95
   <211> 55
   <212> PRT
   <213> Solenopsis
<220>
   <221> PEPTIDE
   <222> (1)..(55)
<400> 95
   Cys Asn Arg Glu Asn Glu Glu Tyr Gln Cys Gly Ser Ala Cys Gln Thr
   1               5                   10                  15
   Thr Cys Thr Asn Leu Gly Gln Asn Cys Ser Ile Ile Asn Ile Arg Cys
               20                  25                  30
   Asn Asp Ala Cys Tyr Cys Lys Pro Gly Tyr Ala Arg Met Gly Gly Asp
           35                  40                  45
   Ser Ser Pro Cys Ile Pro Ile
       50                  55